# EUROPEAN PATENT SPECIFICATION

(11) **EP 2 191 002 B1**
(45) Date of publication and mention of the grant of the patent: **13.04.2016**
(21) Application number: 08792433.8
(22) Date of filing: 06.08.2008
(51) Int. Cl.: C12N 15/866, A61K 39/145, A61K 39/015

(54) **BACULOVIRAL VECTORS WITH A DUAL VERTEBRATE AND BACULOVIRUS PROMOTER CONTROLLING AN IMMUNOGENIC FUSION GENE.**
BACULOVIRUSVEKTOREN MIT EINEM EIN IMMUNOGENES FUSIONSGEN KONTROLLIERENDEN VERTEBRATEN- UND BACULOVIRUS-DOPPELPROMOTOR
VECTEURS BACULOVIRAUX AVEC UN PROMOTEUR DOUBLE VERTÉBRÉ ET BACULOVIRAL CONTRÔLANT UN GÈNE IMMUNOGÈNE DE FUSION

(30) Priority: 07.08.2007 JP 2007205785
(43) Date of publication of application: 02.06.2010
(73) Proprietor: Educational Foundation Jichi Medical University, Tochigi 329-0498 (JP); Otsuka Pharmaceutical Co., Ltd., Tokyo 101-8535 (JP)
(72) Inventor: YOSHIDA, Shigeto, Shimotsuke-shi Tochigi 329-0434 (JP); KAWASAKI, Masanori, Osaka-shi Osaka 541-0045 (JP); MATSUMOTO, Makoto, Osaka-shi Osaka 541-0045 (JP); OHBA, Yoshio, Osaka-shi Osaka 541-0045 (JP); SAITO, Masahiro, Osaka-shi Osaka 541-0045 (JP); GOTO, Yoshihiro, Osaka-shi Osaka 541-0045 (JP); INAGAKI, Katsuya, Osaka-shi Osaka 541-0045 (JP); MIZUKOSHI, Masami, Osaka-shi Osaka 541-0045 (JP); HARIGUCHI, Norimitsu, Osaka-shi Osaka 541-0045 (JP); HIROTA, Kuniko, Osaka-shi Osaka 541-0045 (JP)
(74) Representative: Hoffmann Eitle
(86) International application number: PCT/JP2008/064503
(87) International publication number: WO 2009/020236

(56) References cited:
- EP-A- 1 548 119
- EP-A- 1 983 047
- WO-A-03/020322
- US-A1- 2004 071 733
- US-A1- 2005 208 066
- STRAUSS ROBERT ET AL: "Baculovirus-based vaccination vectors allow for efficient induction of immune responses against plasmodium falciparum circumsporozoite protein." MOLECULAR THERAPY : THE JOURNAL OF THE AMERICAN SOCIETY OF GENE THERAPY JAN 2007, vol. 15, no. 1, January 2007 (2007-01), pages 193-202, XP009109214 ISSN: 1525-0024
- YOSHIDA S ET AL: "Baculovirus virions displaying Plasmodium berghei circumsporozoite protein protect mice against malaria sporozoite infection" VIROLOGY, ACADEMIC PRESS,ORLANDO, US, vol. 316, no. 1, 10 November 2003 (2003-11-10), pages 161-170, XP004471042 ISSN: 0042-6822

### SEQUENCE LISTING

<110> EDUCATIONAL FOUNDATION JICHI MEDICAL UNIVERSITY
   OTSUKA PHARMACEUTICAL CO., LTD.
<120> Novel virus vector
<130> P08-76
<150> JP 2007-205785
   <151> 2007-08-07
<160> 79
<170> PatentIn version 3.4
<210> 1
   <211> 42
   <212> DNA
   <213> Artificial Sequence
<220>
   <223> primer phsp65-F1
<400> 1
   aataatagat ctaatggcca agacaattgc gtacgacgaa ga 42
<210> 2
   <211> 63
   <212> DNA
   <213> Artificial Sequence
<220>
   <223> primer phsp65-R1
<400> 2
   aatccaatgc ggccgcggga attcgattcc tgcaggtcag aaatccatgc cacccatgtc 60
   gcc 63
<210> 3
   <211> 74
   <212> DNA
   <213> Artificial Sequence
<220>
   <223> primer phsp65-F2
<400> 3
   cacccctgca ggactacaag gacgacgatg acaaggaatt catggccaag acaattgcgt 60
   acgacgaaga ggcc 74
<210> 4
   <211> 37
   <212> DNA
   <213> Artificial Sequence
<220>
   <223> primer phsp65-R2
<400> 4
   cccgggcgaa atccatgcca cccatgtcgc cgccacc 37
<210> 5
   <211> 48
   <212> DNA
   <213> Artificial Sequence
<220>
   <223> primer pPolh-F2
<400> 5
   cacccggacc ggataattaa aatgataacc atctcgcaaa taaataag 48
<210> 6
   <211> 35
   <212> DNA
   <213> Artificial Sequence
<220>
   <223> primer pgp64-R2
<400> 6
   ggtaccatat tgtctattac ggtttctaat catac 35
<210> 7
   <211> 30
   <212> DNA
   <213> Artificial Sequence
<220>
   <223> primer HA-f
<400> 7
   cctgcaggta tgaaggcaaa cctactggtc 30
<210> 8
   <211> 23
   <212> DNA
   <213> Artificial Sequence
<220>
   <223> primer HA-r
<400> 8
   gcccgggcga tgcatattct gca 23
<210> 9
   <211> 37
   <212> DNA
   <213> Artificial Sequence
<220>
   <223> primer HA-F1
<400> 9
   caccgaattc gacacaatat gtataggcta ccatgcg 37
<210> 10
   <211> 32
   <212> DNA
   <213> Artificial Sequence
<220>
   <223> primer pHA-R1
<400> 10
   cccgggcacc tctggattgg atggacggaa tg 32
<210> 11
   <211> 35
   <212> DNA
   <213> Artificial Sequence
<220>
   <223> primer Polh-f RsrII
<400> 11
   gggcggaccg gataattaaa atgataacca tctcg 35
<210> 12
   <211> 37
   <212> DNA
   <213> Artificial Sequence
<220>
   <223> primer GP64-r DraIII
<400> 12
   gggcacttag tgatattgtc tattacggtt tctaatc 37
<210> 13
   <211> 27
   <212> DNA
   <213> Artificial Sequence
<220>
   <223> primer NP-f EcoRI
<400> 13
   acggaattcc attcaattca aactgga 27
<210> 14
   <211> 31
   <212> DNA
   <213> Artificial Sequence
<220>
   <223> primer NP-r Cfr9I
<400> 14
   gatcccgggc cttgtcaatg ctgaatggca a 31
<210> 15
   <211> 31
   <212> DNA
   <213> Artificial Sequence
<220>
   <223> primer gp64(272)-f
<400> 15
   gactccccgg gtcgagcacc gagtcaagaa g 31
<210> 16
   <211> 31
   <212> DNA
   <213> Artificial Sequence
<220>
   <223> primer gp64(467)-f
<400> 16
   gactccccgg gacatcactt ccatggctga a 31
<210> 17
   <211> 39
   <212> DNA
   <213> Artificial Sequence
<220>
   <223> primer PfCSP-f(19)
<400> 17
   gactctgcag ttattccagg aataccagtg ctatggaag 39
<210> 18
   <211> 44
   <212> DNA
   <213> Artificial Sequence
<220>
   <223> PfCSP-r(373)
<400> 18
   cgatcccggg ctttttccat tttacaaatt tttttttcaa tatc 44
<210> 19
   <211> 40
   <212> DNA
   <213> Artificial Sequence
<220>
   <223> primer pAMA-F1
<400> 19
   caccgaattc aatccatggg aaaagtatac ggaaaaatat 40
<210> 20
   <211> 37
   <212> DNA
   <213> Artificial Sequence
<220>
   <223> primer pAMA1-R1
<400> 20
   cccgggcttc tctggtttga tgggctttca tatgcac 37
<210> 21
   <211> 40
   <212> DNA
   <213> Artificial Sequence
<220>
   <223> primer pPfCSP-F1
<400> 21
   caccgaattc ttattccagg aataccagtg ctatggaagt 40
<210> 22
   <211> 34
   <212> DNA
   <213> Artificial Sequence
<220>
   <223> primer pPfCSP-R1
<400> 22
   cccgggcttt ttccatttta caaatttttt tttc 34
<210> 23
   <211> 43
   <212> DNA
   <213> Artificial Sequence
<220>
   <223> primer pPfs25-F1
<400> 23
   caccgaattc aaagttaccg tggatactgt atgcaaaaga gga 43
<210> 24
   <211> 66
   <212> DNA
   <213> Artificial Sequence
<220>
   <223> primer pPfs25-R2
<400> 24
   caattgagat ccgccgccac cgccaccagt acatatagag ctttcattat ctattataaa 60
   tccatc 66
<210> 25
   <211 > 41
   <212> DNA
   <213> Artificial Sequence
<220>
   <223> primer pPfMSP119-F1
<400> 25
   caccgaattc aacatttcac aacaccaatg cgtaaaaaaa c 41
<210> 26
   <211> 64
   <212> DNA
   <213> Artificial Sequence
<220>
   <223> primer pPfMSP119-R2
<400> 26
   caattgagat ccgccgccac cgccaccgtt agaggaactg cagaaaatac catcgaaaag 60
   tgga 64
<210> 27
   <211> 50
   <212> DNA
   <213> Artificial Sequence
<220>
   <223> primer PfCSP-r(373 A361E)
<400> 27
   cgatcccggg ctttttccat tttacaaatt tttttttcaa tatcattttc 50
<210> 28
   <211> 35
   <212> DNA
   <213> Artificial Sequence
<220>
   <223> primer PfCSP-f(76)
<400> 28
   gactctgcag gatgatggaa ataacgaaga caacg 35
<210> 29
   <211> 1194
   <212> DNA
   <213> Plasmodium falciparum
<400> 29
   atgatgcgca aactggccat tctgagcgtg agcagctttc tgtttgtgga agccctgttt 60
   caggaatacc agtgctacgg cagcagcagc aacacccgcg tgctgaacga actgaactac 120
   gacaacgccg gcaccaacct gtacaacgaa ctggaaatga actactacgg caaacaggaa 180
   aactggtaca gcctgaaaaa aaacagccgc agcctgggcg aaaacgacga cggcaacaac 240
   gaagacaacg aaaaactgcg caaacccaaa cacaaaaaac tgaaacagcc cgccgacggc 300
   aaccccgacc ccaacgccaa ccccaacgtg gaccccaatg ccaacccaaa tgtggaccca 360
   aatgccaacc caaatgtgga tcctaacgcc aacccaaacg caaatcccaa tgccaaccct 420
   aacgctaatc caaacgccaa ccccaacgct aaccctaatg ctaacccaaa cgctaaccct 480
   aacgctaacc ctaacgccaa tcccaatgcc aaccccaacg ccaacccaaa cgctaaccca 540
   aacgctaacc ctaacgccaa cccaaacgcc aatcccaacg ctaaccctaa cgtggacccc 600
   aatgcaaatc ccaacgccaa tccaaacgct aatccaaacg ctaatcccaa cgctaatccc 660
   aatgccaacc caaacgcaaa tccaaatgcc aaccccaacg ccaaccctaa cgccaaccct 720
   aacgcaaacc caaacgccaa ccccaatgcc aaccctaacg ctaacccaaa cgccaatccc 780
   aatgccaacc caaacgctaa ccctaacgcc aatcccaaca agaacaacca gggcaatggc 840
   cagggccaca atatgccaaa tgaccccaac cgcaacgtgg acgaaaacgc caacgccaac 900
   agcgccgtga aaaacaacaa caacgaagaa cccagcgaca aacacattaa agaatacctg 960
   aacaaaattc agaacagcct gagcaccgaa tggagcccct gcagcgtgac ctgcggcaac 1020
   ggcattcagg tgcgcattaa acccggcagc gccaacaaac ccaaagacga actggactac 1080
   gaaaacgaca ttgaaaaaaa aatttgcaaa atggaaaaat gcagcagcgt gtttaacgtg 1140
   gtgaacagca gcattggcct gattatggtg ctgagctttc tgtttctgaa ctag 1194
<210> 30
   <211> 52
   <212> DNA
   <213> Artificial Sequence
<220>
   <223> primer PfCSP-f(+209)
<400> 30
   gactctgcag aacgctaatc caaacgctaa tcccaacgct aatcccaatg cc 52
<210> 31
   <211> 35
   <212> DNA
   <213> Artificial Sequence
<220>
   <223> primer PfCSP-r(+ A361 E)
<400> 31
   cgatcccggg ctttttccat tttgcaaatt ttttt 35
<210> 32
   <211> 35
   <212> DNA
   <213> Artificial Sequence
<220>
   <223> primer PfCSP-f(+76)
<400> 32
   gactctgcag gacgacggca acaacgaaga caacg 35
<210> 33
   <211> 32
   <212> DNA
   <213> Artificial Sequence
<220>
   <223> primer PfCSP-r(+128)
<400> 33
   cgttaggatc cacatttggg ttggcatttg gg 32
<210> 34
   <211> 35
   <212> DNA
   <213> Artificial Sequence
<220>
   <223> primer PfCSP-f(+209)
<400> 34
   gactggatcc taacgctaat ccaaacgcta atccc 35
<210> 35
   <211> 987
   <212> DNA
   <213> Influenza virus
<400> 35
   gaccagattt gcatcggata ccacgccaac aacagcaccg agcaggtcga taccatcatg 60
   gagaaaaacg tgaccgtcac ccacgctcag gacatcctgg agaagactca caatggaaag 120
   ctctgcgacc tggacggcgt gaaacccctc atcctgagag attgttctgt ggccggatgg 180
   ctgctgggaa accccatgtg cgatgaattt atcaacgtcc cagagtggag ttacatcgtg 240
   gagaaggcca accctgccaa cgacctgtgt taccccggca acttcaacga ctacgaggag 300
   ctgaagcacc tgctctcacg catcaaccac ttcgagaaga tccagattat ccctaagtct 360
   agttggagtg accacgaggc cagttccggc gtgtcctctg cctgtccata ccagggcaca 420
   cccagtttct tcagaaacgt cgtctggctg atcaagaaga acaacacata ccccaccatc 480
   aagcgaagtt acaacaacac caaccaggag gacctcctca tcctgtgggg aatccaccac 540
   tctaacgacg ctgccgaaca gacaaagctg taccagaatc ccaccaccta catctccgtg 600
   ggaacaagca ccctcaacca gcgcctggtg cccaagatcg ctacacgatc aaaggtgaat 660
   ggccagtccg gcaggatgga ctttttctgg accatcctca aacccaacga cgccatcaat 720
   tttgagtcta atggcaactt catcgccccc gagtacgctt acaagatcgt caagaaagga 780
   gactccgcca tcgtgaagtc cgaggtggag tacggcaact gcaacaccaa gtgccagacc 840
   ccaattggag ccattaactc cagtatgccc ttccacaata tccacccact gacaattggc 900
   gaatgcccca aatacgtgaa aagcaacaaa ctggtcctgg ctaccggact gcgcaacagc 960
   cccctgcgcg agcgcaggcg caagaga 987
<210> 36
   <211> 25
   <212> DNA
   <213> Artificial Sequence
<220>
   <223> primer AH-F1
<400> 36
   cagtctgcag gaccagattt gcatc 25
<210> 37
   <211> 25
   <212> DNA
   <213> Artificial Sequence
<220>
   <223> primer AH-R4
<400> 37
   cagtcccggg ctctcttgcg cctgc 25
<210> 38
   <211> 990
   <212> DNA
   <213> influenza virus
<400> 38
   gaccagatct gtatcggata ccacgccaac aacagtaccg aacaggtgga caccattatg 60
   gagaagaatg tgaccgtgac ccacgcccag gatatcctgg agaagaagca caacggcaaa 120
   ctgtgcgatc tggacggcgt gaagcccctg atcctgcgcg attgctccgt ggccggatgg 180
   ctgctgggca accctatgtg cgacgaattt atcaacgtgc ccgaatggag ttacattgtg 240
   gagaaggcta accccgtgaa tgacctgtgc taccccggag acttcaacga ctacgaagag 300
   ctgaagcatc tgctgtcaag gattaaccac ttcgagaaga tccagattat tcccaagtct 360
   agctggagct cccacgaggc ctcactggga gtgtccagcg cctgccccta ccagggcaag 420
   tcaagcttct ttcgcaacgt ggtgtggctg atcaagaaga atagtaccta ccccacaatc 480
   aagaggtcct acaacaacac caaccaggaa gacctgctgg tgctgtgggg aatccatcac 540
   cccaatgacg ctgccgaaca gaccaagctg taccagaacc caactaccta catcagcgtg 600
   ggcaccagca cactgaacca gcgcctggtg cctagaatcg ccaccagatc caaagtgaac 660
   ggccagtccg gccgcatgga atttttctgg acaatcctga agcccaatga tgccatcaac 720
   ttcgagagca atggaaactt catcgccccc gaatacgcct acaagattgt gaaaaaaggc 780
   gattccacca tcatgaagtc agaactggag tacggcaact gtaacaccaa gtgccagact 840
   cccatgggcg ccatcaactc cagcatgcca ttccacaaca tccatccact gaccatcggc 900
   gagtgcccca agtacgtgaa gtccaacaga ctggtgctgg ctaccggact gcgcaattcc 960
   ccacagaggg agagacgcag gaagaagaga 990
<210> 39
   <211> 25
   <212> DNA
   <213> Artificial Sequence
<220>
   <223> primer VN-F1
<400> 39
   cagtctgcag gaccagatct gtatc 25
<210> 40
   <211> 25
   <212> DNA
   <213> Artificial Sequence
<220>
   <223> primer VN-R4
<400> 40
   cagtcccggg ctctcttctt cctgc 25
<210> 41
   <211> 34
   <212> DNA
   <213> Artificial Sequence
<220>
   <223> primer gp64(51)-f
<400> 41
   gactccccgg gtggaaatca ccatcgtgga gacg 34
<210> 42
   <211> 34
   <212> DNA
   <213> Artificial Sequence
<220>
   <223> primer gp64(101)-f
<400> 42
   gactccccgg gatttgctta tgtggagcat cagg 34
<210> 43
   <211> 34
   <212> DNA
   <213> Artificial Sequence
<220>
   <223> primer gp64(154)-f
<400> 43
   gactccccgg gcgcaccaca cgtgcaacaa atcg 34
<210> 44
   <211> 34
   <212> DNA
   <213> Artificial Sequence
<220>
   <223> primer gp64(201)-f
<400> 44
   gactccccgg gacactgtgc ttcatcgaga cggc 34
<210> 45
   <211> 1701
   <212> DNA
   <213> influenza virus
<400> 45
   atggagaaga tcgtgctgtt gctggcaata gttagtttgg tcaagtcaga tcagatctgt 60
   attgggtacc acgctaataa ttctacagaa caggtagaca cgatcatgga gaaaaacgtg 120
   accgtcactc atgcgcaaga tattttggag aagacacaca acgggaagct ctgcgatctg 180
   gatggggtga agcctctgat tcttcgggac tgctccgtgg cggggtggtt gcttggcaac 240
   cctatgtgtg atgagttcat caacgtgcct gaatggtctt atattgtgga aaaagcgaat 300
   cccgctaacg acctttgtta ccctggtaac ttcaacgatt acgaagaact caaacacctc 360
   ctcagcagaa tcaatcactt cgaaaaaata cagattattc ccaaatcttc ctggtccgac 420
   catgaggcat ccagcggagt atcaagtgca tgcccgtacc agggcactcc ctcatttttc 480
   cgcaacgtgg tgtggttgat caagaaaaat aacacttatc cgaccatcaa gagaagctac 540
   aacaacacta accaggagga cctgttgatc ctttggggca tacatcatag caacgacgcg 600
   gcagaacaga ccaagcttta ccagaaccct acaacatata tcagcgtggg caccagtact 660
   cttaatcaac ggttggtgcc caagatcgct acaaggagta aggtgaatgg gcagagcggg 720
   cgaatggatt tcttctggac cattcttaaa cccaatgacg ctataaactt tgagagcaac 780
   ggcaacttta ttgcccccga atatgcatac aagattgtga agaagggtga cagcgccatt 840
   gtaaaaagcg aggtggagta cggtaattgt aacacaaagt gccaaacacc tataggggcc 900
   attaatagct caatgccttt ccacaacatt cacccactga ctatcggtga atgcccaaaa 960
   tacgtgaagt caaacaaact ggtactggca acagggctcc ggaattctcc cctgcgcgag 1020
   cggaggagaa agagaggact ttttggggcc attgcaggct tcattgaggg agggtggcag 1080
   ggcatggtag acggatggta tgggtatcat catagtaacg aacagggatc cggctacgcg 1140
   gccgataagg agtcaaccca gaaggcaatt gacggcgtca caaataaggt caactccata 1200
   attgataaaa tgaacaccca gttcgaggcc gtagggcgcg aatttaacaa cctcgaaaga 1260
   aggatcgaga acctgaataa gaagatggag gatgggttcc tcgacgtttg gacttataat 1320
   gctgaactct tggtcctcat ggaaaacgaa cgaacacttg actttcacga tagtaacgtc 1380
   aaaaatctgt atgataaagt gcgccttcaa ctcagagaca acgccaagga actcgggaac 1440
   gggtgcttcg agttctatca caaatgcgac aacgaatgca tggagagcgt gagaaacggc 1500
   acttatgact acccacaata ctctgaggaa gcccgactga agcgagagga aattagtggt 1560
   gtgaagctgg aaagcatcgg aacctatcaa attttgagta tttactctac agtggcaagc 1620
   tcactggcgc ttgcaatcat ggtggctggc cttagcttgt ggatgtgctc caatggaagc 1680
   ttgcagtgcc gaatttgcat c 1701
<210> 46
   <211> 32
   <212> DNA
   <213> Artificial Sequence
<220>
   <223> primer AH17-F
<400> 46
   gactctgcag gatcagatct gtattgggta cc 32
<210> 47
   <211> 31
   <212> DNA
   <213> Artificial Sequence
<220>
   <223> primer AH345-R
<400> 47
   cgatcccggg ctctctttct cctccgctcg c 31
<210> 48
   <211> 33
   <212> DNA
   <213> Artificial Sequence
<220>
   <223> primer AH410-R
<400> 48
   cgatcccggg cggcctcgaa ctgggtgttc att 33
<210> 49
   <211> 32
   <212> DNA
   <213> Artificial Sequence
<220>
   <223> primer AH473-R
<400> 49
   cgatcccggg cgtctctgag ttgaaggcgc ac 32
<210> 50
   <211> 33
   <212> DNA
   <213> Artificial Sequence
<220>
   <223> primer AH520-R
<400> 50
   cgatcccggg caccactaat ttcctctcgc ttc 33
<210> 51
   <211> 1704
   <212> DNA
   <213> influenza virus
<400> 51
   atggagaaaa ttgtcctgct gttcgctatt gtttccctgg ttaaatccga tcagatctgt 60
   atcggatatc acgcgaataa tagcacagag caagtggata ccattatgga aaagaatgtg 120
   actgtgaccc acgctcagga cattctggag aaaaagcaca acggaaaatt gtgcgacctt 180
   gatggggtga agccattgat tctgagagac tgctctgtgg ctggatggct gctggggaac 240
   cctatgtgcg atgagttcat taatgttccc gagtggtcct acatagtcga aaaggctaat 300
   cctgtcaatg atctttgcta ccctggggat tttaatgact atgaggagct gaaacatttg 360
   ttgagtagaa tcaaccactt tgagaaaatc cagatcatcc ccaagagttc ctggtcatct 420
   catgaagcaa gccttggtgt gagctcagcc tgcccttatc aaggcaaatc cagcttcttt 480
   cggaacgtgg tctggctcat caagaaaaat tcaacctatc cgactatcaa gagatcctat 540
   aacaacacaa atcaggagga tctgttggta ctgtggggca tccaccatcc taacgatgca 600
   gcagagcaga ccaagctcta ccagaaccca actacctaca tctccgttgg aactagcaca 660
   ctgaaccaga gattggtacc tagaattgct acccgatcca aagtcaatgg ccagtccgga 720
   agaatggaat tcttctggac aattctgaaa cccaatgacg ccattaattt cgagtcaaac 780
   ggcaatttca ttgctccaga gtatgcttac aagatcgtga aaaagggtga tagtacaatt 840
   atgaagagtg agttggagta cggcaactgc aatacaaaat gtcaaacacc catgggcgct 900
   atcaattcat ccatgccttt ccacaatatc caccccctta ctatcggaga gtgcccgaag 960
   tatgtcaagt ccaacaggct ggtcctggca actggactgc ggaatagccc gcaacgcgaa 1020
   cggaggagga aaaagcgggg actgtttgga gctattgcag gcttcatcga aggtggttgg 1080
   cagggcatgg tggacggttg gtatgggtat catcactcca acgaacaggg gagcggttat 1140
   gccgcagaca aagagtcaac tcagaaggca attgatggag ttacaaacaa agtgaatagc 1200
   attatcgaca aaatgaatac gcagtttgag gctgtcggcc gcgagttcaa taatctggag 1260
   cggagaatcg aaaacctgaa caaaaagatg gaggacggct tcctggacgt gtggacatat 1320
   aacgcagaac tgctcgtgct tatggagaat gaacggaccc tcgattttca cgactccaac 1380
   gtaaagaatc tgtatgacaa agtcaggctc cagcttagag ataacgccaa ggaattgggg 1440
   aatggatgtt ttgaattcta ccataagtgc gacaacgagt gcatggagtc cgtaagaaac 1500
   ggaacctatg actatcccca gtactcagag gaggcaagac ttaaaagaga ggagattagt 1560
   ggtgtgaaac tcgagtccat aggcatctat cagatcctga gtatctactc tacggtggcg 1620
   tcatccctgg ccctggccat catggttgct ggcttgtcac tctggatgtg tagtaacggg 1680
   agtctgcaat gcagaatatg tatt 1704
<210> 52
   <211> 32
   <212> DNA
   <213> Artificial Sequence
<220>
   <223> primer VN17-F
<400> 52
   gactctgcag gatcagatct gtatcggata tc 32
<210> 53
   <211> 33
   <212> DNA
   <213> Artificial Sequence
<220>
   <223> primer VN346-R
<400> 53
   cgatcccggg cccgcttttt cctcctccgt tcg 33
<210> 54
   <211> 33
   <212> DNA
   <213> Artificial Sequence
<220>
   <223> primer VN410-R
<400> 54
   cgatcccggg cctcaaactg cgtattcatt ttg 33
<210> 55
   <211> 35
   <212> DNA
   <213> Artificial Sequence
<220>
   <223> primer VN473-R
<400> 55
   cgatcccggg ctctaagctg gagcctgact ttgtc 35
<210> 56
   <211> 34
   <212> DNA
   <213> Artificial Sequence
<220>
   <223> primer VN520-R
<400> 56
   cgatcccggg cactaatctc ctctctttta agtc 34
<210> 57
   <211> 1191
   <212> DNA
   <213> Plasmodium falciparum
<400> 57
   atgatgcgaa aattggccat actgtcagtc agcagcttct tgttcgtgga ggccctgttt 60
   caggaatacc agtgctatgg ttccagctct aatacgcgag ttctgaacga gctgaactac 120
   gataacgccg gcaccaacct ctacaatgag ctggagatga attactacgg caagcaggag 180
   aattggtact cactcaagaa gaactccaga agtctcgggg agaacgacga cggaaataat 240
   gaggacaacg aaaaacttag aaaacccaaa cacaagaaac tgaaacaacc tgccgatggt 300
   aatcctgatc ctaatgcaaa cccaaatgtg gaccccaatg ctaaccccaa cgtcgatccg 360
   aacgcgaacc ctaatgtgga tcctaacgcc aatccaaacg cgaatccgaa tgccaaccca 420
   aacgccaacc caaacgctaa ccccaacgcg aaccccaacg ctaatccgaa cgccaatccc 480
   aatgctaatc ccaatgcgaa ccctaacgct aatcccaacg caaatccgaa cgcaaaccct 540
   aacgcaaacc ccaatgccaa ccctaacgcc aacccgaatg ccaatcctaa tgtggacccg 600
   aacgccaatc cgaatgcaaa cccaaatgcc aatccaaacg ctaatcctaa cgccaacccc 660
   aacgccaacc ctaatgctaa tccgaatgcg aatccaaatg ctaacccgaa cgctaatcca 720
   aatgcaaatc ccaatgcaaa tccaaatgcg aacccgaatg ctaaccctaa tgcaaatcct 780
   aatgcaaacc ctaatgcgaa tcccaatgca aaccccaata agaataatca gggaaatggc 840
   cagggacata atatgcctaa tgaccctaac aggaacgttg atgagaacgc gaatgcgaac 900
   tctgctgtaa agaacaacaa caatgaagag ccctccgata aacatattaa ggagtatctg 960
   aataagatcc agaactcctt gtctaccgaa tggtccccct gttctgtgac gtgtggtaac 1020
   ggaatccagg taaggatcaa acccggcagt gccaacaagc caaaggacga gctcgattac 1080
   gctaatgata ttgaaaagaa aatttgtaag atggagaagt gcagctccgt attcaatgtg 1140
   gtcaacagct caattggcct catcatggtt ctttcctttc tgttcctcaa t 1191
<210> 58
   <211> 34
   <212> DNA
   <213> Artificial Sequence
<220>
   <223> primer PfCSP_opt-f
<400> 58
   gactctgcag atgatgcgaa aattggccat actg 34
<210> 59
   <211> 39
   <212> DNA
   <213> Artificial Sequence
<220>
   <223> primer PfCSP_opt-r(397)
<400> 59
   cgatcccggg cattgaggaa cagaaaggaa agaaccatg 39
<210> 60
   <211> 36
   <212> DNA
   <213> Artificial Sequence
<220>
   <223> primer PfCSP_opt-f(19)
<400> 60
   gactctgcag ctgtttcagg aataccagtg ctatgg 36
<210> 61
   <211> 50
   <212> DNA
   <213> Artificial Sequence
<220>
   <223> primer PfCSP_opt-r(373)
<400> 61
   cgatcccggg ccttctccat cttacaaatt ttcttttcaa tatcattagc 50
<210> 62
   <211> 35
   <212> DNA
   <213> Artificial Sequence
<220>
   <223> primer PfCSP_opt-f(76)
<400> 62
   gactctgcag gacgacggaa ataatgagga caacg 35
<210> 63
   <211> 39
   <212> DNA
   <213> Artificial Sequence
<220>
   <223> primer PfCSP_opt-f(205)
<400> 63
   gactctgcag aatgcaaacc caaatgccaa tccaaacgc 39
<210> 64
   <211> 40
   <212> DNA
   <213> Artificial Sequence
<220>
   <223> primer gp64-p-f
<400> 64
   gactcggacc ggccagataa aaataatctt atcaattaag 40
<210> 65
   <211> 35
   <212> DNA
   <213> Artificial Sequence
<220>
   <223> primer gp64-p-r
<400> 65
   cgatactagt agcactgagg cttcttatat acccg 35
<210> 66
   <211> 38
   <212> DNA
   <213> Artificial Sequence
<220>
   <223> primer vp39-p-f
<400> 66
   gactcggacc gcgtcgtaca aatcgaaata ttgttgtg 38
<210> 67
   <211> 36
   <212> DNA
   <213> Artificial Sequence
<220>
   <223> primer gp64-p-r
<400> 67
   cgatactagt gtgattgaga aagaaatctc ttattc 36
<210> 68
   <211> 35
   <212> DNA
   <213> Artificial Sequence
<220>
   <223> primer VSV-G-f
<400> 68
   gactccccgg gcgttcgaac atcctcacat tcaag 35
<210> 69
   <211> 34
   <212> DNA
   <213> Artificial Sequence
<220>
   <223> primer VSV-G-r
<400> 69
   gactcactta gtgctttcca agtcggttca tctc 34
<210> 70
   <211> 397
   <212> PRT
   <213> PfCSP protein XP_001351122
<400> 70
   Met Met Arg Lys Leu Ala Ile Leu Ser Val Ser Ser Phe Leu Phe Val
   1 5 10 15
   Glu Ala Leu Phe Gln Glu Tyr Gln Cys Tyr Gly Ser Ser Ser Asn Thr
   20 25 30
   Arg Val Leu Asn Glu Leu Asn Tyr Asp Asn Ala Gly Thr Asn Leu Tyr
   35 40 45
   Asn Glu Leu Glu Met Asn Tyr Tyr Gly Lys Gln Glu Asn Trp Tyr Ser
   50 55 60
   Leu Lys Lys Asn Ser Arg Ser Leu Gly Glu Asn Asp Asp Gly Asn Asn
   65 70 75 80
   Glu Asp Asn Glu Lys Leu Arg Lys Pro Lys His Lys Lys Leu Lys Gln
   85 90 95
   Pro Ala Asp Gly Asn Pro Asp Pro Asn Ala Asn Pro Asn Val Asp Pro
   100 105 110
   Asn Ala Asn Pro Asn Val Asp Pro Asn Ala Asn Pro Asn Val Asp Pro
   115 120 125
   Asn Ala Asn Pro Asn Ala Asn Pro Asn Ala Asn Pro Asn Ala Asn Pro
   130 135 140
   Asn Ala Asn Pro Asn Ala Asn Pro Asn Ala Asn Pro Asn Ala Asn Pro
   145 150 155 160
   Asn Ala Asn Pro Asn Ala Asn Pro Asn Ala Asn Pro Asn Ala Asn Pro
   165 170 175
   Asn Ala Asn Pro Asn Ala Asn Pro Asn Ala Asn Pro Asn Ala Asn Pro
   180 185 190
   Asn Ala Asn Pro Asn Val Asp Pro Asn Ala Asn Pro Asn Ala Asn Pro
   195 200 205
   Asn Ala Asn Pro Asn Ala Asn Pro Asn Ala Asn Pro Asn Ala Asn Pro
   210 215 220
   Asn Ala Asn Pro Asn Ala Asn Pro Asn Ala Asn Pro Asn Ala Asn Pro
   225 230 235 240
   Asn Ala Asn Pro Asn Ala Asn Pro Asn Ala Asn Pro Asn Ala Asn Pro
   245 250 255
   Asn Ala Asn Pro Asn Ala Asn Pro Asn Ala Asn Pro Asn Ala Asn Pro
   260 265 270
   Asn Lys Asn Asn Gln Gly Asn Gly Gln Gly His Asn Met Pro Asn Asp
   275 280 285
   Pro Asn Arg Asn Val Asp Glu Asn Ala Asn Ala Asn Ser Ala Val Lys
   290 295 300
   Asn Asn Asn Asn Glu Glu Pro Ser Asp Lys His Ile Lys Glu Tyr Leu
   305 310 315 320
   Asn Lys Ile Gln Asn Ser Leu Ser Thr Glu Trp Ser Pro Cys Ser Val
   325 330 335
   Thr Cys Gly Asn Gly Ile Gln Val Arg Ile Lys Pro Gly Ser Ala Asn
   340 345 350
   Lys Pro Lys Asp Glu Leu Asp Tyr Ala Asn Asp Ile Glu Lys Lys Ile
   355 360 365
   Cys Lys Met Glu Lys Cys Ser Ser Val Phe Asn Val Val Asn Ser Ser
   370 375 380
   Ile Gly Leu Ile Met Val Leu Ser Phe Leu Phe Leu Asn
   385 390 395
<210> 71
   <211> 567
   <212> PRT
   <213> HA/A/Anhui/1/2005 protein ABD28180
<400> 71
   Met Glu Lys Ile Val Leu Leu Leu Ala Ile Val Ser Leu Val Lys Ser
   1 5 10 15
   Asp Gln Ile Cys Ile Gly Tyr His Ala Asn Asn Ser Thr Glu Gln Val
   20 25 30
   Asp Thr Ile Met Glu Lys Asn Val Thr Val Thr His Ala Gln Asp Ile
   35 40 45
   Leu Glu Lys Thr His Asn Gly Lys Leu Cys Asp Leu Asp Gly Val Lys
   50 55 60
   Pro Leu Ile Leu Arg Asp Cys Ser Val Ala Gly Trp Leu Leu Gly Asn
   65 70 75 80
   Pro Met Cys Asp Glu Phe Ile Asn Val Pro Glu Trp Ser Tyr Ile Val
   85 90 95
   Glu Lys Ala Asn Pro Ala Asn Asp Leu Cys Tyr Pro Gly Asn Phe Asn
   100 105 110
   Asp Tyr Glu Glu Leu Lys His Leu Leu Ser Arg Ile Asn His Phe Glu
   115 120 125
   Lys Ile Gln Ile Ile Pro Lys Ser Ser Trp Ser Asp His Glu Ala Ser
   130 135 140
   Ser Gly Val Ser Ser Ala Cys Pro Tyr Gln Gly Thr Pro Ser Phe Phe
   145 150 155 160
   Arg Asn Val Val Trp Leu Ile Lys Lys Asn Asn Thr Tyr Pro Thr Ile
   165 170 175
   Lys Arg Ser Tyr Asn Asn Thr Asn Gln Glu Asp Leu Leu Ile Leu Trp
   180 185 190
   Gly Ile His His Ser Asn Asp Ala Ala Glu Gln Thr Lys Leu Tyr Gln
   195 200 205
   Asn Pro Thr Thr Tyr Ile Ser Val Gly Thr Ser Thr Leu Asn Gln Arg
   210 215 220
   Leu Val Pro Lys Ile Ala Thr Arg Ser Lys Val Asn Gly Gln Ser Gly
   225 230 235 240
   Arg Met Asp Phe Phe Trp Thr Ile Leu Lys Pro Asn Asp Ala Ile Asn
   245 250 255
   Phe Glu Ser Asn Gly Asn Phe Ile Ala Pro Glu Tyr Ala Tyr Lys Ile
   260 265 270
   Val Lys Lys Gly Asp Ser Ala Ile Val Lys Ser Glu Val Glu Tyr Gly
   275 280 285
   Asn Cys Asn Thr Lys Cys Gln Thr Pro Ile Gly Ala Ile Asn Ser Ser
   290 295 300
   Met Pro Phe His Asn Ile His Pro Leu Thr Ile Gly Glu Cys Pro Lys
   305 310 315 320
   Tyr Val Lys Ser Asn Lys Leu Val Leu Ala Thr Gly Leu Arg Asn Ser
   325 330 335
   Pro Leu Arg Glu Arg Arg Arg Lys Arg Gly Leu Phe Gly Ala Ile Ala
   340 345 350
   Gly Phe Ile Glu Gly Gly Trp Gln Gly Met Val Asp Gly Trp Tyr Gly
   355 360 365
   Tyr His His Ser Asn Glu Gln Gly Ser Gly Tyr Ala Ala Asp Lys Glu
   370 375 380
   Ser Thr Gln Lys Ala Ile Asp Gly Val Thr Asn Lys Val Asn Ser Ile
   385 390 395 400
   Ile Asp Lys Met Asn Thr Gln Phe Glu Ala Val Gly Arg Glu Phe Asn
   405 410 415
   Asn Leu Glu Arg Arg Ile Glu Asn Leu Asn Lys Lys Met Glu Asp Gly
   420 425 430
   Phe Leu Asp Val Trp Thr Tyr Asn Ala Glu Leu Leu Val Leu Met Glu
   435 440 445
   Asn Glu Arg Thr Leu Asp Phe His Asp Ser Asn Val Lys Asn Leu Tyr
   450 455 460
   Asp Lys Val Arg Leu Gln Leu Arg Asp Asn Ala Lys Glu Leu Gly Asn
   465 470 475 480
   Gly Cys Phe Glu Phe Tyr His Lys Cys Asp Asn Glu Cys Met Glu Ser
   485 490 495
   Val Arg Asn Gly Thr Tyr Asp Tyr Pro Gln Tyr Ser Glu Glu Ala Arg
   500 505 510
   Leu Lys Arg Glu Glu Ile Ser Gly Val Lys Leu Glu Ser Ile Gly Thr
   515 520 525
   Tyr Gln Ile Leu Ser Ile Tyr Ser Thr Val Ala Ser Ser Leu Ala Leu
   530 535 540
   Ala Ile Met Val Ala Gly Leu Ser Leu Trp Met Cys Ser Asn Gly Ser
   545 550 555 560
   Leu Gln Cys Arg Ile Cys Ile
   565
<210> 72
   <211> 568
   <212> PRT
   <213> HA/A/Vietnam/1203/2004 protein AAW80717
<400> 72
   Met Glu Lys Ile Val Leu Leu Phe Ala Ile Val Ser Leu Val Lys Ser
   1 5 10 15
   Asp Gln Ile Cys Ile Gly Tyr His Ala Asn Asn Ser Thr Glu Gln Val
   20 25 30
   Asp Thr Ile Met Glu Lys Asn Val Thr Val Thr His Ala Gln Asp Ile
   35 40 45
   Leu Glu Lys Lys His Asn Gly Lys Leu Cys Asp Leu Asp Gly Val Lys
   50 55 60
   Pro Leu Ile Leu Arg Asp Cys Ser Val Ala Gly Trp Leu Leu Gly Asn
   65 70 75 80
   Pro Met Cys Asp Glu Phe Ile Asn Val Pro Glu Trp Ser Tyr Ile Val
   85 90 95
   Glu Lys Ala Asn Pro Val Asn Asp Leu Cys Tyr Pro Gly Asp Phe Asn
   100 105 110
   Asp Tyr Glu Glu Leu Lys His Leu Leu Ser Arg Ile Asn His Phe Glu
   115 120 125
   Lys Ile Gln Ile Ile Pro Lys Ser Ser Trp Ser Ser His Glu Ala Ser
   130 135 140
   Leu Gly Val Ser Ser Ala Cys Pro Tyr Gln Gly Lys Ser Ser Phe Phe
   145 150 155 160
   Arg Asn Val Val Trp Leu Ile Lys Lys Asn Ser Thr Tyr Pro Thr Ile
   165 170 175
   Lys Arg Ser Tyr Asn Asn Thr Asn Gln Glu Asp Leu Leu Val Leu Trp
   180 185 190
   Gly Ile His His Pro Asn Asp Ala Ala Glu Gln Thr Lys Leu Tyr Gln
   195 200 205
   Asn Pro Thr Thr Tyr Ile Ser Val Gly Thr Ser Thr Leu Asn Gln Arg
   210 215 220
   Leu Val Pro Arg Ile Ala Thr Arg Ser Lys Val Asn Gly Gln Ser Gly
   225 230 235 240
   Arg Met Glu Phe Phe Trp Thr Ile Leu Lys Pro Asn Asp Ala Ile Asn
   245 250 255
   Phe Glu Ser Asn Gly Asn Phe Ile Ala Pro Glu Tyr Ala Tyr Lys Ile
   260 265 270
   Val Lys Lys Gly Asp Ser Thr Ile Met Lys Ser Glu Leu Glu Tyr Gly
   275 280 285
   Asn Cys Asn Thr Lys Cys Gln Thr Pro Met Gly Ala Ile Asn Ser Ser
   290 295 300
   Met Pro Phe His Asn Ile His Pro Leu Thr Ile Gly Glu Cys Pro Lys
   305 310 315 320
   Tyr Val Lys Ser Asn Arg Leu Val Leu Ala Thr Gly Leu Arg Asn Ser
   325 330 335
   Pro Gln Arg Glu Arg Arg Arg Lys Lys Arg Gly Leu Phe Gly Ala Ile
   340 345 350
   Ala Gly Phe Ile Glu Gly Gly Trp Gln Gly Met Val Asp Gly Trp Tyr
   355 360 365
   Gly Tyr His His Ser Asn Glu Gln Gly Ser Gly Tyr Ala Ala Asp Lys
   370 375 380
   Glu Ser Thr Gln Lys Ala Ile Asp Gly Val Thr Asn Lys Val Asn Ser
   385 390 395 400
   Ile Ile Asp Lys Met Asn Thr Gln Phe Glu Ala Val Gly Arg Glu Phe
   405 410 415
   Asn Asn Leu Glu Arg Arg Ile Glu Asn Leu Asn Lys Lys Met Glu Asp
   420 425 430
   Gly Phe Leu Asp Val Trp Thr Tyr Asn Ala Glu Leu Leu Val Leu Met
   435 440 445
   Glu Asn Glu Arg Thr Leu Asp Phe His Asp Ser Asn Val Lys Asn Leu
   450 455 460
   Tyr Asp Lys Val Arg Leu Gln Leu Arg Asp Asn Ala Lys Glu Leu Gly
   465 470 475 480
   Asn Gly Cys Phe Glu Phe Tyr His Lys Cys Asp Asn Glu Cys Met Glu
   485 490 495
   Ser Val Arg Asn Gly Thr Tyr Asp Tyr Pro Gln Tyr Ser Glu Glu Ala
   500 505 510
   Arg Leu Lys Arg Glu Glu Ile Ser Gly Val Lys Leu Glu Ser Ile Gly
   515 520 525
   Ile Tyr Gln Ile Leu Ser Ile Tyr Ser Thr Val Ala Ser Ser Leu Ala
   530 535 540
   Leu Ala Ile Met Val Ala Gly Leu Ser Leu Trp Met Cys Ser Asn Gly
   545 550 555 560
   Ser Leu Gln Cys Arg Ile Cys Ile
   565
<210> 73
   <211> 1720
   <212> PRT
   <213> PfMSP1 protein XP_001352170
<400> 73
   Met Lys Ile Ile Phe Phe Leu Cys Ser Phe Leu Phe Phe Ile Ile Asn
   1 5 10 15
   Thr Gln Cys Val Thr His Glu Ser Tyr Gln Glu Leu Val Lys Lys Leu
   20 25 30
   Glu Ala Leu Glu Asp Ala Val Leu Thr Gly Tyr Ser Leu Phe Gln Lys
   35 40 45
   Glu Lys Met Val Leu Asn Glu Glu Glu Ile Thr Thr Lys Gly Ala Ser
   50 55 60
   Ala Gln Ser Gly Ala Ser Ala Gln Ser Gly Ala Ser Ala Gln Ser Gly
   65 70 75 80
   Ala Ser Ala Gln Ser Gly Ala Ser Ala Gln Ser Gly Ala Ser Ala Gln
   85 90 95
   Ser Gly Thr Ser Gly Pro Ser Gly Pro Ser Gly Thr Ser Pro Ser Ser
   100 105 110
   Arg Ser Asn Thr Leu Pro Arg Ser Asn Thr Ser Ser Gly Ala Ser Pro
   115 120 125
   Pro Ala Asp Ala Ser Asp Ser Asp Ala Lys Ser Tyr Ala Asp Leu Lys
   130 135 140
   His Arg Val Arg Asn Tyr Leu Phe Thr Ile Lys Glu Leu Lys Tyr Pro
   145 150 155 160
   Glu Leu Phe Asp Leu Thr Asn His Met Leu Thr Leu Cys Asp Asn Ile
   165 170 175
   His Gly Phe Lys Tyr Leu Ile Asp Gly Tyr Glu Glu Ile Asn Glu Leu
   180 185 190
   Leu Tyr Lys Leu Asn Phe Tyr Phe Asp Leu Leu Arg Ala Lys Leu Asn
   195 200 205
   Asp Val Cys Ala Asn Asp Tyr Cys Gln Ile Pro Phe Asn Leu Lys Ile
   210 215 220
   Arg Ala Asn Glu Leu Asp Val Leu Lys Lys Leu Val Phe Gly Tyr Arg
   225 230 235 240
   Lys Pro Leu Asp Asn Ile Lys Asp Asn Val Gly Lys Met Glu Asp Tyr
   245 250 255
   Ile Lys Lys Asn Lys Thr Thr Ile Ala Asn Ile Asn Glu Leu Ile Glu
   260 265 270
   Gly Ser Lys Lys Thr Ile Asp Gln Asn Lys Asn Ala Asp Asn Glu Glu
   275 280 285
   Gly Lys Lys Lys Leu Tyr Gln Ala Gln Tyr Asp Leu Ser Ile Tyr Asn
   290 295 300
   Lys Gln Leu Glu Glu Ala His Asn Leu Ile Ser Val Leu Glu Lys Arg
   305 310 315 320
   Ile Asp Thr Leu Lys Lys Asn Glu Asn Ile Lys Lys Leu Leu Asp Lys
   325 330 335
   Ile Asn Glu Ile Lys Asn Pro Pro Pro Ala Asn Ser Gly Asn Thr Pro
   340 345 350
   Asn Thr Leu Leu Asp Lys Asn Lys Lys Ile Glu Glu His Glu Glu Lys
   355 360 365
   Ile Lys Glu Ile Ala Lys Thr Ile Lys Phe Asn Ile Asp Ser Leu Phe
   370 375 380
   Thr Asp Pro Leu Glu Leu Glu Tyr Tyr Leu Arg Glu Lys Asn Lys Lys
   385 390 395 400
   Val Asp Val Thr Pro Lys Ser Gln Asp Pro Thr Lys Ser Val Gln Ile
   405 410 415
   Pro Lys Val Pro Tyr Pro Asn Gly Ile Val Tyr Pro Leu Pro Leu Thr
   420 425 430
   Asp Ile His Asn Ser Leu Ala Ala Asp Asn Asp Lys Asn Ser Tyr Gly
   435 440 445
   Asp Leu Met Asn Pro His Thr Lys Glu Lys Ile Asn Glu Lys Ile Ile
   450 455 460
   Thr Asp Asn Lys Glu Arg Lys Ile Phe Ile Asn Asn Ile Lys Lys Lys
   465 470 475 480
   Ile Asp Leu Glu Glu Lys Asn Ile Asn His Thr Lys Glu Gln Asn Lys
   485 490 495
   Lys Leu Leu Glu Asp Tyr Glu Lys Ser Lys Lys Asp Tyr Glu Glu Leu
   500 505 510
   Leu Glu Lys Phe Tyr Glu Met Lys Phe Asn Asn Asn Phe Asn Lys Asp
   515 520 525
   Val Val Asp Lys Ile Phe Ser Ala Arg Tyr Thr Tyr Asn Val Glu Lys
   530 535 540
   Gln Arg Tyr Asn Asn Lys Phe Ser Ser Ser Asn Asn Ser Val Tyr Asn
   545 550 555 560
   Val Gln Lys Leu Lys Lys Ala Leu Ser Tyr Leu Glu Asp Tyr Ser Leu
   565 570 575
   Arg Lys Gly Ile Ser Glu Lys Asp Phe Asn His Tyr Tyr Thr Leu Lys
   580 585 590
   Thr Gly Leu Glu Ala Asp Ile Lys Lys Leu Thr Glu Glu Ile Lys Ser
   595 600 605
   Ser Glu Asn Lys Ile Leu Glu Lys Asn Phe Lys Gly Leu Thr His Ser
   610 615 620
   Ala Asn Gly Ser Leu Glu Val Ser Asp Ile Val Lys Leu Gln Val Gln
   625 630 635 640
   Lys Val Leu Leu Ile Lys Lys Ile Glu Asp Leu Arg Lys Ile Glu Leu
   645 650 655
   Phe Leu Lys Asn Ala Gln Leu Lys Asp Ser Ile His Val Pro Asn Ile
   660 665 670
   Tyr Lys Pro Gln Asn Lys Pro Glu Pro Tyr Tyr Leu Ile Val Leu Lys
   675 680 685
   Lys Glu Val Asp Lys Leu Lys Glu Phe Ile Pro Lys Val Lys Asp Met
   690 695 700
   Leu Lys Lys Glu Gln Ala Val Leu Ser Ser Ile Thr Gln Pro Leu Val
   705 710 715 720
   Ala Ala Ser Glu Thr Thr Glu Asp Gly Gly His Ser Thr His Thr Leu
   725 730 735
   Ser Gln Ser Gly Glu Thr Glu Val Thr Glu Glu Thr Glu Glu Thr Glu
   740 745 750
   Glu Thr Val Gly His Thr Thr Thr Val Thr Ile Thr Leu Pro Pro Thr
   755 760 765
   Gln Pro Ser Pro Pro Lys Glu Val Lys Val Val Glu Asn Ser Ile Glu
   770 775 780
   His Lys Ser Asn Asp Asn Ser Gln Ala Leu Thr Lys Thr Val Tyr Leu
   785 790 795 800
   Lys Lys Leu Asp Glu Phe Leu Thr Lys Ser Tyr Ile Cys His Lys Tyr
   805 810 815
   Ile Leu Val Ser Asn Ser Ser Met Asp Gln Lys Leu Leu Glu Val Tyr
   820 825 830
   Asn Leu Thr Pro Glu Glu Glu Asn Glu Leu Lys Ser Cys Asp Pro Leu
   835 840 845
   Asp Leu Leu Phe Asn Ile Gln Asn Asn Ile Pro Ala Met Tyr Ser Leu
   850 855 860
   Tyr Asp Ser Met Asn Asn Asp Leu Gln His Leu Phe Phe Glu Leu Tyr
   865 870 875 880
   Gln Lys Glu Met Ile Tyr Tyr Leu His Lys Leu Lys Glu Glu Asn His
   885 890 895
   Ile Lys Lys Leu Leu Glu Glu Gln Lys Gln Ile Thr Gly Thr Ser Ser
   900 905 910
   Thr Ser Ser Pro Gly Asn Thr Thr Val Asn Thr Ala Gln Ser Ala Thr
   915 920 925
   His Ser Asn Ser Gln Asn Gln Gln Ser Asn Ala Ser Ser Thr Asn Thr
   930 935 940
   Gln Asn Gly Val Ala Val Ser Ser Gly Pro Ala Val Val Glu Glu Ser
   945 950 955 960
   His Asp Pro Leu Thr Val Leu Ser Ile Ser Asn Asp Leu Lys Gly Ile
   965 970 975
   Val Ser Leu Leu Asn Leu Gly Asn Lys Thr Lys Val Pro Asn Pro Leu
   980 985 990
   Thr Ile Ser Thr Thr Glu Met Glu Lys Phe Tyr Glu Asn Ile Leu Lys
   995 1000 1005
   Asn Asn Asp Thr Tyr Phe Asn Asp Asp Ile Lys Gln Phe Val Lys
   1010 1015 1020
   Ser Asn Ser Lys Val Ile Thr Gly Leu Thr Glu Thr Gln Lys Asn
   1025 1030 1035
   Ala Leu Asn Asp Glu Ile Lys Lys Leu Lys Asp Thr Leu Gln Leu
   1040 1045 1050
   Ser Phe Asp Leu Tyr Asn Lys Tyr Lys Leu Lys Leu Asp Arg Leu
   1055 1060 1065
   Phe Asn Lys Lys Lys Glu Leu Gly Gln Asp Lys Met Gln Ile Lys
   1070 1075 1080
   Lys Leu Thr Leu Leu Lys Glu Gln Leu Glu Ser Lys Leu Asn Ser
   1085 1090 1095
   Leu Asn Asn Pro His Asn Val Leu Gln Asn Phe Ser Val Phe Phe
   1100 1105 1110
   Asn Lys Lys Lys Glu Ala Glu Ile Ala Glu Thr Glu Asn Thr Leu
   1115 1120 1125
   Glu Asn Thr Lys Ile Leu Leu Lys His Tyr Lys Gly Leu Val Lys
   1130 1135 1140
   Tyr Tyr Asn Gly Glu Ser Ser Pro Leu Lys Thr Leu Ser Glu Val
   1145 1150 1155
   Ser Ile Gln Thr Glu Asp Asn Tyr Ala Asn Leu Glu Lys Phe Arg
   1160 1165 1170
   Val Leu Ser Lys Ile Asp Gly Lys Leu Asn Asp Asn Leu His Leu
   1175 1180 1185
   Gly Lys Lys Lys Leu Ser Phe Leu Ser Ser Gly Leu His His Leu
   1190 1195 1200
   Ile Thr Glu Leu Lys Glu Val Ile Lys Asn Lys Asn Tyr Thr Gly
   1205 1210 1215
   Asn Ser Pro Ser Glu Asn Asn Lys Lys Val Asn Glu Ala Leu Lys
   1220 1225 1230
   Ser Tyr Glu Asn Phe Leu Pro Glu Ala Lys Val Thr Thr Val Val
   1235 1240 1245
   Thr Pro Pro Gln Pro Asp Val Thr Pro Ser Pro Leu Ser Val Arg
   1250 1255 1260
   Val Ser Gly Ser Ser Gly Ser Thr Lys Glu Glu Thr Gln Ile Pro
   1265 1270 1275
   Thr Ser Gly Ser Leu Leu Thr Glu Leu Gln Gln Val Val Gln Leu
   1280 1285 1290
   Gln Asn Tyr Asp Glu Glu Asp Asp Ser Leu Val Val Leu Pro Ile
   1295 1300 1305
   Phe Gly Glu Ser Glu Asp Asn Asp Glu Tyr Leu Asp Gln Val Val
   1310 1315 1320
   Thr Gly Glu Ala Ile Ser Val Thr Met Asp Asn Ile Leu Ser Gly
   1325 1330 1335
   Phe Glu Asn Glu Tyr Asp Val Ile Tyr Leu Lys Pro Leu Ala Gly
   1340 1345 1350
   Val Tyr Arg Ser Leu Lys Lys Gln Ile Glu Lys Asn Ile Phe Thr
   1355 1360 1365
   Phe Asn Leu Asn Leu Asn Asp Ile Leu Asn Ser Arg Leu Lys Lys
   1370 1375 1380
   Arg Lys Tyr Phe Leu Asp Val Leu Glu Ser Asp Leu Met Gln Phe
   1385 1390 1395
   Lys His Ile Ser Ser Asn Glu Tyr Ile Ile Glu Asp Ser Phe Lys
   1400 1405 1410
   Leu Leu Asn Ser Glu Gln Lys Asn Thr Leu Leu Lys Ser Tyr Lys
   1415 1420 1425
   Tyr Ile Lys Glu Ser Val Glu Asn Asp Ile Lys Phe Ala Gln Glu
   1430 1435 1440
   Gly Ile Ser Tyr Tyr Glu Lys Val Leu Ala Lys Tyr Lys Asp Asp
   1445 1450 1455
   Leu Glu Ser Ile Lys Lys Val Ile Lys Glu Glu Lys Glu Lys Phe
   1460 1465 1470
   Pro Ser Ser Pro Pro Thr Thr Pro Pro Ser Pro Ala Lys Thr Asp
   1475 1480 1485
   Glu Gln Lys Lys Glu Ser Lys Phe Leu Pro Phe Leu Thr Asn Ile
   1490 1495 1500
   Glu Thr Leu Tyr Asn Asn Leu Val Asn Lys Ile Asp Asp Tyr Leu
   1505 1510 1515
   Ile Asn Leu Lys Ala Lys Ile Asn Asp Cys Asn Val Glu Lys Asp
   1520 1525 1530
   Glu Ala His Val Lys Ile Thr Lys Leu Ser Asp Leu Lys Ala Ile
   1535 1540 1545
   Asp Asp Lys Ile Asp Leu Phe Lys Asn Pro Tyr Asp Phe Glu Ala
   1550 1555 1560
   Ile Lys Lys Leu Ile Asn Asp Asp Thr Lys Lys Asp Met Leu Gly
   1565 1570 1575
   Lys Leu Leu Ser Thr Gly Leu Val Gln Asn Phe Pro Asn Thr Ile
   1580 1585 1590
   Ile Ser Lys Leu Ile Glu Gly Lys Phe Gln Asp Met Leu Asn Ile
   1595 1600 1605
   Ser Gln His Gln Cys Val Lys Lys Gln Cys Pro Glu Asn Ser Gly
   1610 1615 1620
   Cys Phe Arg His Leu Asp Glu Arg Glu Glu Cys Lys Cys Leu Leu
   1625 1630 1635
   Asn Tyr Lys Gln Glu Gly Asp Lys Cys Val Glu Asn Pro Asn Pro
   1640 1645 1650
   Thr Cys Asn Glu Asn Asn Gly Gly Cys Asp Ala Asp Ala Thr Cys
   1655 1660 1665
   Thr Glu Glu Asp Ser Gly Ser Ser Arg Lys Lys Ile Thr Cys Glu
   1670 1675 1680
   Cys Thr Lys Pro Asp Ser Tyr Pro Leu Phe Asp Gly Ile Phe Cys
   1685 1690 1695
   Ser Ser Ser Asn Phe Leu Gly Ile Ser Phe Leu Leu Ile Leu Met
   1700 1705 1710
   Leu Ile Leu Tyr Ser Phe Ile
   1715 1720
<210> 74
   <211> 217
   <212> PRT
   <213> Pfs25 protein XP_001347587
<400> 74
   Met Asn Lys Leu Tyr Ser Leu Phe Leu Phe Leu Phe Ile Gln Leu Ser
   1 5 10 15
   Ile Lys Tyr Asn Asn Ala Lys Val Thr Val Asp Thr Val Cys Lys Arg
   20 25 30
   Gly Phe Leu Ile Gln Met Ser Gly His Leu Glu Cys Lys Cys Glu Asn
   35 40 45
   Asp Leu Val Leu Val Asn Glu Glu Thr Cys Glu Glu Lys Val Leu Lys
   50 55 60
   Cys Asp Glu Lys Thr Val Asn Lys Pro Cys Gly Asp Phe Ser Lys Cys
   65 70 75 80
   Ile Lys Ile Asp Gly Asn Pro Val Ser Tyr Ala Cys Lys Cys Asn Leu
   85 90 95
   Gly Tyr Asp Met Val Asn Asn Val Cys Ile Pro Asn Glu Cys Lys Asn
   100 105 110
   Val Thr Cys Gly Asn Gly Lys Cys Ile Leu Asp Thr Ser Asn Pro Val
   115 120 125
   Lys Thr Gly Val Cys Ser Cys Asn Ile Gly Lys Val Pro Asn Val Gln
   130 135 140
   Asp Gln Asn Lys Cys Ser Lys Asp Gly Glu Thr Lys Cys Ser Leu Lys
   145 150 155 160
   Cys Leu Lys Glu Asn Glu Thr Cys Lys Ala Val Asp Gly Ile Tyr Lys
   165 170 175
   Cys Asp Cys Lys Asp Gly Phe Ile Ile Asp Asn Glu Ser Ser Ile Cys
   180 185 190
   Thr Ala Phe Ser Ala Tyr Asn Ile Leu Asn Leu Ser Ile Met Phe Ile
   195 200 205
   Leu Phe Ser Val Cys Phe Phe Ile Met
   210 215
<210> 75
   <211> 1194
   <212> DNA
   <213> PfCSP gene XM_001351086
<400> 75
   atgatgagaa aattagctat tttatctgtt tcttcctttt tatttgttga ggccttattc 60
   caggaatacc agtgctatgg aagttcgtca aacacaaggg ttctaaatga attaaattat 120
   gataatgcag gcactaattt atataatgaa ttagaaatga attattatgg gaaacaggaa 180
   aattggtata gtcttaaaaa aaatagtaga tcacttggag aaaatgatga tggaaataac 240
   gaagacaacg agaaattaag gaaaccaaaa cataaaaaat taaagcaacc agcggatggt 300
   aatcctgatc caaatgcaaa cccaaatgta gatcccaatg ccaacccaaa tgtagatcca 360
   aatgcaaacc caaatgtaga tccaaatgca aacccaaatg caaacccaaa tgcaaaccca 420
   aatgcaaacc caaatgcaaa cccaaatgca aacccaaatg caaacccaaa tgcaaaccca 480
   aatgcaaacc caaatgcaaa cccaaatgca aacccaaatg caaacccaaa tgcaaaccca 540
   aatgcaaacc ccaatgcaaa tcctaatgca aacccaaatg caaacccaaa cgtagatcct 600
   aatgcaaatc caaatgcaaa cccaaacgca aaccccaatg caaatcctaa tgcaaacccc 660
   aatgcaaatc ctaatgcaaa tcctaatgcc aatccaaatg caaatccaaa tgcaaaccca 720
   aacgcaaacc ccaatgcaaa tcctaatgcc aatccaaatg caaatccaaa tgcaaaccca 780
   aatgcaaacc caaatgcaaa ccccaatgca aatcctaata aaaacaatca aggtaatgga 840
   caaggtcaca atatgccaaa tgacccaaac cgaaatgtag atgaaaatgc taatgccaac 900
   agtgctgtaa aaaataataa taacgaagaa ccaagtgata agcacataaa agaatattta 960
   aacaaaatac aaaattctct ttcaactgaa tggtccccat gtagtgtaac ttgtggaaat 1020
   ggtattcaag ttagaataaa gcctggctct gctaataaac ctaaagacga attagattat 1080
   gcaaatgata ttgaaaaaaa aatttgtaaa atggaaaaat gttccagtgt gtttaatgtc 1140
   gtaaatagtt caataggatt aataatggta ttatccttct tgttccttaa ttag 1194
<210> 76
   <211> 1704
   <212> DNA
   <213> HA/A/Anhui/1/2005 gene DQ371928
<400> 76
   atggagaaaa tagtgcttct tcttgcaata gtcagccttg ttaaaagtga tcagatttgc 60
   attggttacc atgcaaacaa ctcgacagag caggttgaca caataatgga aaagaacgtt 120
   actgttacac atgcccaaga catactggaa aagacacaca acgggaagct ctgcgatcta 180
   gatggagtga agcctctgat tttaagagat tgtagtgtag ctggatggct cctcggaaac 240
   ccaatgtgtg acgaattcat caatgtgccg gaatggtctt acatagtgga gaaggccaac 300
   ccagccaatg acctctgtta cccagggaat ttcaacgact atgaagaact gaaacaccta 360
   ttgagcagaa taaaccattt tgagaaaatt cagatcatcc ccaaaagttc ttggtccgat 420
   catgaagcct catcaggggt gagctcagca tgtccatacc agggaacgcc ctcctttttc 480
   agaaatgtgg tatggcttat caaaaagaac aatacatacc caacaataaa gagaagctac 540
   aataatacca accaggaaga tcttttgata ctgtggggga ttcatcattc taatgatgcg 600
   gcagagcaga caaagctcta tcaaaaccca accacctata tttccgttgg gacatcaaca 660
   ctaaaccaga gattggtacc aaaaatagct actagatcca aagtaaacgg gcaaagtgga 720
   aggatggatt tcttctggac aattttaaaa ccgaatgatg caatcaactt cgagagtaat 780
   ggaaatttca ttgctccaga atatgcatac aaaattgtca agaaagggga ctcagcaatt 840
   gttaaaagtg aagtggaata tggtaactgc aacacaaagt gtcaaactcc aataggggcg 900
   ataaactcta gtatgccatt ccacaacata caccctctca ccatcgggga atgccccaaa 960
   tatgtgaaat caaacaaatt agtccttgcg actgggctca gaaatagtcc tctaagagaa 1020
   agaagaagaa aaagaggact atttggagct atagcagggt ttatagaggg aggatggcag 1080
   ggaatggtag atggttggta tgggtaccac catagcaatg agcaggggag tgggtacgct 1140
   gcagacaaag aatccactca aaaggcaata gatggagtca ccaataaggt caactcgatc 1200
   attgacaaaa tgaacactca gtttgaggcc gttggaaggg aatttaataa cttagaaagg 1260
   agaatagaga atttaaacaa gaaaatggaa gacggattcc tagatgtctg gacttataat 1320
   gctgaacttc tggttctcat ggaaaatgag agaactctag acttccatga ttcaaatgtc 1380
   aagaaccttt acgacaaggt ccgactacag cttagggata atgcaaagga gctgggtaac 1440
   ggttgtttcg agttctatca caaatgtgat aatgaatgta tggaaagtgt aagaaacgga 1500
   acgtatgact acccgcagta ttcagaagaa gcaagattaa aaagagagga aataagtgga 1560
   gtaaaattgg aatcaatagg aacttaccaa atactgtcaa tttattcaac agttgcgagt 1620
   tctctagcac tggcaatcat ggtggctggt ctatctttgt ggatgtgctc caatgggtcg 1680
   ttacaatgca gaatttgcat ttaa 1704
<210> 77
   <211> 1707
   <212> DNA
   <213> HA/A/Vietnam/1203/2004 gene AY818135
<400> 77
   atggagaaaa tagtgcttct ttttgcaata gtcagtcttg ttaaaagtga tcagatttgc 60
   attggttacc atgcaaacaa ctcgacagag caggttgaca caataatgga aaagaacgtt 120
   actgttacac atgcccaaga catactggaa aagaaacaca acgggaagct ctgcgatcta 180
   gatggagtga agcctctaat tttgagagat tgtagcgtag ctggatggct cctcggaaac 240
   ccaatgtgtg acgaattcat caatgtgccg gaatggtctt acatagtgga gaaggccaat 300
   ccagtcaatg acctctgtta cccaggggat ttcaatgact atgaagaatt gaaacaccta 360
   ttgagcagaa taaaccattt tgagaaaatt cagatcatcc ccaaaagttc ttggtccagt 420
   catgaagcct cattaggggt gagctcagca tgtccatacc agggaaagtc ctcctttttc 480
   agaaatgtgg tatggcttat caaaaagaac agtacatacc caacaataaa gaggagctac 540
   aataatacca accaagaaga tcttttggta ctgtggggga ttcaccatcc taatgatgcg 600
   gcagagcaga caaagctcta tcaaaaccca accacctata tttccgttgg gacatcaaca 660
   ctaaaccaga gattggtacc aagaatagct actagatcca aagtaaacgg gcaaagtgga 720
   aggatggagt tcttctggac aattttaaag ccgaatgatg caatcaactt cgagagtaat 780
   ggaaatttca ttgctccaga atatgcatac aaaattgtca agaaagggga ctcaacaatt 840
   atgaaaagtg aattggaata tggtaactgc aacaccaagt gtcaaactcc aatgggggcg 900
   ataaactcta gcatgccatt ccacaatata caccctctca ccattgggga atgccccaaa 960
   tatgtgaaat caaacagatt agtccttgcg actgggctca gaaatagccc tcaaagagag 1020
   agaagaagaa aaaagagagg attatttgga gctatagcag gttttataga gggaggatgg 1080
   cagggaatgg tagatggttg gtatgggtac caccatagca atgagcaggg gagtgggtac 1140
   gctgcagaca aagaatccac tcaaaaggca atagatggag tcaccaataa ggtcaactcg 1200
   atcattgaca aaatgaacac tcagtttgag gccgttggaa gggaatttaa caacttagaa 1260
   aggagaatag agaatttaaa caagaagatg gaagacgggt tcctagatgt ctggacttat 1320
   aatgctgaac ttctggttct catggaaaat gagagaactc tagactttca tgactcaaat 1380
   gtcaagaacc tttacgacaa ggtccgacta cagcttaggg ataatgcaaa ggagctgggt 1440
   aacggttgtt tcgagttcta tcataaatgt gataatgaat gtatggaaag tgtaagaaat 1500
   ggaacgtatg actacccgca gtattcagaa gaagcgagac taaaaagaga ggaaataagt 1560
   ggagtaaaat tggaatcaat aggaatttac caaatactgt caatttattc tacagtggcg 1620
   agttccctag cactggcaat catggtagct ggtctatcct tatggatgtg ctccaatgga 1680
   tcgttacaat gcagaatttg catttaa 1707
<210> 78
   <211> 5163
   <212> DNA
   <213> PfMSP1 gene XM_001352134
<400> 78
   atgaagatca tattcttttt atgttcattt ctttttttta ttataaatac acaatgtgta 60
   acacatgaaa gttatcaaga acttgtcaaa aaactagaag ctttagaaga tgcagtattg 120
   acaggttata gtttatttca aaaggaaaaa atggtattaa atgaagaaga aattactaca 180
   aaaggtgcaa gtgctcaaag tggtgcaagt gctcaaagtg gtgcaagtgc tcaaagtggt 240
   gcaagtgctc aaagtggtgc aagtgctcaa agtggtgcaa gtgctcaaag tggtacaagt 300
   ggtccaagtg gtccaagtgg tacaagtcca tcatctcgtt caaacacttt acctcgttca 360
   aatacttcat ctggtgcaag ccctccagct gatgcaagcg attcagatgc taaatcttac 420
   gctgatttaa aacacagagt acgaaattac ttgttcacta ttaaagaact caaatatccc 480
   gaactctttg atttaaccaa tcatatgtta actttgtgtg ataatattca tggtttcaaa 540
   tatttaattg atggatatga agaaattaat gaattattat ataaattaaa cttttatttt 600
   gatttattaa gagcaaaatt aaatgatgta tgtgctaatg attattgtca aatacctttc 660
   aatcttaaaa ttcgtgcaaa tgaattagac gtacttaaaa aacttgtgtt cggatataga 720
   aaaccattag acaatattaa agataatgta ggaaaaatgg aagattacat taaaaaaaat 780
   aaaacaacca tagcaaatat aaatgaatta attgaaggaa gtaagaaaac aattgatcaa 840
   aataagaatg cagataatga agaagggaaa aaaaaattat accaagctca atatgatctt 900
   tctatttaca ataaacaatt agaagaagca cataatttaa taagcgtttt agaaaaacgt 960
   attgacactt taaaaaaaaa tgaaaacata aagaaattac ttgataagat aaatgaaatt 1020
   aaaaatcccc caccggccaa ttctggaaat acaccaaata ctctccttga taagaacaaa 1080
   aaaatcgagg aacacgaaga aaaaataaaa gaaattgcca aaactattaa atttaacatt 1140
   gatagtttat ttactgatcc acttgaatta gaatattatt taagagaaaa aaataaaaaa 1200
   gttgatgtaa cacctaaatc acaagatcct acgaaatctg ttcaaatacc aaaagttcct 1260
   tatccaaatg gtattgtata tcctttacca ctcactgata ttcataattc attagctgca 1320
   gataatgata aaaattcata tggtgattta atgaatcctc atactaaaga aaaaattaat 1380
   gaaaaaatta ttacagataa taaggaaaga aaaatattca ttaataacat taaaaaaaaa 1440
   attgatttag aagaaaaaaa cattaatcac acaaaagaac aaaataaaaa attacttgaa 1500
   gattatgaaa agtcaaaaaa ggattatgaa gaattacttg aaaaatttta tgaaatgaaa 1560
   tttaataata attttaacaa agatgtcgta gataaaatat tcagtgcaag atatacatat 1620
   aatgttgaaa aacaaagata taataataaa ttttcatcct ctaataattc tgtatataat 1680
   gttcaaaaat taaaaaaggc tctttcatat cttgaagatt attctttaag aaaaggaatt 1740
   tctgaaaaag attttaatca ttattatact ttgaaaactg gcctcgaagc tgatataaaa 1800
   aaattaacag aagaaataaa gagtagtgaa aacaaaattc tagaaaaaaa ttttaaagga 1860
   ctaacacatt cagcaaatgg ttccttagaa gtatctgata ttgtaaaatt acaagtacaa 1920
   aaagttttat taattaaaaa aatagaagac ttaagaaaga tagaattatt tttaaaaaat 1980
   gcacaactaa aagatagtat tcatgtacca aatatttata aaccacaaaa taaaccagaa 2040
   ccatattatt taattgtatt aaaaaaagaa gtagataaat taaaagaatt tataccaaaa 2100
   gtaaaagaca tgttaaagaa agaacaagct gtcttatcaa gtattacaca acctttagtt 2160
   gcagcaagcg aaacaactga agatgggggt cactccacac acacattatc ccaatcagga 2220
   gaaacagaag taacagaaga aacagaagaa acagaagaaa cagtaggaca cacaacaacg 2280
   gtaacaataa cattaccacc aacacaacca tcaccaccaa aagaagtaaa agttgttgaa 2340
   aattcaatag aacataagag taatgacaat tcacaagcct tgacaaaaac agtttatcta 2400
   aagaaattag atgaattttt aactaaatca tatatatgtc ataaatatat tttagtatca 2460
   aactctagta tggaccaaaa attattagag gtatataatc ttactccaga agaagaaaat 2520
   gaattaaaat catgtgatcc attagattta ttatttaata ttcaaaataa catacctgct 2580
   atgtattcat tatatgatag tatgaacaat gatttacaac atctcttttt tgaattatat 2640
   caaaaggaaa tgatttatta tttacataaa ctaaaagagg aaaatcacat caaaaaatta 2700
   ttagaggagc aaaaacaaat aactggaaca tcatctacat ccagtcctgg aaatacaacc 2760
   gtaaatactg ctcaatccgc aactcacagt aattcccaaa accaacaatc aaatgcatcc 2820
   tctaccaata cccaaaatgg tgtagctgta tcatctggtc ctgctgtagt tgaagaaagt 2880
   catgatccct taacagtatt gtctattagt aacgatttga aaggtattgt tagtctctta 2940
   aatcttggaa ataaaactaa agtacctaat ccattaacca tttctacaac agagatggaa 3000
   aaattttatg agaatatttt aaaaaataat gatacctatt ttaatgatga tatcaaacaa 3060
   ttcgtaaaat ctaattcaaa agtaattaca ggtttgaccg aaacacaaaa aaatgcatta 3120
   aatgatgaaa ttaaaaaatt aaaagatact ttacagttat catttgattt atataataaa 3180
   tataaattaa aattagatag attatttaat aagaaaaaag aacttggcca agacaaaatg 3240
   caaattaaaa aacttacttt attaaaagaa caattagaat caaaattgaa ttcacttaat 3300
   aacccacata atgtattaca aaacttttct gttttcttta acaaaaaaaa agaagctgaa 3360
   atagcagaaa ctgaaaacac attagaaaac acaaaaatat tattgaaaca ttataaagga 3420
   cttgttaaat attataatgg tgaatcatct ccattaaaaa ctttaagtga agtatcaatt 3480
   caaacagaag ataattatgc caatttagaa aaatttagag tattaagtaa aatagatgga 3540
   aaactcaatg ataatttaca tttaggaaag aaaaaattat ctttcttatc aagtggatta 3600
   catcatttaa ttactgaatt aaaagaagta ataaaaaata aaaattatac aggtaattct 3660
   ccaagtgaaa ataataagaa agttaacgaa gctttaaaat cttacgaaaa ttttctccca 3720
   gaagcaaaag ttacaacagt tgtaactcca cctcaaccag atgtaactcc atctccatta 3780
   tctgtaaggg taagtggtag ttcaggatcc acaaaagaag aaacacaaat accaacttca 3840
   ggctctttat taacagaatt acaacaagta gtacaattac aaaattatga cgaagaagat 3900
   gattccttag ttgtattacc catttttgga gaatccgaag ataatgacga atatttagat 3960
   caagtagtaa ctggagaagc aatatctgtc acaatggata atatcctctc aggatttgaa 4020
   aatgaatatg atgttatata tttaaaacct ttagctggag tatatagaag cttaaaaaaa 4080
   caaattgaaa aaaacatttt tacatttaat ttaaatttga acgatatctt aaattcacgt 4140
   cttaagaaac gaaaatattt cttagatgta ttagaatctg atttaatgca atttaaacat 4200
   atatcctcaa atgaatacat tattgaagat tcatttaaat tattgaattc agaacaaaaa 4260
   aacacacttt taaaaagtta caaatatata aaagaatcag tagaaaatga tattaaattt 4320
   gcacaggaag gtataagtta ttatgaaaag gttttagcga aatataagga tgatttagaa 4380
   tcaattaaaa aagttatcaa agaagaaaag gagaagttcc catcatcacc accaacaaca 4440
   cctccgtcac cagcaaaaac agacgaacaa aagaaggaaa gtaagttcct tccattttta 4500
   acaaacattg agaccttata caataactta gttaataaaa ttgacgatta cttaattaac 4560
   ttaaaggcaa agattaacga ttgtaatgtt gaaaaagatg aagcacatgt taaaataact 4620
   aaacttagtg atttaaaagc aattgatgac aaaatagatc tttttaaaaa cccttacgac 4680
   ttcgaagcaa ttaaaaaatt gataaatgat gatacgaaaa aagatatgct tggcaaatta 4740
   cttagtacag gattagttca aaattttcct aatacaataa tatcaaaatt aattgaagga 4800
   aaattccaag atatgttaaa catttcacaa caccaatgcg taaaaaaaca atgtccagaa 4860
   aattctggat gtttcagaca tttagatgaa agagaagaat gtaaatgttt attaaattac 4920
   aaacaagaag gtgataaatg tgttgaaaat ccaaatccta cttgtaacga aaataatggt 4980
   ggatgtgatg cagatgccac atgtaccgaa gaagattcag gtagcagcag aaagaaaatc 5040
   acatgtgaat gtactaaacc tgattcttat ccacttttcg atggtatttt ctgcagttcc 5100
   tctaacttct taggaatatc attcttatta atactcatgt taatattata cagtttcatt 5160
   taa 5163
<210> 79
   <211> 654
   <212> DNA
   <213> Pfs25 gene XM_001347551
<400> 79
   atgaataaac tttacagttt gtttcttttc cttttcattc aacttagcat aaaatataat 60
   aatgcgaaag ttaccgtgga tactgtatgc aaaagaggat ttttaattca gatgagtggt 120
   catttggaat gtaaatgtga aaatgatttg gtgttagtaa atgaagaaac atgtgaagaa 180
   aaagttctga aatgtgacga aaagactgta aataaaccat gtggagattt ttccaaatgt 240
   attaaaatag atggaaatcc cgtttcatac gcttgtaaat gtaatcttgg atatgatatg 300
   gtaaataatg tttgtatacc aaatgaatgt aagaatgtaa cttgtggtaa cggtaaatgt 360
   atattagata caagcaatcc tgttaaaact ggagtttgct catgtaatat aggcaaagtt 420
   cccaatgtac aagatcaaaa taaatgttca aaagatggag aaaccaaatg ctcattaaaa 480
   tgcttaaaag aaaatgaaac ctgtaaagct gttgatggaa tttataaatg tgattgtaaa 540
   gatggattta taatagataa tgaaagctct atatgtactg ctttttcagc atataatatt 600
   ttaaatctaa gcattatgtt tatactattt tcagtatgct tttttataat gtaa 654

## Description

### TECHNICAL FIELD

The present disclosure provides a novel transfer vector, a recombinant baculovirus obtained by homologous recombination of the transfer vector and a baculovirus DNA, and methods for production thereof.

The present disclosure further provides pharmaceuticals (e.g., vaccines, and preventive or therapeutic drugs for infectious diseases such as malaria and influenza) containing the recombinant baculovirus as an active ingredient.

### BACKGROUND ART

Baculovirus has been used as a vector in methods of industrially producing a desired protein using insect cells. In recent years, it has been found that baculovirus can introduce a foreign gene not only into insect cells but also into mammalian cells, and the possibility of their use as a vector for introducing a therapeutic gene has been found. Patent Document 1 discloses a recombinant baculovirus expression vector containing multiple independent promoters comprising a promoter derived from an early gene of baculovirus, which has a DNA region containing a gene encoding a viral non-structural protein, and a promoter derived from a late gene of baculovirus, which has a DNA region containing a gene encoding a viral structural protein.

Patent Document 2 discloses a method comprising introducing into mammalian cells a non-mammalian DNA viral vector containing multiple independent promoters controlled to express a desired foreign gene linked to the promoters; and expressing the foreign gene in the mammalian cells.

Further, Patent Document 3 discloses a method of producing a protein with gene recombination technology using a baculovirus. The method comprises expressing a fusion gene obtained by linking the gp64 gene of baculovirus to a gene encoding a desired protein to produce the desired protein in a form fused with viral particles, collecting the viral particles fused with the desired protein, and cleaving the desired protein from the viral particles to collect the protein.

With respect to a baculovirus expression system, Patent Document 4 discloses a recombinant baculovirus expression vector having multiple independent promoters containing a first nucleic acid sequence encoding a detection marker, which is linked, in a form capable of functioning, to a first promoter that is active in host cells and inactive in non-acceptable cells, and a second nucleic acid sequence containing a foreign nucleic acid sequence, which is linked, in a form capable of functioning, to a second promoter that is active in non-acceptable cells.

Patent Document 5 discloses that an influenza virus hemagglutinin (HA) antigen-expressing recombinant baculovirus vector linked to a CAG promoter derived from chicken β actin has influenza virus infection-preventing effects and is therefore useful as a vaccine formulation.

Patent Document 6 discloses a method of producing a baculovirus vector comprising the step of co-transfecting into insect cells a plasmid containing a baculovirus promoter and a mammalian cell-derived promoter having linked thereto a gene encoding a protein expressible on the cell surface, or a plasmid containing two baculovirus promoters having linked thereto a gene encoding a protein expressible on the cell surface has been linked.

Patent Document 7 describes research on anti-influenza virus activity, i.e., activity against influenza virus infection, of a recombinant baculovirus having cDNA of influenza virus HA incorporated in a CAG promoter, and discloses that not only the recombinant baculovirus but also wild-type baculovirus have anti-influenza virus activity.

As shown above, various recombinant baculovirus vectors have been developed in recent years, and many attempts have been made to develop pharmaceuticals for mammals containing such a recombinant baculovirus vector as an active ingredient.

In this technical field, the development of pharmaceutical formulations, particularly vaccine formulations containing, as an active ingredient, a novel recombinant baculovirus, which is a recombinant baculovirus vector having a novel structure and being effective against malaria, influenza, tuberculosis, and like infectious diseases, or diseases such as cancer, has been desired.
Patent Document 1: Japanese Patent No. 3366328, Multiple Promoter Baculovirus Expression System and Defect Particle Products.
Patent Document 2: WO98/011243, Non-mammalian DNA Virus Having Modified Coating Protein.
Patent Document 3: JP No. 2002-235236-A, Methods of Producing Proteins.
Patent Document 4: JP No. 2003-284557-A, Novel Baculovirus-Transfecting Vector and Recombinant Baculovirus for Expression of Foreign Gene.
Patent Document 5: WO02/062381, Baculovirus Vector Vaccine.
Patent Document 6: WO04/029259, Baculovirus Vector, Method of Producing Baculovirus Vector, and Method of Introducing Gene.
Patent Document 7: JP No. 2005-15346-A, Baculovirus-containing Anti-viral Agent.
Patent Document 8: EP1983047 (2008-10-22)

Available only as Article 54(3) EPC prior art.

### BRIEF DESCRIPTION OF THE DRAWINGS

FIG. 1 shows the test results of the expression of vaccine antigens from recombinant baculoviruses of the present invention in insect cells in Example 3.
   Detected by gp64 Monoclonal Antibody (AcV5)
   Lane 1: AcNPV-WT
   Lane 2: AcNPV-CAP-PfCSP
   Lane 3: AcNPV-CAP-HA1/Anhui
   Lane 4: AcNPV-CAP-HA1/Vietnam
FIG. 2 (A) shows a Western blotting analysis showing the expression of the CSP gene (PfCSP) of human malaria in viral particles of recombinant baculoviruses produced from recombinant transfer vectors.
   Detected by Anti-PfCSP Monoclonal Antibody (2A10)
   Lane 1: AcNPV-CAP-PfCSP
   Lane 2: AcNPV-CAP-PfCSP/272
   Lane 3: AcNPV-CAP-PfCSP/467
FIG. 2 (B) shows a Western blotting analysis showing the expression of H5N1/HA1 gene in viral particles of recombinant baculoviruses produced from recombinant transfer vectors.
   Detected by H5N1/Vietnam Rabbit Polyclonal Antibody (IT-003-005)
   Lane 1: AcNPV-WT (Cell Lysate)
   Lane 2: AcNPV-CAP-HA1/Anhui (Cell Lysate)
   Lane 3: AcNPV-CAP-HA1/Vietnam (Cell Lysate)
   Lane 4: AcNPV-WT (Virion)
   Lane 5: AcNPV-CAP-HA1/Vietnam (Virion)
   Lane 6: Purified H5N1/Anhui Antigen (IT-003-0053p)

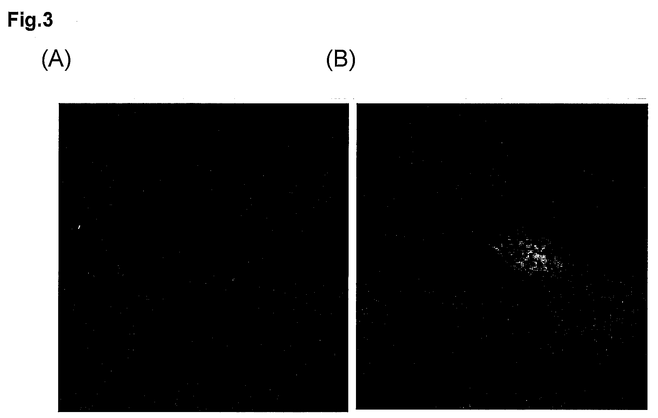

FIG. 3 shows HepG2 cells stained with a fluorescence-labeled antibody, which indicates that an antigen has been expressed by a recombinant baculovirus containing a fusion gene of PfMSP1 gene and PfCSP gene in the HepG2 cells. The results of FIG. 3 (A) confirmed that a PfCSP antigen has been expressed. The results of FIG. 3 (B) confirmed that a PfMSP-1₁₉ antigen has been expressed.

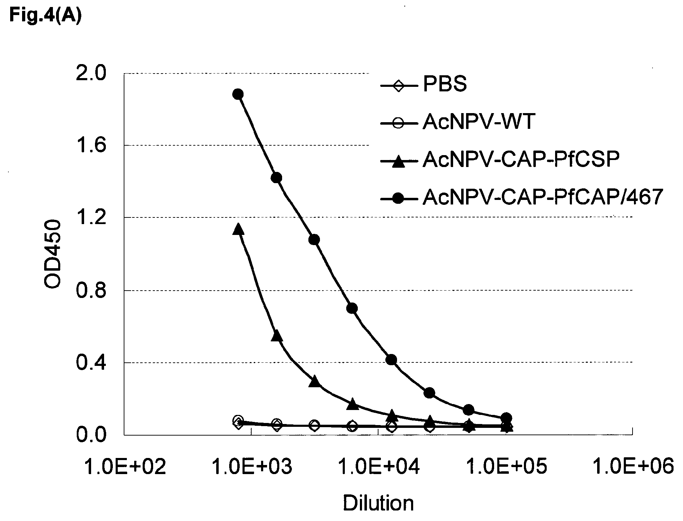

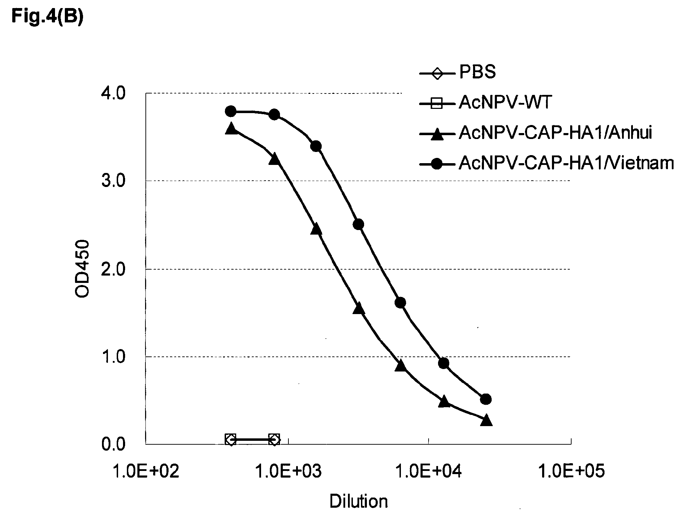

FIG. 4 shows the results of measurement of antibody titers obtained in Example 6.

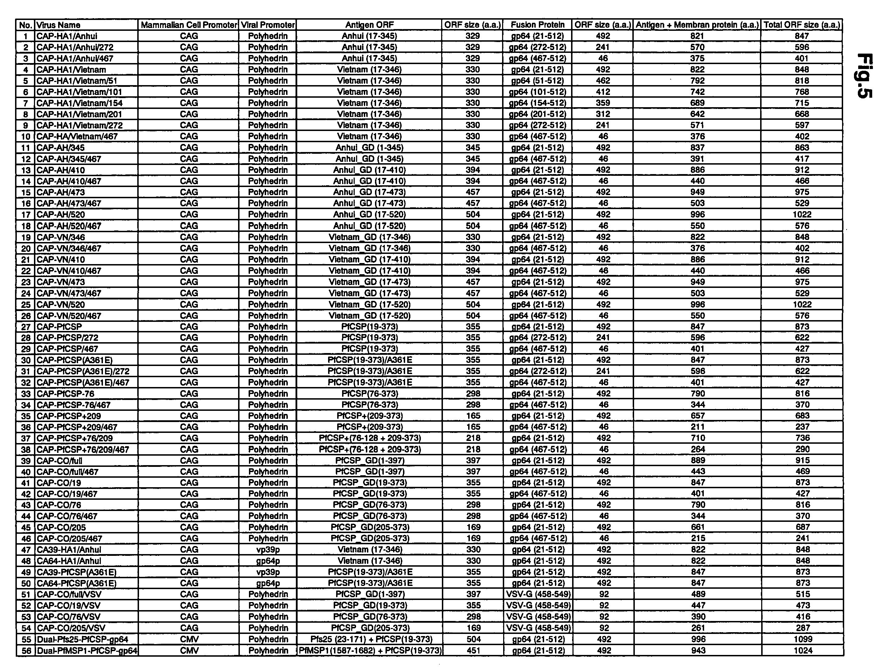

FIG. 5 shows the transfer vectors of the present invention.

### DISCLOSURE OF THE INVENTION

### PROBLEMS TO BE SOLVED BY THE INVENTION

An object of the present disclosure is to provide a novel recombinant transfer vector, a recombinant baculovirus obtained by homologous recombination of the recombinant transfer vector and a baculovirus DNA, and methods for production thereof. Another object of the present invention is to provide a pharmaceutical preparation as claimed, particularly a vaccine formulation containing the recombinant baculovirus as an active ingredient.

### MEANS FOR SOLVING THE PROBLEMS

The present inventors found a transfer vector having a novel structure capable of expressing a fusion protein of a protein having a desired immunogenicity or a partial protein thereof with a viral structural protein in insect cells and in vertebrate (particularly mammalian and avian) cells; and a recombinant baculovirus obtained by homologous recombination of the transfer vector and a baculovirus DNA. Using the obtained recombinant baculovirus, the present inventors carried out extensive research on pharmaceuticals containing as an active ingredient a recombinant baculovirus having infectious disease-preventive and therapeutic effects. As a result, the inventors found that the obtained recombinant baculovirus has the desired pharmaceutical effects.

Thus, the inventors of the present invention found a recombinant transfer vector having a novel structure, a recombinant baculovirus obtained by homologous recombination of the transfer vector and a baculovirus DNA, and methods for the production thereof, and ascertained that the recombinant baculovirus itself is useful as a pharmaceutical capable of expressing a protein having the desired immunogenicity in target cells, and as a pharmaceutical for preventing infectious diseases such as malaria and influenza. The present invention has been accomplished, based on these findings.

The present invention provides the inventions shown in the following items 1 to 2:
[1] A pharmaceutical composition comprising a recombinant Autographa californica nucleopolyhedrosis virus (AcNPV) as an active agent and, optionally, a pharmaceutically acceptable carrier,
   wherein the recombinant AcNPV contains a gene structure having a fusion gene of
   (A) a gene encoding an amino acid sequence consisting of amino acids 19-373 of SEQ ID NO:70 (Plasmodium falciparum CircumSporozoite Protein; PfCSP), and
   (B) a gene encoding an amino acid sequence consisting of amino acids 21-512 of gp64 viral particle protein (gp64) encoded in the AcNPV genome (GenBank Accession No. L22858) under the control of a dual promoter comprising Polyhedrin promoter and CAG promoter linked to each other (CAP).
[2] A pharmaceutical composition of [1], which is a malaria vaccine.

Also disclosed herein are:
Item 1. A transfer vector which is any one of pCAP-PfCSP, pCAP-PfCSP/272, pCAP-PfCSP/467, pCAP-PfCSP(A361E), pCAP-PfCSP(A361E)/272, pCAP-PfCSP(A361E)/467, pCAP-PfCSP-76, pCAP-PfCSP-76/467, pCAP-PfCSP+209, pCAP-PfCSP+209/467, pCAP-PfCSP+76/209, pCAP-PfCSP+76/209/467, pCAP-HA1/Anhui, pCAP-HAl/Anhui/272, pCAP-HA1/Anhui/467, pCAP-HA1/Vietnam, pCAP-HAl/Vietnam/51, pCAP-HAl/Vietnam/101, pCAP-HA1/Vietnam/154, pCAP-HAl/Vietnam/201, pCAP-HA1/Vietnam/272, pCAP-HA1/Vietnam/467, pCAP-AH/345, pCAP-AH/345/467, pCAP-AH/410, pCAP-AH/410/467, pCAP-AH/473, pCAP-AH/473/467, pCAP-AH/520, pCAP-AH/520/467, pCAP-VN/346, pCAP-VN/346/467, pCAP-VN/410, pCAP-VN/410/467, pCAP-VN/473, pCAP-VN/473/467, pCAP-VN/520, pCAP-VN/520/467, pCAP-CO/full, pCAP-CO/full/467, pCAP-CO/19, pCAP-CO/19/467, pCAP-CO/76, pCAP-CO/76/467, pCAP-CO/205, pCAP-CO/205/467, pCA39-HA1/Anhui, pCA64-HA1/Anhui, pCA39-PfCSP(A361E), pCA64-PfCSP(A361E), pCAP-CO/full/VSV, pCAP-CO/19/VSV, pCAP-CO/76/VSV, pCAP-CO/205/VSV, pDual-Pfs25-PfCSP-gp64, and pDual-PfMSP1-PfCSP-gp64.
Item 2. A recombinant baculovirus which is any one of AcNPV-CAP-PfCSP, AcNPV-CAP-PfCSP/272, AcNPV-CAP-PfCSP/467, AcNPV-CAP-PfCSP(A361E), AcNPV-CAP-PfCSP(A361E)/272, AcNPV-CAP-PfCSP(A361E)/467, AcNPV-CAP-PfCSP-76, AcNPV-CAP-PfCSP-76/467, AcNPV-CAP-PfCSP+209, AcNPV-CAP-PfCSP+209/467, AcNPV-CAP-PfCSP+76/209, AcNPV-CAP-PfCSP+76/209/467, AcNPV-CAP-HA1/Anhui, AcNPV-CAP-HA1/Anhui/272, AcNPV-CAP-HA1/Anhui/467, AcNPV-CAP-HA1/Vietnam, AcNPV-CAP-HA1/Vietnam/51, AcNPV-CAP-HA1/Vietnam/101, AcNPV-CAP-HA1/Vietnam/154, AcNPV-CAP-HA1/Vietnam/201, AcNPV-CAP-HA1/Vietnam/272, AcNPV-CAP-HA1/Vietnam/467, AcNPV-CAP-AH/345, AcNPV-CAP-AH/345/467, AcNPV-CAP-AH/410, AcNPV-CAP-AH/410/467, AcNPV-CAP-AH/473, AcNPV-CAP-AH/473/467, AcNPV-CAP-AH/520, AcNPV-CAP-AH/520/467, AcNPV-CAP-VN/346, AcNPV-CAP-VN/346/467, AcNPV-CAP-VN/410, AcNPV-CAP-VN/410/467, AcNPV-CAP-VN/473, AcNPV-CAP-VN/473/467, AcNPV-CAP-VN/520, AcNPV-CAP-VN/520/467, AcNPV-CAP-CO/full, AcNPV-CAP-CO/full/467, AcNPV-CAP-CO/19, AcNPV-CAP-CO/19/467, AcNPV-CAP-CO/76, AcNPV-CAP-CO/76/467, AcNPV-CAP-CO/205, AcNPV-CAP-CO/205/467, AcNPV-CA39-HA1/Anhui, AcNPV-CA64-HA1/Anhui, AcNPV-CA39-PfCSP(A361E), AcNPV-CA64-PfCSP(A361E), AcNPV-CAP-CO/full/VSV, AcNPV-CAP-CO/19/VSV, AcNPV-CAP-CO/76/VSV, AcNPV-CAP-CO/205/VSV, AcNPV-Dual-Pfs25-PfCSP-gp64, and AcNPV-Dual-PfMSP1-PfCSP-gp64.
Item 3. A composition for infectious diseases comprising the recombinant baculovirus of item 2 as an active ingredient.
Item 4. A composition for infectious diseases comprising the recombinant baculovirus of item 2 as an active ingredient, the composition being administered by the intramuscular, respiratory, or nasal route.
Item 5. A vaccine comprising the recombinant baculovirus of item 2 as an active ingredient.
Item 6. A vaccine comprising the recombinant baculovirus of item 3 as an active ingredient, the composition being administered by the intramuscular, respiratory, or nasal route.
Item 7. A therapeutic or preventive agent for influenza virus infection, comprising as an active ingredient any one of AcNPV-CAP-HA1/Anhui, AcNPV-CAP-HA1/Anhui/272, AcNPV-CAP-HA1/Anhui/467, AcNPV-CAP-HA1/Vietnam, AcNPV-CAP-HA1/Vietnam/51, AcNPV-CAP-HA1/Vietnam/101, AcNPV-CAP-HA1/Vietnam/154, AcNPV-CAP-HAl/Vietnam/201, AcNPV-CAP-HA1/Vietnam/272, AcNPV-CAP-HA1/Vietnam/467, AcNPV-CAP-AH/345, AcNPV-CAP-AH/345/467, AcNPV-CAP-AH/410, AcNPV-CAP-AH/410/467, AcNPV-CAP-AH/473, AcNPV-CAP-AH/473/467, AcNPV-CAP-AH/520, AcNPV-CAP-AH/520/467, AcNPV-CAP-VN/346, AcNPV-CAP-VN/346/467, AcNPV-CAP-VN/410, AcNPV-CAP-VN/410/467, AcNPV-CAP-VN/473, AcNPV-CAP-VN/473/467, AcNPV-CAP-VN/520, AcNPV-CAP-VN/520/467, AcNPV-CA39-HA1/Anhui, and AcNPV-CA64-HA1/Anhui.
Item 8. A therapeutic or preventive agent for influenza virus infection according to item 7, which is administered by the intramuscular, respiratory, or nasal route.
Item 9. A vaccine against influenza virus infection, comprising as an active ingredient any one of AcNPV-CAP-HA1/Anhui, AcNPV-CAP-HA1/Anhui/272, AcNPV-CAP-HA1/Anhui/467, AcNPV-CAP-HA1/Vietnam, AcNPV-CAP-HA1/Vietnam/51, AcNPV-CAP-HA1/Vietnam/101, AcNPV-CAP-HA1/Vietnam/154, AcNPV-CAP-HA1/Vietnam/201, AcNPV-CAP-HA1/Vietnam/272, AcNPV-CAP-HA1/Vietnam/467, AcNPV-CAP-AH/345, AcNPV-CAP-AH/345/467, AcNPV-CAP-AH/410, AcNPV-CAP-AH/410/467, AcNPV-CAP-AH/473, AcNPV-CAP-AH/473/467, AcNPV-CAP-AH/520, AcNPV-CAP-AH/520/467, AcNPV-CAP-VN/346, AcNPV-CAP-VN/346/467, AcNPV-CAP-VN/410, AcNPV-CAP-VN/410/467, AcNPV-CAP-VN/473, AcNPV-CAP-VN/473/467, AcNPV-CAP-VN/520, AcNPV-CAP-VN/520/467, AcNPV-CA39-HAl/Anhui, and AcNPV-CA64-HA1/Anhui.
Item 10. A vaccine against influenza virus infection according to item 7, which is administered by the intramuscular, respiratory, or nasal route.
Item 11. A therapeutic or preventive agent for human malaria infection, comprising as an active ingredient any one of AcNPV-CAP-PfCSP, AcNPV-CAP-PfCSP/272, AcNPV-CAP-PfCSP/467, AcNPV-CAP-PfCSP(A361E), AcNPV-CAP-PfCSP(A361E)/272, AcNPV-CAP-PfCSP(A361E)/467, AcNPV-CAP-PfCSP-76, AcNPV-CAP-PfCSP-76/467, AcNPV-CAP-PfCSP+209, AcNPV-CAP-PfCSP+209/467, AcNPV-CAP-PfCSP+76/209, AcNPV-CAP-PfCSP+76/209/467, AcNPV-CAP-CO/full, AcNPV-CAP-CO/full/467, AcNPV-CAP-CO/19, AcNPV-CAP-CO/19/467, AcNPV-CAP-CO/76, AcNPV-CAP-CO/76/467, AcNPV-CAP-CO/205, AcNPV-CAP-CO/205/467, AcNPV-CA39-PfCSP(A361E), AcNPV-CA64-PfCSP(A361E), AcNPV-CAP-CO/full/VSV, AcNPV-CAP-CO/19/VSV, AcNPV-CAP-CO/76/VSV, AcNPV-CAP-CO/205/VSV, AcNPV-Dual-Pfs25-PfCSP-gp64, and AcNPV-Dual-PfMSP1-PfCSP-gp64.
Item 12. A therapeutic or preventive agent for human malaria infection according to item 11, which is administered by the intramuscular, respiratory, or nasal route.
Item 13. A therapeutic or preventive agent for human malaria infection, comprising as an active ingredient any one of AcNPV-CAP-PfCSP, AcNPV-CAP-PfCSP/272, AcNPV-CAP-PfCSP/467, AcNPV-CAP-PfCSP(A361E), AcNPV-CAP-PfCSP(A361E)/272, AcNPV-CAP-PfCSP(A361E)/467, AcNPV-CAP-PfCSP-76, AcNPV-CAP-PfCSP-76/467, AcNPV-CAP-PfCSP+209, AcNPV-CAP-PfCSP+209/467, AcNPV-CAP-PfCSP+76/209, AcNPV-CAP-PfCSP+76/209/467, AcNPV-CAP-CO/full, AcNPV-CAP-CO/full/467, AcNPV-CAP-CO/19, AcNPV-CAP-CO/19/467, AcNPV-CAP-CO/76, AcNPV-CAP-CO/76/467, AcNPV-CAP-CO/205, AcNPV-CAP-CO/205/467, AcNPV-CA39-PfCSP(A361E), AcNPV-CA64-PfCSP(A361E), AcNPV-CAP-CO/full/VSV, AcNPV-CAP-CO/19/VSV, AcNPV-CAP-CO/76/VSV, AcNPV-CAP-CO/205/VSV, AcNPV-Dual-Pfs25-PfCSP-gp64, and AcNPV-Dual-PfMSP1-PfCSP-gp64.
Item 14. A therapeutic or preventive agent for human malaria infection according to item 13, which is administered by the intramuscular, respiratory, or nasal route.
Item 15. A method of preventing malaria or influenza infection or of treating malaria or influenza, comprising administering to a subject an effective amount of the recombinant baculovirus of item 2, the composition for infectious diseases of item 3 or 4, or the vaccine of item 5, 6, 9, 10, 13 or 14.
Item 16. A method according to item 15, wherein the recombinant baculovirus, composition, or vaccine is administered to the subject as a liposomal formulation.
Item 17. A method according to item 15, wherein the recombinant baculovirus, composition, or vaccine is administered to the subject by the intramuscular, respiratory, or nasal route.
Item 18. A method according to item 16, wherein the recombinant baculovirus, composition, or vaccine is administered to the subject by the intramuscular, respiratory, or nasal route.
Item 19. A method of immunostimulation comprising administering to a subject an effective amount of the recombinant baculovirus of item 2, the composition for infectious diseases of item 3 or 4, or the vaccine of item 5, 6, 9, 10, 13 or 14.
Item 20. A method according to item 19, wherein the recombinant baculovirus, composition, or vaccine is administered to the subject as a liposomal formulation.
Item 21. A method according to item 19, wherein the recombinant baculovirus, composition, or vaccine is administered to the subject by the intramuscular, respiratory, or nasal route.
Item 22. A method according to item 20, wherein the recombinant baculovirus, composition, or vaccine is administered to the subject by the intramuscular, respiratory, or nasal route.

### EFFECT OF THE INVENTION

According to the present disclosure, a novel recombinant transfer vector, a recombinant baculovirus obtained by homologous recombination of the recombinant transfer vector and a baculovirus DNA, and methods for production thereof are provided. Pharmaceuticals containing the recombinant baculovirus of the present disclosure as an active ingredient are useful as therapeutic or preventive drugs for infectious diseases such as malaria and influenza, or as cellular medicine and vaccine formulations.

### BEST MODE FOR CARRYING OUT THE INVENTION

The abbreviations used for the amino acids, peptides, base sequences, and nucleic acids in the present specification are based on the abbreviations specified in the IUPAC-IUB Communication on Biochemical Nomenclature, Eur. J. Biochem., 138: 9 (1984) and "Guideline for Preparing Specifications Including Base Sequences and Amino Acid Sequences" (Patent Office), and those commonly used in this technical field. In the present specification, the DNA molecule may include not only double strand DNA but also single strand DNA, i.e., a sense chain and an antisense chain constituting the double strand DNA, and is not limited to a full length thereof. The polynucleotide (DNA molecule) encoding the immunogenic foreign gene of the present disclosure encompasses double strand DNA containing genomic DNA, single strand DNA (sense chain) containing cDNA, and single strand DNA (antisense chain) having a sequence complementary to the sense chain, synthetic DNA, and fragments thereof, unless otherwise mentioned.

The polynucleotide or DNA molecule used herein is not limited in the functional region, and may include at least one of an expression suppression region, a coding region, a leader sequence, an exon, and an intron.

Further, examples of the polynucleotide include RNA and DNA. The polypeptide containing a specific amino acid sequence and the polynucleotide containing a specific DNA sequence include fragments, homologs, derivatives, and mutants of the polynucleotide.

Examples of mutants of the polynucleotide, such as mutant DNA, include naturally occurring allelic mutants; artificial mutants; and mutants having deletion, substitution, addition, and/or insertion. However, it should be understood that such mutants encode polypeptides having substantially the same function as the polypeptide encoded by the original non-mutated polynucleotide.

In the present invention, the transfer vector refers to a plasmid for producing a recombinant baculovirus having a structure in which a fusion gene, formed by linking at least one gene encoding a protein capable of being a component of viral particles to at least one immunogenic foreign gene, is incorporated downstream of a dual promoter comprising two linked promoters, i.e., one vertebrate promoter (e.g., a mammalian promoter or an avian promoter) and one baculovirus promoter.

In one preferable embodiment of the invention, the immunogenic foreign gene is located downstream of the dual promoter and upstream of the gene encoding a protein capable of being a component of viral particles. The recombinant baculovirus of the present disclosure is used as an active ingredient of pharmaceuticals or vaccines for vertebrates. Examples of vertebrates include mammals such as humans, cattle, horses, swine, sheep, goats, monkeys, mice, dogs, and cats. Examples of birds include chickens, quail, geese, wild ducks, pigeons, turkeys, guinea fowls, and parrots.

In one embodiment, the present disclosure provides a transfer vector containing a novel structure having incorporated therein a fusion gene of one immunogenic foreign gene and a gene encoding a viral membrane protein that can be expressed in insect cells under the control of a dual promoter comprising one vertebrate promoter and one baculovirus promoter linked to each other. Co-transfection of this transfer vector with a baculovirus DNA into insect cells induces a homologous recombination and thereby produces a recombinant baculovirus having incorporated therein the fusion gene that is expressed under the control of a baculovirus promoter in insect cells and that can produce a fusion protein capable of being a component of budded viral particles.

In the present invention, when the recombinant baculovirus is administered to a vertebrate, a fusion protein of a protein capable of being a component of budded viral particles with an immunogenic protein seems to function as a component vaccine. Further, the recombinant baculovirus administered to the vertebrate enters the vertebrate cell, and a fusion antigen with the desired immunogenic foreign antigen is produced from the viral genome in the vertebrate cell, and functions as a DNA vaccine.

Thus, for example, when the recombinant baculovirus of the present disclosure is administered to a mammal, a fusion protein of a protein capable of being a component of viral particles with an immunogenic protein is presented as an antigen on the surface of virus particles, and a fusion protein of a protein capable of being a component of viral particles with an immunogenic protein is produced in the mammalian cell, and seems to function as a preventive or therapeutic agent for infectious diseases, such as viral, protozoan, and bacterial infection, due to its immunopotentiative action.

The baculovirus DNA to be co-transfected with the transfer vector may be a wild type, mutant, or recombinant baculovirus DNA. Examples of host cells to be co-transfected include insect cells such as cells of *Spodoptera frugiperda.*

In the present disclosure, the immunogenic foreign gene refers to a gene encoding the amino acid sequence of an antigenic protein used as an immunogen in immunotherapy, such as vaccine therapy, for the prevention and treatment of infectious diseases, such as malaria and influenza. Specific examples thereof include genes encoding the amino acid sequences of proteins, such as malaria antigen and influenza virus antigen.

The "foreign" gene as used herein refers to a gene introduced from the outside. Even if the same gene is present in the cell, the gene introduced from the outside is referred to as the "foreign" gene.

In the present disclosure, the gene encoding the amino acid sequence of a protein used as the immunogen is not particularly limited, as long as the gene is capable of encoding the amino acid sequence of an antigenic protein having immunogenicity against a substance that causes diseases such as infectious diseases. Examples of genes encoding the amino acid sequence of an antigenic protein having immunogenicity are as follows.

Examples of genes encoding the amino acid sequence of a malaria antigen include genes encoding the amino acid sequences of sporozoite surface antigen CSP (Circumsporozoite Protein) of malaria parasites, merozoite surface membrane protein MSP1 (Merozoite Surface Protein 1), malaria S antigen secreted from erythrocytes infected with malaria, PfEMP1 protein present in the knobs of erythrocytes infected with malaria, SERA protein, TRAMP protein, AMA1 protein, and like proteins.

Examples of genes encoding the amino acid sequences of influenza virus antigens include genes encoding the amino acid sequences of HA antigen (hemagglutinin antigen), NA antigen (neuraminidase antigen), M2 antigen (matrix protein antigen), NP antigen (nucleoprotein antigen), and like proteins.

Of the vertebrate genes, examples of mammalian genes include genes encoding the amino acid sequences of antigenic proteins of infectious diseases in humans, cattle, horses, swine, sheep, monkeys, mice, dogs, and cats. Examples of avian genes include antigen genes (e.g., avian influenza HA antigen) of infectious diseases in chickens, wild ducks, pigeons, turkeys, guinea fowls, and parrots.

Pathogen genes whose association with infectious diseases in mammals and birds as mentioned above has been reported are easily available from institutions storing registered public data on pathogen genes, such as Genbank.

The present disclosure can provide a transfer vector having such an immunogenic foreign gene, and a recombinant baculovirus obtained by homologous recombination of the transfer vector. Further, the present invention can provide a pharmaceutical composition as claimed containing as an active ingredient the recombinant baculovirus having the immunogenic foreign gene, and a vaccine formulation of the pharmaceutical composition.

The baculovirus used in the present disclosure refers to insect pathogen viruses that cause infection in insects, which are one group (*Baculoviridae*) of DNA viruses having a cyclic double strand DNA as a gene. Among these, one group of the viruses referred to as the nucleopolyhedrosis virus (NPV) produce an inclusion body, referred to as a polyhedron, in the nucleus of infected cells in the late phase of infection. Even if a foreign gene to be expressed is inserted in place of the polyhedrin gene, virus infection and growth sufficiently occur to produce a large amount of the desired foreign gene product. Therefore, this virus has been used for the production of desired proteins in recent years.

Examples of the baculovirus used in the present disclosure include *Autographa californica nucleopolyhedrosis* virus: AcNPV, *Bombyx mori nucleopolyhedrosis* virus: BmNPV, *Orgyia pseudotsugata nucleopolyhedrosis* virus: OpNPV, and *Lymantria disper nucleopolyhedrosis* virus: LdNPV.

The baculovirus DNA may be any DNA that can be homologously recombined with the transfer vector of the present disclosure. More specifically, the viral gene of the baculovirus DNA that can be homologously recombined with the transfer vector of the present disclosure is as huge as 130 kbp in size, and an immunogenic foreign gene of 15 kbp or more can be inserted therein. Because the baculovirus gene itself is scarcely expressed in vertebrate cells, there is almost no need to consider its cytotoxicity. Thus, it is thought that no harmful immune response is induced.

### (1) Transfer Vector and Production of the Transfer Vector used in the Present Invention

### Production of Immunogenic Foreign Gene DNA

The immunogenic foreign gene DNA capable of being fused to the viral gene, which is one of the components of the baculovirus transfer vector, can be easily produced or obtained by synthesis based on the nucleic acid sequence information of the polynucleotide encoding the amino acid sequence of an antigenic protein having the desired immunogenicity disclosed in this specification, or directly synthesizing the DNA corresponding to the nucleic acid sequence of the coding region of the immunogenic foreign gene, based on the nucleic acid sequence information of the immunogenic foreign gene (chemical DNA synthesis method). General gene engineering techniques can be used for this production (see, for example, "Molecular Cloning" 2d Ed, Cold Spring Harbor Lab. Press (1989); Zoku Seikagaku Jikken Kouza, "Idenshi Kenkyuho I, II, III", edited by the Japanese Biochemistry Society, 1986).

Examples of methods for synthesizing the DNA include chemical synthesis means, such as the phosphate triester method and the phosphate amidite method (J. Am. Chem. Soc., 89, 4801 (1967); ibid., 91, 3350 (1969); Science, 150, 178 (1968); Tetrahedron Lett., 22, 1859 (1981); ibid., 24, 245 (1983)) and combination methods thereof. The DNA can also be chemically synthesized by the phosphoramidite method or the triester method, or synthesized by using a commercially available automatic oligonucleotide synthesizer. A double strand fragment can be obtained by synthesizing a complementary strand and annealing the complementary strand with a chemically synthesized single strand under appropriate conditions, or adding the complementary strand with appropriate primer sequences to a chemically synthesized single strand using a DNA polymerase.

Specific examples of the immunogenic foreign gene DNA produced in the present disclosure include DNA comprising a DNA sequence encoding the amino acid sequence of a malaria antigen protein, and a DNA sequence encoding the amino acid sequence of an influenza virus antigen protein.

The DNA used in the present disclosure is not limited to a full-length DNA sequence encoding the amino acid sequence of the polypeptide of an antigenic protein having the immunogenicity, and may be a DNA sequence encoding a partial sequence thereof, as long as the protein of the amino acid sequence encoded by the DNA sequence has the immunogenicity.

The DNA of the immunogenic foreign gene used in the present disclosure is not limited to DNA molecules having such a specific DNA sequence, and may be DNA molecules having a DNA sequence produced by suitably selecting codons for each amino acid residue. The choice of codons can be made in accordance with a standard method, for example, by considering the frequency of codon use in the host used (Nucleic Acids Res., 9, 43(1981)).

The DNA of the immunogenic foreign gene used in the present invention can be produced by gene engineering techniques, more specifically by preparing a cDNA library from an appropriate origin that expresses the DNA of an immunogenic foreign gene in accordance with a standard method, and selecting a desired clone from the library using an appropriate probe or antibody against the expressed product, which is specific to the immunogenic foreign gene (see, for example, Proc. Natl. Acad. Sci., USA., 78, 6613 (1981); Science, 222, 778 (1983)).

Examples of the genomic DNA origin include various cells, tissues, and cultured cells derived therefrom, which express the DNA of the immunogenic foreign gene. Extracts of erythrocytes infected with malaria parasites, and extracts of cells infected with influenza viruses are particularly preferable. The extraction and separation of total DNA and RNA from the origin, separation and purification of mRNA, and the production and cloning of cDNA can be performed in accordance with standard methods.

As described above, the DNA of the immunogenic foreign gene can be produced by using the cDNA library of each immunogen prepared by extraction, separation and purification of the mRNA of the immunogenic tissue or cells from the above-mentioned extract. The DNA of the immunogenic foreign gene can also be produced by using a phage library prepared by extracting the mRNA of each immunogen, adding poly A to the RNA, collecting the poly A-added RNA, producing cDNA using a reverse transcriptase, adding restriction enzyme sites to both ends of the cDNA, and incorporating the cDNA into a phage.

The method of screening the immunogenic foreign gene DNA from the cDNA library is not particularly limited, and can be performed in accordance with a usual method. Specific examples of such methods include a method of selecting a corresponding cDNA clone by immunological screening using a specific antibody (e.g., anti-malaria antibody, anti-influenza virus antibody, etc.) against the protein produced by the cDNA; a plaque hybridization method using a probe selectively binding to the target DNA sequence; a colony hybridization method; and combinations thereof.

The probe used in such hybridization methods is usually a DNA fragment chemically synthesized based on the information on the DNA sequence of the immunogenic foreign gene. The DNA sequences of the immunogenic foreign gene already obtained or fragments thereof can also be advantageously used as the probe. A sense primer and an antisense primer designed based on the DNA sequence information of the immunogenic foreign gene can also be used as the probe for the screening.

The DNA (nucleotides) used as the probe is a partial DNA (nucleotides) corresponding to the DNA sequence of an immunogenic foreign gene. The DNA (nucleotides) has an at least 15 consecutive DNA, preferably at least 20 consecutive DNA, and more preferably at least 30 consecutive DNA sequence. A positive clone itself for producing the DNA as mentioned above can also be used as the probe.

To obtain the DNA of the immunogenic foreign gene, a DNA/RNA amplification method by PCR (Science, 230, 1350 (1985)) is preferably used. In particular, when a full length cDNA is hardly obtained from the library, the RACE method (Rapid amplification of cDNA ends; Jikken Igaku 12(6), 35(1994)), particularly the 5'-RACE method (M. A. Frohman, et al., Proc. Natl. Acad. Sci., USA., 8, 8998(1988)) is preferably used.

The primers used for PCR can be designed, based on the DNA sequence information of the immunogenic foreign gene, and synthesized in accordance with standard methods. As the primers, DNA portions (SP6 promoter/primer and T7 terminator/primer) added to both ends of a vector plasmid having the DNA of the immunogenic foreign gene incorporated therein can also be used, as described in the Examples below.

The isolation/purification of the DNA/RNA fragment amplified by PCR can be performed in accordance with standard methods, for example, by gel electrophoresis.

The DNA sequence of the DNA of the immunogenic foreign gene thus obtained or various DNA fragments thereof can be determined in accordance with standard methods, for example, by the dideoxy method (Proc. Natl. Acad. Sci., USA., 74, 5463(1977)) or Maxam-Gilbert method (Methods in Enzymology, 65, 499(1980)), or by simply using a commercially available sequencing kit.

The gene encoding the amino acids of a protein capable of being a component of viral particles may be any gene that can encode a protein that is formed into a fusion protein with an immunogenic foreign gene as mentioned above in target cells and can be expressed as a protein capable of being a component of viral particles in insect cells.

Examples of the gene encoding the amino acids of a protein capable of being a component of viral particles include genes of gp64 protein (GenBank Accession No. L22858), vesicular stomatitis virus glycoprotein G (VSVG: GenBank Accession No. J02428), herpes simplex virus glycoprotein (KOS: GenBank Accession No. K01760), human immunodeficiency virus type I gp120 (GenBank Accession No. U47783), human respiratory syncytial virus membrane glycoprotein (GenBank Accession No. M86651), influenza A virus hemagglutinin protein (GenBank Accession No. U38242), and genes of envelope proteins of viruses closely related to baculovirus.
Amino acid sequence of gp64 viral particle protein (gp64) encoded by nucleotides 108179 to 109717 in the AcNPV genome (GenBank Accession No. L22858):
Amino acid 21-512 of gp64 encoded in the AcNPV genome (GenBank Accession No. L22858):

In the present invention, the gp64 gene described in Examples below is used.

The DNA of the gene encoding the amino acids of a protein capable of being a component of viral particles can be easily produced or obtained by synthesis based on the nucleic acid sequence information of the polynucleotide encoding the amino acid sequence of the polypeptide of the gene encoding the amino acids of the target protein capable of being a component of viral particles; or by directly synthesizing the DNA corresponding to the nucleotide sequence encoding the amino acid sequence, based on the amino acid sequence information of the gene encoding the amino acids of the protein capable of being a component of viral particles (chemical DNA synthesis), as in the case of the production of the DNA of the immunogenic foreign gene.

The DNA sequence corresponding to the nucleic acid sequence encoding the amino acids of a protein capable of being a component of viral particles is not limited to a full-length coding region, and may be DNA containing a partial DNA sequence thereof.

As in the case of the production of the DNA molecule of the immunogenic foreign gene, the DNA of the gene encoding the amino acids of a protein capable of being a component of viral particles can be produced by general gene engineering techniques (see, for example, Molecular Cloning 2d Ed, Cold Spring Harbor Lab. Press (1989); Zoku Seikagaku Jikken Kouza, "Idenshi Kenkyuho I, II, III", edited by the Japanese Biochemistry Society, 1986).

In the present invention, commercially available vector plasmids into which a part of the promoter that controls the expression of the immunogenic foreign gene described later has been incorporated and a (partial) gene encoding the amino acids of a protein capable of being a component of viral particles has been introduced can also be used.

### Vertebrate Promoters

Examples of the vertebrate promoter (promoter capable of functioning in vertebrate cells) that is one of the components of the transfer vector used in the present disclosure include mammalian promoters and avian promoters.

### Mammalian Promoters

Examples of the mammalian promoter (promoter capable of functioning in mammalian cells) that is one of the components of the transfer vector used in the present disclosure include cytomegalovirus promoters, SV40 promoters, retrovirus promoters, metallothionein promoters, heat shock protein promoters, CAG promoters, elongation factor 1α promoters, actin promoters, ubiquitin promoters, albumin promoters, and MHC promoters.

### Avian Promoters

Examples of the avian promoter include β actin promoters, heat shock protein promoters, elongation factor promoters, ubiquitin promoters, and albumin promoters.

### Baculovirus Promoters

Examples of the baculovirus promoter that is one of the components of the baculovirus transfer vector used in the present disclosure include polyhedrin promoter, p10 promoter, ie1 promoter, p35 promoter, vp39 promoter, and gp64 promoter.

### Production of Recombinant Transfer Vector

The present disclosure relates to a novel transfer vector having a structure capable of expressing the desired immunogenic foreign gene as an antigenic protein in both insect cells and vertebrate cells, particularly mammalian cells. The novel transfer vector produced in the present invention has a structure in which a DNA sequence encoding the amino acid sequence of the desired immunogenic protein and a DNA sequence encoding the amino acid sequence of a protein capable of being a component of viral particles are linked downstream of linked promoters comprising one vertebrate promoter, particularly a mammalian promoter, and one baculovirus promoter. The DNA regions containing the DNA sequences of the two promoters, i.e., one vertebrate promoter, particularly a mammalian promoter, and one baculovirus promoter, may be directly linked to each other, or an intervening DNA sequence may be present between the DNA sequences of the two promoters (provided, however, that the two promoters should have promoter activity in insect cells and in vertebrate cells, and particularly mammalian cells). The promoter region has a structure such that either of a vertebrate promoter, particularly a mammalian promoter, and a baculovirus promoter, linked to each other, may be disposed more closely to the gene to be expressed. In the Examples described later, a baculovirus promoter is disposed more closely to the gene to be expressed than a mammalian promoter.

In the above structure, the DNA sequence of the fusion gene of a gene encoding a protein capable of being a component of viral particles and a desired immunogenic foreign gene may be such that these two genes are directly linked to each other, or an intervening DNA sequence is present between the genes (provided, however, that it is necessary not to cause a frameshift of the downstream gene and the upstream gene). Preferably, the antigen-presenting domain of the protein of a foreign gene having the desired immunogenicity is fused to a protein capable of being a component of viral particles. Therefore, the protein of a foreign gene having the desired immunogenicity should not be cut off from the protein capable of being a component of viral particles, but should be used in a form fused therewith.

A fusion gene containing these two genes may be prepared in advance, and then incorporated into the vector. Alternatively, one of the genes may first be incorporated into the vector and then the other may be incorporated into the vector to form a fusion gene in the vector.

To produce such a transfer vector, commercially available expression vectors already having some essential components of the transfer vector used in the present invention, i.e., a promoter region containing a vertebrate promoter, particularly a mammalian promoter, and a baculovirus promoter, and a gene region encoding the amino acid sequence of a protein capable of being a component of viral particles, may be used, and the required components can be inserted by optionally cleaving such a commercially available expression vector with restriction enzymes and incorporating other promoter to insert a fused DNA sequence of a foreign gene having the desired immunogenicity and a gene encoding the amino acid sequence of a protein capable of being a component of viral particles into the cloning region of the vector, or by inserting a foreign gene having the desired immunogenicity into the N terminus side of the DNA region of a gene encoding the amino acid sequence of a protein capable of being a component of viral particles, already incorporated in a plasmid.

In the present invention, the plasmid vector having a structure capable of expressing a foreign protein having the desired immunogenicity as an antigenic protein in both insect cells and vertebrate cells, particularly mammalians cells, may be produced by using a commercially available plasmid already having a partial structure thereof. The amino acid sequence of a peptide for cleaving the fusion protein with enzymes in vertebrate cells may intervene. In the transfer vector used in the present invention, an enhancer for increasing the transcription activity in vertebrate cells, particularly mammalian cells, may be disposed upstream of the two promoters, or a DNA sequence encoding the amino acid sequence of a signal peptide for facilitating extracellular secretion of the expressed protein from host cells may be bound to the gene to be fused and expressed. To terminate the transcription, a vertebrate terminator region, such as a rabbit β globin terminator, which is effective in vertebrate cells, may be disposed downstream of the gene to be fused and expressed.

A transfer vector capable of expressing a fusion gene of an immunogenic foreign gene capable of expressing the desired immunogenicity in baculovirus particles and a gene encoding the amino acid sequence of a protein capable of being a component of viral particles can be thereby produced.

Specific examples of the transfer vector and the method for production thereof according to the present disclosure are as shown in the Examples described later. More specifically, transfer vectors having a structure in which a CAG promoter modified from a cytomegalovirus (CMV) promoter as a vertebrate promoter, particularly a mammalian promoter, and a polyhedrin (polh) promoter, vp39 promoter, or gp64 promoter as a baculovirus promoter, are linked to each other, and a fused DNA sequence of an influenza virus antigen gene or malaria antigen gene as a foreign gene and a gp64 antigen gene as a gene encoding the amino acid sequence of a protein capable of being a component of the viral particle is inserted can be mentioned as examples of pCAP-PfCSP, pCAP-PfCSP/272, pCAP-PfCSP/467, pCAP-PfCSP(A361E), pCAP-PfCSP(A361E)/272, pCAP-PfCSP(A361E)/467, pCAP-PfCSP-76, pCAP-PfCSP-76/467, pCAP-PfCSP+209, pCAP-PfCSP+209/467, pCAP-PfCSP+76/209, pCAP-PfCSP+76/209/467, pCAP-HA1/Anhui, pCAP-HA1/Anhui/272, pCAP-HA1/Anhui/467, pCAP-HA1/Vietnam, pCAP-HA1/Vietnam/51, pCAP-HA1/Vietnam/101, pCAP-HAl/Vietnam/154, pCAP-HA1/Vietnam/201, pCAP-HA1/Vietnam/272, pCAP-HA1/Vietnam/467, pCAP-AH/345, pCAP-AH/345/467, pCAP-AH/410, pCAP-AH/410/467, pCAP-AH/473, pCAP-AH/473/467, pCAP-AH/520, pCAP-AH/520/467, pCAP-VN/346, pCAP-VN/346/467, pCAP-VN/410, pCAP-VN/410/467, pCAP-VN/473, pCAP-VN/473/467, pCAP-VN/520, pCAP-VN/520/467, pCAP-CO/full, pCAP-CO/full/467, pCAP-CO/19, pCAP-CO/19/467, pCAP-CO/76, pCAP-CO/76/467, pCAP-CO/205, pCAP-CO/205/467, pCA39-HA1/Anhui, pCA64-HA1/Anhui, pCA39-PfCSP(A361E), pCA64-PfCSP(A361E), pCAP-CO/full/VSV, pCAP-CO/19/VSV, pCAP-CO/76/VSV, pCAP-CO/205/VSV, pDual-Pfs25-PfCSP-gp64, and pDual-PfMSP1-PfCSP-gp64.

### (2) Production of Recombinant Baculovirus

The present disclosure provides a method of producing a recombinant baculovirus comprising the step of producing a transfer vector having a structure in which a fusion gene containing at least one gene encoding a protein capable of being a component of the viral particle and at least one immunogenic foreign gene is incorporated downstream of a dual promoter consisting of two linked promoters, i.e., one vertebrate promoter and one baculovirus promoter, and the step of co-transfecting the transfer vector and a baculovirus DNA into a host cell and isolating the recombinant baculovirus.

In the above process of producing the recombinant baculovirus, the method of introducing the desired recombinant DNA (transfer vector) into the host cell and the method of transformation thereby are not particularly limited. Various methods that are well known and commonly used in this field can be used. For example, general genetic recombination techniques (e.g., Science, 224, 1431 (1984); Biochem. Biophys. Res. Comm., 130, 692 (1985); Proc. Natl. Acad. Sci. USA, 80, 5990 (1983) can be used to prepare the recombinant baculovirus. The recombinant DNA (transfer vector) can be expressed and produced with reference to Ohno et al., "Tanpaku Jikken Protocol 1 Functional Analysis, Saibo Kogaku Bessatsu, Jikken Protocol Series, 1997, Shujun-sha". The general methods used for the handling of insect cells, gene recombination, and co-transfection may be the same as well-known methods of producing a recombinant virus in insect cells (BACULOVIRUS EXPRESSION VECTORS: A LABORATORY MANUAL, Oxford University Press, 1994).

The resulting recombinant baculovirus can be cultured in accordance with standard methods. A fusion product (expressed product) of the DNA of a foreign gene having the desired immunogenicity with the DNA encoding the amino acid sequence of a protein capable of being a component of viral particles used in the present invention is expressed and produced (accumulated and secreted) inside, outside, or on the cell membrane of insect cells by the culturing of the baculovirus.

As a medium used for the culturing, various commonly used media can be selectively used according to the host cell used. The culture can be performed under conditions suitable for the growth of the host cell.

More specifically, the method of producing the recombinant baculovirus comprises the step of preparing a baculovirus DNA to be subjected to homologous recombination with a transfer vector produced above, and the step of co-transfecting the transfer vector and the baculovirus DNA into insect cells, such as Sf9 cells and Sf21 cells derived from *Spodoptera frugiperda,* and Tn5 cells (High Five cells) derived from *Trichoplusia ni,* as host cells.

The baculovirus DNA thus produced to be subjected to homologous recombination with the transfer vector may be a wild type, mutant, or recombinant baculovirus DNA. The baculovirus DNA can enhance the probability of its homologous recombination with the transfer vector used in the present invention as long as it has a DNA structure homologous to the baculovirus-derived DNA containing DNA of the dual promoter region, and DNA of a fusion gene obtained by fusion of an immunogenic foreign gene and a gene encoding a protein capable of being a component of viral particles.

To induce homologous recombination, the mixing ratio of the transfer vector to the baculovirus DNA by weight is preferably about 1:1 to about 10:1.

After the co-transfection step of simultaneous introduction into insect cells, the cells are cultured, and viral plaques are obtained from the culture supernatant and then suspended in a medium. The virus is eluted from agar by vortex and centrifuged to yield a solution containing the recombinant virus.

In the above process, commercially available products may be used as the baculovirus DNA. For example, BacVector-1000 DNA and BacVector-2000 DNA (supplied from Novagen) in which the polyhedrin gene has been removed from AcNPV can be used.

The co-transfection for the homologous recombination of the obtained transfer vector and baculovirus DNA into insect cells can be performed using a commercially available vector transfection kit (BacVector Transfection Kits, supplied from Novagen) as described above in accordance with the instructions packaged with the vector transfection kit. Thus, the obtained transfer vector can be co-transfected together with the baculovirus DNA into insect cells such as Sf9 cell to yield a recombinant baculovirus.

In the present disclosure, in accordance with the above method of producing a recombinant baculovirus, a transfer vector that is any one of pCAP-PfCSP, pCAP-PfCSP/272, pCAP-PfCSP/467, pCAP-PfCSP(A361E), pCAP-PfCSP(A361E)/272, pCAP-PfCSP(A361E)/467, pCAP-PfCSP-76, pCAP-PfCSP-76/467, pCAP-PfCSP+209, pCAP-PfCSP+209/467, pCAP-PfCSP+76/209, pCAP-PfCSP+76/209/467, pCAP-HA1/Anhui, pCAP-HA1/Anhui/272, pCAP-HA1/Anhui/467, pCAP-HA1/Vietnam, pCAP-HA1/Vietnam/51, pCAP-HA1/Vietnam/101, pCAP-HA1/Vietnam/154, pCAP-HA1/Vietnam/201, pCAP-HA1/Vietnam/272, pCAP-HA1/Vietnam/467, pCAP-AH/345, pCAP-AH/345/467, pCAP-AH/410, pCAP-AH/410/467, pCAP-AH/473, pCAP-AH/473/467, pCAP-AH/520, pCAP-AH/520/467, pCAP-VN/346, pCAP-VN/346/467, pCAP-VN/410, pCAP-VN/410/467, pCAP-VN/473, pCAP-VN/473/467, pCAP-VN/520, pCAP-VN/520/467, pCAP-CO/full, pCAP-CO/full/467, pCAP-CO/19, pCAP-CO/19/467, pCAP-CO/76, pCAP-CO/76/467, pCAP-CO/205, pCAP-CO/205/467, pCA39-HA1/Anhui, pCA64-HA1/Anhui, pCA39-PfCSP(A361E), pCA64-PfCSP(A361E), pCAP-CO/full/VSV, pCAP-CO/19/VSV, pCAP-CO/76-/VSV, pCAP-CO/205/VSV, pDual-Pfs25-PfCSP-gp64, and pDual-PfMSP1-PfCSP-gp64, and a baculovirus DNA can be co-transfected into Sf9 insect cells to yield a recombinant baculovirus of any one of AcNPV-CAP-PfCSP, AcNPV-CAP-PfCSP/272, AcNPV-CAP-PfCSP/467, AcNPV-CAP-PfCSP(A361E), AcNPV-CAP-PfCSP(A361E)/272, AcNPV-CAP-PfCSP(A361E)/467, AcNPV-CAP-PfCSP-76, AcNPV-CAP-PfCSP-76/467, AcNPV-CAP-PfCSP+209, AcNPV-CAP-PfCSP+209/467, AcNPV-CAP-PfCSP+76/209, AcNPV-CAP-PfCSP+76/209/467, AcNPV-CAP-HA1/Anhui, AcNPV-CAP-HA1/Anhui/272, AcNPV-CAP-HA1/Anhui/467, AcNPV-CAP-HA1/Vietnam, AcNPV-CAP-HA1/Vietnam/51, AcNPV-CAP-HA1/Vietnam/101, AcNPV-CAP-HA1/Vietnam/154, AcNPV-CAP-HA1/Vietnam/201, AcNPV-CAP-HA1/Vietnam/272, AcNPV-CAP-HA1/Vietnam/467, AcNPV-CAP-AH/345, AcNPV-CAP-AH/345/467, AcNPV-CAP-AH/410, AcNPV-CAP-AH/410/467, AcNPV-CAP-AH/473, AcNPV-CAP-AH/473/467, AcNPV-CAP-AH/520, AcNPV-CAP-AH/520/467, AcNPV-CAP-VN/346, AcNPV-CAP-VN/346/467, AcNPV-CAP-VN/410, AcNPV-CAP-VN/410/467, AcNPV-CAP-VN/473, AcNPV-CAP-VN/473/467, AcNPV-CAP-VN/520, AcNPV-CAP-VN/520/467, AcNPV-CAP-CO/full, AcNPV-CAP-CO/full/467, AcNPV-CAP-CO/19, AcNPV-CAP-CO/19/467, AcNPV-CAP-CO/76, AcNPV-CAP-CO/76/467, AcNPV-CAP-CO/205, AcNPV-CAP-CO/205/467, AcNPV-CA39-HA1/Anhui, AcNPV-CA64-HA1/Anhui, AcNPV-CA39-PfCSP(A361E), AcNPV-CA64-PfCSP(A361E), AcNPV-CAP-CO/full/VSV, AcNPV-CAP-CO/19/VSV, AcNPV-CAP-CO/76/VSV, AcNPV-CAP-CO/205/VSV, AcNPV-Dual-Pfs25-PfCSP-gp64, and AcNPV-Dual-PfMSP1-PfCSP-gp64.

In addition to the above method of producing a recombinant baculovirus, other methods of producing a recombinant baculovirus include, for example, a method of using a transposon as a phagemid (bacmid) having the entire baculovirus genome incorporated therein to efficiently insert a foreign gene into *Escherichia coli.*

According to this method, a bacmid bearing a viral gene is extracted from bacterial cells and transfected into insect cells to thereby easily produce and collect a recombinant baculovirus.

The purification of the recombinant baculovirus of the present disclosure obtained by the above method of producing a recombinant baculovirus can be performed by using known virus purification methods.

For the purification of the recombinant baculovirus, for example, 0.5 to 1.0 mL of a stock virus obtained by the above method of producing a recombinant baculovirus is inoculated into insect cells (1 x 10⁷ cells/10 cm dish), such as Sf9 cells, the culture supernatant is collected several days after the infection, and a virus pellet obtained by centrifugation is suspended in a buffer, such as PBS. The resulting suspension is subjected to a sucrose gradient of 10 to 60%, and then centrifuged (25,000 rpm, 60 minutes, 4°C) to collect a virus band. The collected virus is further suspended in PBS, subsequently centrifuged (under the same conditions as above), and the resulting purified recombinant virus pellet is stored at 4°C in a buffer, such as PBS.

The infectivity titer of the above resulting purified recombinant virus can be measured by plaque assay (BACULOVIRUS EXPRESSION VECTORS: A LABORATORY MANUAL, Oxford University Press, 1994) using insect cells such as Sf9 cells.

In the recombinant viruses described in the Examples, the N terminus of the baculovirus protein gp64 is exposed outside the particle, whereas the C terminus thereof is inside the particle. Therefore, if the protein encoded by a foreign gene having the desired immunogenicity is fused to the N terminus of gp64, the entity thereof is exposed as a component of the viral particle outside the viral protein particle in insect cells, and thus the antigen is more easily presented, which is suitable for use as the vaccine formulation of the present invention.

### (3) Pharmaceutical Composition of the Present Invention (Pharmaceutical Containing the Recombinant Baculovirus of the Present Invention as an Active Ingredient)

The recombinant baculovirus used in the present invention, which is an active ingredient of the pharmaceutical composition of the present invention, can be obtained by the genetic engineering techniques shown in the above (2).

The pharmaceutical composition of the present invention essentially contains as the active ingredient a recombinant baculovirus obtained by homologous recombination of a baculovirus DNA and a transfer vector constructed so that a fusion gene obtained by the fusion of an immunogenic foreign gene used in the present invention to a gene encoding the amino acid sequence of a protein capable of being a component of the viral particle can be expressed in insect cells and vertebrate cells, particularly cells of mammalians, including humans.

The present disclosure particularly provides a pharmaceutical composition comprising as an active ingredient a specific recombinant baculovirus that is any one of AcNPV-CAP-PfCSP, AcNPV-CAP-PfCSP/272, AcNPV-CAP-PfCSP/467, AcNPV-CAP-PfCSP(A361E), AcNPV-CAP-PfCSP(A361E)/272, AcNPV-CAP-PfCSP(A361E)/467, AcNPV-CAP-PfCSP-76, AcNPV-CAP-PfCSP-76/467, AcNPV-CAP-PfCSP+209, AcNPV-CAP-PfCSP+209/467, AcNPV-CAP-PfCSP+76/209, AcNPV-CAP-PfCSP+76/209/467, AcNPV-CAP-HA1/Anhui, AcNPV-CAP-HA1/Anhui/272, AcNPV-CAP-HA1/Anhui/467, AcNPV-CAP-HA1/Vietnam, AcNPV-CAP-HA1/Vietnam/51, AcNPV-CAP-HA1/Vietnam/101, AcNPV-CAP-HA1/Vietnam/154, AcNPV-CAP-HA1/Vietnam/201, AcNPV-CAP-HA1/Vietnam/272, AcNPV-CAP-HA1/Vietnam/467, AcNPV-CAP-AH/345, AcNPV-CAP-AH/345/467, AcNPV-CAP-AH/410, AcNPV-CAP-AH/410/467, AcNPV-CAP-AH/473, AcNPV-CAP-AH/473/467, AcNPV-CAP-AH/520, AcNPV-CAP-AH/520/467, AcNPV-CAP-VN/346, AcNPV-CAP-VN/346/467, AcNPV-CAP-VN/410, AcNPV-CAP-VN/410/467, AcNPV-CAP-VN/473, AcNPV-CAP-VN/473/467, AcNPV-CAP-VN/520, AcNPV-CAP-VN/520/467, AcNPV-CAP-CO/full, AcNPV-CAP-CO/full/467, AcNPV-CAP-CO/19, AcNPV-CAP-CO/19/467, AcNPV-CAP-CO/76, AcNPV-CAP-CO/76/467, AcNPV-CAP-CO/205, AcNPV-CAP-CO/205/467, AcNPV-CA39-HA1/Anhui, AcNPV-CA64-HA1/Anhui, AcNPV-CA39-PfCSP(A361E), AcNPV-CA64-PfCSP(A361E), AcNPV-CAP-CO/full/VSV, AcNPV-CAP-CO/19/VSV, AcNPV-CAP-CO/76/VSV, AcNPV-CAP-CO/205/VSV, AcNPV-Dual-Pfs25-PfCSP-gp64, and AcNPV-Dual-PfMSP1-PfCSP-gp64. disclosure

The recombinant baculovirus of the present disclosure that is any one of AcNPV-CAP-PfCSP, AcNPV-CAP-PfCSP/272, AcNPV-CAP-PfCSP/467, AcNPV-CAP-PfCSP(A361E), AcNPV-CAP-PfCSP(A361E)/272, AcNPV-CAP-PfCSP(A361E)/467, AcNPV-CAP-PfCSP-76, AcNPV-CAP-PfCSP-76/467, AcNPV-CAP-PfCSP+209, AcNPV-CAP-PfCSP+209/467, AcNPV-CAP-PfCSP+76/209, AcNPV-CAP-PfCSP+76/209/467, AcNPV-CAP-HA1/Anhui, AcNPV-CAP-HA1/Anhui/272, AcNPV-CAP-HA1/Anhui/467, AcNPV-CAP-HA1/Vietnam, AcNPV-CAP-HA1/Vietnam/5 AcNPV-CAP-HA1/Vietnam/101, AcNPV-CAP-HA1/Vietnam/154, AcNPV-CAP-HA1/Vietnam/201, AcNPV-CAP-HA1/Vietnam/272, AcNPV-CAP-HA1/Vietnam/467, AcNPV-CAP-AH/345, AcNPV-CAP-AH/345/467, AcNPV-CAP-AH/410, AcNPV-CAP-AH/410/467, AcNPV-CAP-AH/473, AcNPV-CAP-AH/473/467, AcNPV-CAP-AH/520, AcNPV-CAP-AH/520/467, AcNPV-CAP-VN/346, AcNPV-CAP-VN/346/467, AcNPV-CAP-VN/410, AcNPV-CAP-VN/410/467, AcNPV-CAP-VN/473, AcNPV-CAP-VN/473/467, AcNPV-CAP-VN/520, AcNPV-CAP-VN/520/467, AcNPV-CAP-CO/full, AcNPV-CAP-CO/full/467, AcNPV-CAP-CO/19, AcNPV-CAP-CO/19/467, AcNPV-CAP-CO/76, AcNPV-CAP-CO/76/467, AcNPV-CAP-CO/205, AcNPV-CAP-CO/205/467, AcNPV-CA39-HA1/Anhui, AcNPV-CA64-HA1/Anhui, AcNPV-CA39-PfCSP(A361E), AcNPV-CA64-PfCSP(A361E), AcNPV-CAP-CO/full/VSV, AcNPV-CAP-CO/19/VSV, AcNPV-CAP-CO/76/VSV, AcNPV-CAP-CO/205/VSV, AcNPV-Dual-Pfs25-PfCSP-gp64, and AcNPV-Dual-PfMSP1-PfCSP-gp64, used as an active ingredient of the pharmaceutical composition of the present disclosure, has the action of enhancing infection-preventing effects against an infectious antigen and reducing the infectivity titer. Utilizing this action and activity, the recombinant baculovirus can be used for the treatment of diseases associated with infection of target cells and tissues. Examples of target cells affected by such infection include blood cells, hepatic cells, renal cells, brain cells, lung cells, epithelial cells, and muscular cells. Examples of tissues comprising such cells include the lung, liver, kidney, brain, arteries and veins, the stomach, intestines, urethra, skin, and muscle.

The pharmaceutical composition enhances infection-preventing effects against infectious antigens, for example, malaria antigens such as sporozoite surface antigens (CSP and TRAP) of malaria parasites, merozoite surface membrane protein MSP1, malaria S antigen secreted from erythrocytes infected with malaria, PfEMP1 protein present in the knobs of erythrocytes infected with malaria, SERA protein, TRAMP protein, AMA1 protein, and Pfs25 known as a transmission-blocking antigen; and influenza antigens such as HA antigen, NA antigen, M2 antigen, and NP antigen, and reduces the infectivity titer (e.g., viral infectivity titer), thereby increasing the survival period and survival rate of mammals including humans, compared to the group not administered the pharmaceutical composition of the present disclosure. Therefore, the pharmaceutical composition is particularly useful as a preventive or therapeutic agent for malaria and influenza virus infections.

The pharmaceutical composition of the invention has the action of enhancing infection-preventing effects against infectious antigens and reducing the infectivity titers, and is therefore useful as a preventive or therapeutic agent for infectious diseases caused by pathogens such as malaria, and complications thereof.

By using a gene immunogenic to non-human vertebrates as an immunogenic foreign gene of the transfer vector to obtain a recombinant baculovirus used as an active ingredient of the pharmaceutical composition of the present disclosure, for example, a chicken influenza vaccine can be prepared, which has the action of enhancing infection-preventing effects against the infectious antigen and reducing the infectivity titer. By utilizing this action and activity, the pharmaceutical composition can be used for the treatment of diseases associated with the infection of target cells and tissues.

The pharmaceutical composition of the present invention can be prepared as a composition containing a pharmaceutically effective amount of the recombinant baculovirus used in the invention and a pharmaceutically acceptable carrier.

The infection-preventing effect of the recombinant baculovirus used in the present invention in vertebrates, particularly mammals, including humans, or mammalian cells can be provided, for example, by administering the pharmaceutical composition containing the recombinant baculovirus used in the present invention and additives for pharmaceutical administration to vertebrates, particularly mammals, including humans, by the intramuscular, subcutaneous, intracutaneous, intraperitoneal, nasal, or respiratory route, and then immunizing the vertebrates with the pharmaceutical composition containing the recombinant baculovirus used in the present invention as an active ingredient several times. Respiratory administration of the pharmaceutical composition of the invention is particularly preferable.

The infection-preventing effect can be evaluated by comparing the survival rate of vertebrates, particularly mammals, including humans, that have been immunized with the pharmaceutical composition of the invention several times and then infected with a target pathogen with the survival rate of those not administered with the pharmaceutical composition.

### (4) Vaccine of the present invention

The recombinant baculovirus that is any one of AcNPV-CAP-PfCSP, AcNPV-CAP-PfCSP/272, AcNPV-CAP-PfCSP/467, AcNPV-CAP-PfCSP(A361E), AcNPV-CAP-PfCSP(A361E)/272, AcNPV-CAP-PfCSP(A361E)/467, AcNPV-CAP-PfCSP-76, AcNPV-CAP-PfCSP-76/467, AcNPV-CAP-PfCSP+209, AcNPV-CAP-PfCSP+209/467, AcNPV-CAP-PfCSP+76/209, AcNPV-CAP-PfCSP+76/209/467, AcNPV-CAP-HA1/Anhui, AcNPV-CAP-HA1/Anhui/272, AcNPV-CAP-HA1/Anhui/467, AcNPV-CAP-HA1/Vietnam, AcNPV-CAP-HA1/Vietnam/51, AcNPV-CAP-HA1/Vietnam/101, AcNPV-CAP-HA1/Vietnam/159, AcNPV-CAP-HA1/Vietnam/201, AcNPV-CAP-HA1/Vietnam/272, AcNPV-CAP-HA1/Vietnam/467, AcNPV-CAP-AH/345, AcNPV-CAP-AH/345/467, AcNPV-CAP-AH/410, AcNPV-CAP-AH/410/467, AcNPV-CAP-AH/473, AcNPV-CAP-AH/473/467, AcNPV-CAP-AH/520, AcNPV-CAP-AH/520/467, AcNPV-CAP-VN/346, AcNPV-CAP-VN/346/467, AcNPV-CAP-VN/410, AcNPV-CAP-VN/410/467, AcNPV-CAP-VN/473, AcNPV-CAP-VN/473/467, AcNPV-CAP-VN/520, AcNPV-CAP-VN/520/467, AcNPV-CAP-CO/full, AcNPV-CAP-CO/full/467, AcNPV-CAP-CO/19, AcNPV-CAP-CO/19/467, AcNPV-CAP-CO/76, AcNPV-CAP-CO/76/467, AcNPV-CAP-CO/205, AcNPV-CAP-CO/205/467, AcNPV-CA39-HA1/Anhui, AcNPV-CA64-HA1/Anhui, AcNPV-CA39-PfCSP(A361E), AcNPV-CA64-PfCSP(A361E), AcNPV-CAP-CO/full/VSV, AcNPV-CAP-CO/19/VSV, AcNPV-CAP-CO/76/VSV, AcNPV-CAP-CO/205/VSV, AcNPV-Dual-Pfs25-PfCSP-gp64, and AcNPV-Dual-PfMSP1-PfCSP-gp64 used as the active ingredient of the pharmaceutical composition of the present disclosure can enhance pathogen infection-preventing effects as described in the Examples below, and an expressed fusion product of the DNA sequence obtained by fusion of a gene encoding the amino acid sequence of a protein capable of being a component of viral particles and a foreign gene with the desired immunogenicity of the present disclosure capable of reducing the infectivity titer is produced as viral particles budded from insect cells. When the pharmaceutical composition of the invention is administered in the form of viral particles to vertebrates, particularly mammals, including humans, a foreign antigen protein contained as a component of viral particles promotes acquired immunity (humoral immunity and cellular immunity), and further, the antigenic protein as an expressed product of the fusion DNA sequence seems to promote acquired immunity (humoral immunity and cellular immunity) in vertebrate cells, particularly cells of mammals, including humans. Thus, the recombinant baculovirus used in the present invention is useful as a vaccine.

The present disclosure particularly provides a vaccine containing as an active ingredient a specific recombinant baculovirus that is any one of AcNPV-CAP-PfCSP, AcNPV-CAP-PfCSP/272, AcNPV-CAP-PfCSP/467, AcNPV-CAP-PfCSP(A361E), AcNPV-CAP-PfCSP(A361E)/272, AcNPV-CAP-PfCSP(A361E)/467, AcNPV-CAP-PfCSP-76, AcNPV-CAP-PfCSP-76/467, AcNPV-CAP-PfCSP+209, AcNPV-CAP-PfCSP+209/467, AcNPV-CAP-PfCSP+76/209, AcNPV-CAP-PfCSP+76/209/467, AcNPV-CAP-HA1/Anhui, AcNPV-CAP-HA1/Anhui/272, AcNPV-CAP-HA1/Anhui/467, AcNPV-CAP-HA1/Vietnam, AcNPV-CAP-HA2/Vietnam/51, AcNPV-CAP-HA1/Vietnam/101, AcNPV-CAP-HA1/Vietnam/154, AcNPV-CAP-HAl/Vietnam/201, AcNPV-CAP-HA1/Vietnam/272, AcNPV-CAP-HA1/Vietnam/467, AcNPV-CAP-AH/345, AcNPV-CAP-AH/345/467, AcNPV-CAP-AH/410, AcNPV-CAP-AH/410/467, AcNPV-CAP-AH/473, AcNPV-CAP-AH/473/467, AcNPV-CAP-AH/520, AcNPV-CAP-AH/520/467, AcNPV-CAP-VN/346, AcNPV-CAP-VN/346/467, AcNPV-CAP-VN/410, AcNPV-CAP-VN/410/467, AcNPV-CAP-VN/473, AcNPV-CAP-VN/473/467, AcNPV-CAP-VN/520, AcNPV-CAP-VN/520/467, AcNPV-CAP-CO/full, AcNPV-CAP-CO/full/467, AcNPV-CAP-CO/19, AcNPV-CAP-CO/19/467, AcNPV-CAP-CO/76, AcNPV-CAP-CO/76/467, AcNPV-CAP-CO/205, AcNPV-CAP-CO/205/467, AcNPV-CA39-HA1/Anhui, AcNPV-CA64-HA1/Anhui, AcNPV-CA39-PfCSP(A361E), AcNPV-CA64-PfCSP(A361E), AcNPV-CAP-CO/full/VSV, AcNPV-CAP-CO/19/VSV, AcNPV-CAP-CO/76/VSV, AcNPV-CAP-CO/205/VSV, AcNPV-Dual-Pfs25-PfCSP-gp64, and AcNPV-Dual-PfMSP1-PfCSP-gp64.

As in the pharmaceutical composition of the above (3), the vaccine enhances infection-preventing effects against infectious antigens such as malaria antigens such as sporozoite surface antigens (CSP and TRAP) of the malaria parasite, merozoite surface membrane protein MSP1, malaria S antigen secreted from erythrocytes infected with malaria, PfEMP1 protein present in the knobs of erythrocytes infected with malaria, SERA protein, TRAMP protein, and AMA1 protein; and influenza antigens such as influenza virus HA antigen, influenza virus NA antigen, influenza virus M2 antigen, and influenza virus NP antigen; the vaccine also reduces the infectivity titer (e.g., the viral infectivity titer), thereby increasing the survival period and survival rate of mammals, including humans, compared with the group not administered with the pharmaceutical composition of the present disclosure. Thus, the vaccine is particularly useful as a preventive or therapeutic agent for malaria and influenza virus infection.

The vaccine of the disclosure enhances infection-preventing effects against infectious antigens and reduces the infectivity titer, and is therefore useful as a preventive or therapeutic agent for infectious diseases caused by pathogens such as influenza viruses and malaria, and complications thereof.

By using a gene immunogenic to non-human vertebrates as an immunogenic foreign gene of the transfer vector to obtain a recombinant baculovirus used as an active ingredient of the pharmaceutical composition of the present disclosure, for example, a chicken influenza vaccine, that can enhance infection-prevention effects against the infectious antigen and reduce the infectivity titer, can be prepared. By utilizing this activity, the vaccine can be used for the treatment of diseases associated with the infection of target cells and tissues.

The recombinant baculovirus that is any one of AcNPV-CAP-PfCSP, AcNPV-CAP-PfCSP/272, AcNPV-CAP-PfCSP/467, AcNPV-CAP-PfCSP(A361E), AcNPV-CAP-PfCSP(A361E)/272, AcNPV-CAP-PfCSP(A361E)/467, AcNPV-CAP-PfCSP-76, AcNPV-CAP-PfCSP-76/467, AcNPV-CAP-PfCSP+209, AcNPV-CAP-PfCSP+209/467, AcNPV-CAP-PfCSP+76/209, AcNPV-CAP-PfCSP+76/209/467, AcNPV-CAP-HA1/Anhui, AcNPV-CAP-HA1/Anhui/272, AcNPV-CAP-HA1/Anhui/467, AcNPV-CAP-HA1/Vietnam, AcNPV-CAP-HA1/Vietnam/51, AcNPV-CAP-HA1/Vietnam/101, AcNPV-CAP-HA1/Vietnam/15 AcNPV-CAP-HA1/Vietnam/201, AcNPV-CAP-HA1/Vietnam/272, AcNPV-CAP-HA1/Vietnam/467, AcNPV-CAP-AH/345, AcNPV-CAP-AH/345/467, AcNPV-CAP-AH/410, AcNPV-CAP-AH/410/467, AcNPV-CAP-AH/473, AcNPV-CAP-AH/473/467, AcNPV-CAP-AH/520, AcNPV-CAP-AH/520/467, AcNPV-CAP-VN/346, AcNPV-CAP-VN/346/467, AcNPV-CAP-VN/410, AcNPV-CAP-VN/410/467, AcNPV-CAP-VN/473, AcNPV-CAP-VN/473/467, AcNPV-CAP-VN/520, AcNPV-CAP-VN/520/467, AcNPV-CAP-CO/full, AcNPV-CAP-CO/full/467, AcNPV-CAP-CO/19, AcNPV-CAP-CO/19/467, AcNPV-CAP-CO/76, AcNPV-CAP-CO/76/467, AcNPV-CAP-CO/205, AcNPV-CAP-CO/205/467, AcNPV-CA39-HA1/Anhui, AcNPV-CA64-HA1/Anhui, AcNPV-CA39-PfCSP(A361E), AcNPV-CA64-PfCSP(A361E), AcNPV-CAP-CO/full/VSV, AcNPV-CAP-CO/19/VSV, AcNPV-CAP-CO/76/VSV, AcNPV-CAP-CO/205/VSV, AcNPV-Dual-Pfs25-PfCSP-gp64, and AcNPV-Dual-PfMSP1-PfCSP-gp64, used as an active ingredient of the vaccine of the present disclosure, can enhance infection-preventing effects against an infectious antigen and reduce the infectivity titer.

By utilizing this activity, the recombinant baculovirus can be used for the treatment of diseases associated with the infection of target cells and tissues.

Examples of target cells affected by such infection include blood cells, hepatic cells, renal cells, brain cells, lung cells, epithelial cells, and muscular cells. Examples of tissues containing such cells include lung, liver, kidney, brain, arterial and venous veins, stomach, intestine, urethra, skin, and muscle tissues.

The vaccine of the present disclosure can be prepared as a pharmaceutical composition of the above (3) containing a pharmaceutically effective amount of the recombinant baculovirus of the disclosure (any one of AcNPV-CAP-PfCSP, AcNPV-CAP-PfCSP/272, AcNPV-CAP-PfCSP/467, AcNPV-CAP-PfCSP(A361E), AcNPV-CAP-PfCSP(A361E)/272, AcNPV-CAP-PfCSP(A361E)/467, AcNPV-CAP-PfCSP-76, AcNPV-CAP-PfCSP-76/467, AcNPV-CAP-PfCSP+209, AcNPV-CAP-PfCSP+209/467, AcNPV-CAP-PfCSP+76/209, AcNPV-CAP-PfCSP+76/209/467, AcNPV-CAP-HA1/Anhui, AcNPV-CAP-HA1/Anhui/272, AcNPV-CAP-HA1/Anhui/467, AcNPV-CAP-HA1/Vietnam, AcNPV-CAP-HA1/Vietnam/51, AcNPV-CAP-HA1/Vietnam/101, AcNPV-CAP-HA1/Vietnam/154, AcNPV-CAP-HAl/Vietnam/201, AcNPV-CAP-HA1/Vietnam/272, AcNPV-CAP-HA1/Vietnam/467, AcNPV-CAP-AH/345, AcNPV-CAP-AH/345/467, AcNPV-CAP-AH/410, AcNPV-CAP-AH/410/467, AcNPV-CAP-AH/473, AcNPV-CAP-AH/473/467, AcNPV-CAP-AH/520, AcNPV-CAP-AH/520/467, AcNPV-CAP-VN/346, AcNPV-CAP-VN/346/467, AcNPV-CAP-VN/410, AcNPV-CAP-VN/410/467, AcNPV-CAP-VN/473, AcNPV-CAP-VN/473/467, AcNPV-CAP-VN/520, AcNPV-CAP-VN/520/467, AcNPV-CAP-CO/full, AcNPV-CAP-CO/full/467, AcNPV-CAP-CO/19, AcNPV-CAP-CO/19/467, AcNPV-CAP-CO/76, AcNPV-CAP-CO/76/467, AcNPV-CAP-CO/205, AcNPV-CAP-CO/205/467, AcNPV-CA39-HA1/Anhui, AcNPV-CA64-HA1/Anhui, AcNPV-CA39-PfCSP(A361E), AcNPV-CA64-PfCSP(A361E), AcNPV-CAP-CO/full/VSV, AcNPV-CAP-CO/19/VSV, AcNPV-CAP-CO/76/VSV, AcNPV-CAP-CO/205/VSV, AcNPV-Dual-Pfs25-PfCSP-gp64, and AcNPV-Dual-PfMSP1-PfCSP-gp64), and a pharmaceutically acceptable carrier.

The vaccine can be prepared in the form of a pharmaceutical composition by using a pharmaceutically acceptable carrier as used in the pharmaceutical composition of the above (3), according to a standard method. Examples of carriers include physiologically acceptable solutions, such as sterile saline and sterile buffered saline.

The vaccine (the formulation is hereinafter the same as in the pharmaceutical composition) can be prepared as a liposomal formulation containing as an active ingredient the recombinant baculovirus of the disclosure (any one of AcNPV-CAP-PfCSP, AcNPV-CAP-PfCSP/272, AcNPV-CAP-PfCSP/467, AcNPV-CAP-PfCSP(A361E), AcNPV-CAP-PfCSP(A361E)/272, AcNPV-CAP-PfCSP(A361E)/467, AcNPV-CAP-PfCSP-76, AcNPV-CAP-PfCSP-76/467, AcNPV-CAP-PfCSP+209, AcNPV-CAP-PfCSP+209/467, AcNPV-CAP-PfCSP+76/209, AcNPV-CAP-PfCSP+76/209/467, AcNPV-CAP-HA1/Anhui, AcNPV-CAP-HA1/Anhui/272, AcNPV-CAP-HA1/Anhui/467, AcNPV-CAP-HA1/Vietnam, AcNPV-CAP-HA1/Vietnam/51, AcNPV-CAP-HA1/Vietnam/101, AcNPV-CAP-HA1/Vietnam/154, AcNPV-CAP-HA1/Vietnam/201, AcNPV-CAP-HA1/Vietnam/272, AcNPV-CAP-HA1/Vietnam/467, AcNPV-CAP-AH/345, AcNPV-CAP-AH/345/467, AcNPV-CAP-AH/410, AcNPV-CAP-AH/410/467, AcNPV-CAP-AH/473, AcNPV-CAP-AH/473/467, AcNPV-CAP-AH/520, AcNPV-CAP-AH/520/467, AcNPV-CAP-VN/346, AcNPV-CAP-VN/346/467, AcNPV-CAP-VN/410, AcNPV-CAP-VN/410/467, AcNPV-CAP-VN/473, AcNPV-CAP-VN/473/467, AcNPV-CAP-VN/520, AcNPV-CAP-VN/520/467, AcNPV-CAP-CO/full, AcNPV-CAP-CO/full/467, AcNPV-CAP-CO/19, AcNPV-CAP-CO/19/467, AcNPV-CAP-CO/76, AcNPV-CAP-CO/76/467, AcNPV-CAP-CO/205, AcNPV-CAP-CO/205/467, AcNPV-CA39-HA1/Anhui, AcNPV-CA64-HA1/Anhui, AcNPV-CA39-PfCSP(A361E), AcNPV-CA64-PfCSP(A361E), AcNPV-CAP-CO/full/VSV, AcNPV-CAP-CO/19/VSV, AcNPV-CAP-CO/76/VSV, AcNPV-CAP-CO/205/VSV, AcNPV-Dual-Pfs25-PfCSP-gp64, and AcNPV-Dual-PfMSP1-PfCSP-gp64), and can be used in combination with an adjuvant.

Specific examples of the vaccine (the pharmaceutical composition) of the present invention include liposomal formulations. The liposomal formulation may be one in which the recombinant baculovirus used in the present invention is retained in a liposome containing an acidic phospholipid as a membrane component, or containing a neutral phospholipid and an acidic phospholipid as membrane components.

The acidic phospholipid and neutral phospholipid used as membrane components are not particularly limited, and various lipids commonly used for liposomal formulations can be used alone or in a combination of two or more.

The liposome membrane is produced in accordance with a standard method by using an acidic phospholipid alone or together with a neutral phospholipid. When an acidic phospholipid is used together with a neutral phospholipid, the proportion of the acidic phospholipids in the liposome membrane components is preferably about 0.1 to about 100 mol%, more preferably 1 to 90 mol%, and even more preferably about 10 to about 50 mol%.

To prepare the liposome, for example, cholesterol or the like can be added. This can control the fluidity of phospholipids and facilitates the liposome preparation. In general, the cholesterol is preferably added in an equivalent amount or less, and preferably in a 0.5-fold amount to an equivalent amount by weight, to the phospholipid.

The mixing ratio of the acidic phospholipid to the active ingredient in the liposomal formulation is about 0.5 to about 100 equivalents, preferably about 1 to about 60 equivalents, and more preferably about 1.5 to about 20 equivalents.

The recombinant baculovirus used in the present invention as an active ingredient is used in an amount of several mol% to several tens mol%, preferably about 5 to about 10 mol%, and usually around 5 mol%, based on the total amount of the lipids.

The production, concentration, and particle diameter control of the liposomal formulation can be performed in accordance with standard methods. If desired, the various additives as described above can also be incorporated into the liposomal formulation.

To produce the liposomal formulation, the recombinant baculovirus used in the invention as an active ingredient can be used in the form of being bound to a fatty acid (e.g., behenic acid, stearic acid, palmitic acid, myristic acid, and oleic acid), an alkyl group, a cholesteryl group, or the like. The liposomal formulation containing such bound components can also be produced in accordance with a standard method (see, for example, Long Circulating Liposomes: Old Drugs, New Therapeutics., M. C. Woodle, G. Storm, Eds: Springer-Verlag Berlin (1998)).

The vaccine (pharmaceutical composition) of the present invention can preferably be used as a vaccine composition. The vaccine is preferably used in combination with a pharmaceutically effective amount of an adjuvant to enhance the anti-infection (anti-malaria) effects.

Any adjuvant commonly used for this type of vaccine can be used as the adjuvant. Examples of such adjuvants include Freund's complete adjuvant, muramyl dipeptide, aluminium hydroxide, BCG, IL-12, N-acetylmuramine-L-alanyl-D-isoglutamine (MDP), thymosin α1, and QS-21. The amount of adjuvant used can be suitably selected according to the degree of symptoms, such as softening of the skin, pain, erythema, fever, headache, and muscular pain, which might be expressed as part of the immune response in humans or animals after the administration of this type of vaccine.

The vaccine (pharmaceutical composition) of the present invention may be used in combination with other publicly known pharmaceutical products, such as immune response-promoting peptides and antibacterial agents (synthetic antibacterial agents).

The vaccine (pharmaceutical composition) may further contain other drugs and additives. Examples thereof include drugs that aid intracellular uptake of the recombinant baculovirus used in the present invention, such as calcium ions. The liposome, and other drugs and additives that facilitate the transfection, such as fluorocarbon emulsifiers, cochleates, tubules, golden particles, biodegradable microspheres, and cationic polymers, can also be used.

The amount of the active ingredient contained in the vaccine (pharmaceutical composition) (drug) of the present invention is not particularly limited and can be selected from a wide range as long as it is a pharmaceutically effective amount. The dosage of the vaccine (pharmaceutical composition) is not particularly limited, and can be appropriately selected from a wide range according to the desired therapeutic effect, the administration method (administration route), the therapeutic period, the patient's age, gender, and other conditions, etc.

When the recombinant baculovirus as an active ingredient of the vaccine (pharmaceutical composition) of the present invention is administered to a human, the recombinant baculovirus is administered in an amount corresponding to 10² to 10¹⁴ PFU, preferably 10⁵ to 10¹² PFU, and more preferably 10⁶ to 10¹⁰ PFU per patient, calculated as the PFU of the recombinant virus.

The dosage of the recombinant baculovirus (any one of AcNPV-CAP-PfCSP, AcNPV-CAP-PfCSP/272, AcNPV-CAP-PfCSP/467, AcNPV-CAP-PfCSP(A361E), AcNPV-CAP-PfCSP(A361E)/272, AcNPV-CAP-PfCSP(A361E)/467, AcNPV-CAP-PfCSP-76, AcNPV-CAP-PfCSP-76/467, AcNPV-CAP-PfCSP+209, AcNPV-CAP-PfCSP+209/467, AcNPV-CAP-PfCSP+76/209, AcNPV-CAP-PfCSP+76/209/467, AcNPV-CAP-HA1/Anhui, AcNPV-CAP-HA1/Anhui/272, AcNPV-CAP-HA1/Anhui/467, AcNPV-CAP-HA1/Vietnam, AcNPV-CAP-HA1/Vietnam/51, AcNPV-CAP-HA1/Vietnam/101, AcNPV-CAP-HA1/Vietnam/154, AcNPV-CAP-HA1/Vietnam/201, AcNPV-CAP-HA1/Vietnam/272, AcNPV-CAP-HA1/Vietnam/467, AcNPV-CAP-AH/345, AcNPV-CAP-AH/345/467, AcNPV-CAP-AH/410, AcNPV-CAP-AH/410/467, AcNPV-CAP-AH/473, AcNPV-CAP-AH/473/467, AcNPV-CAP-AH/520, AcNPV-CAP-AH/520/467, AcNPV-CAP-VN/346, AcNPV-CAP-VN/346/467, AcNPV-CAP-VN/410, AcNPV-CAP-VN/410/467, AcNPV-CAP-VN/473, AcNPV-CAP-VN/473/467, AcNPV-CAP-VN/520, AcNPV-CAP-VN/520/467, AcNPV-CAP-CO/full, AcNPV-CAP-CO/full/467, AcNPV-CAP-CO/19, AcNPV-CAP-CO/19/467, AcNPV-CAP-CO/76, AcNPV-CAP-CO/76/467, AcNPV-CAP-CO/205, AcNPV-CAP-CO/205/467, AcNPV-CA39-HA1/Anhui, AcNPV-CA64-HA1/Anhui, AcNPV-CA39-PfCSP(A361E), AcNPV-CA64-PfCSP(A361E), AcNPV-CAP-CO/full/VSV, AcNPV-CAP-CO/19/VSV, AcNPV-CAP-CO/76/VSV, AcNPV-CAP-CO/205/VSV, AcNPV-Dual-Pfs25-PfCSP-gp64, and AcNPV-Dual-PfMSP1-PfCSP-gp64), used as an active ingredient of the vaccine (pharmaceutical composition) of the present disclosure, can be selected from a very wide range, in terms of the amount of expressible DNA introduced into the vaccine host or the amount of transcribed RNA. These amounts also depend on the strength of the transcription and translation promoters used in the transfer vector of the disclosure.

The vaccine (pharmaceutical composition) of the present invention is administered by directly injecting a recombinant baculovirus suspension prepared by suspending the recombinant baculovirus in PBS (phosphate buffered saline) or saline into a local site (e.g., into the lung tissue, liver, muscle or brain), or by nasal or respiratory inhalation, or by intravascular (e.g., intra-arterial, intravenous, and portal venous), subcutaneous, intracutaneous, or intraperitoneal administration. The vaccine of the invention is preferably administered by respiratory inhalation.

The vaccine (pharmaceutical composition) of the present invention is preferably administered more than once. More specifically, after the initial administration, additional vaccinations are preferably performed once to several times while the condition is observed. This can enhance the desired effect. After the administration of the vaccine (pharmaceutical composition), booster immunization with a pharmaceutical composition containing the recombinant baculovirus of the present disclosure (any one of AcNPV-CAP-PfCSP, AcNPV-CAP-PfCSP/272, AcNPV-CAP-PfCSP/467, AcNPV-CAP-PfCSP(A361E), AcNPV-CAP-PfCSP(A361E)/272, AcNPV-CAP-PfCSP(A361E)/467, AcNPV-CAP-PfCSP-76, AcNPV-CAP-PfCSP-76/467, AcNPV-CAP-PfCSP+209, AcNPV-CAP-PfCSP+209/467, AcNPV-CAP-PfCSP+76/209, AcNPV-CAP-PfCSP+76/209/467, AcNPV-CAP-HA1/Anhui, AcNPV-CAP-HA1/Anhui/272, AcNPV-CAP-HA1/Anhui/467, AcNPV-CAP-HA1/Vietnam, AcNPV-CAP-HA1/Vietnam/51, AcNPV-CAP-HA1/Vietnam/101, AcNPV-CAP-HA1/Vietnam/154, AcNPV-CAP-HAl/Vietnam/201, AcNPV-CAP-HA1/Vietnam/272, AcNPV-CAP-HA1/Vietnam/467, AcNPV-CAP-AH/345, AcNPV-CAP-AH/345/467, AcNPV-CAP-AH/410, AcNPV-CAP-AH/410/467, AcNPV-CAP-AH/473, AcNPV-CAP-AH/473/467, AcNPV-CAP-AH/520, AcNPV-CAP-AH/520/467, AcNPV-CAP-VN/346, AcNPV-CAP-VN/346/467, AcNPV-CAP-VN/410, AcNPV-CAP-VN/410/467, AcNPV-CAP-VN/473, AcNPV-CAP-VN/473/467, AcNPV-CAP-VN/520, AcNPV-CAP-VN/520/467, AcNPV-CAP-CO/full, AcNPV-CAP-CO/full/467, AcNPV-CAP-CO/19, AcNPV-CAP-CO/19/467, AcNPV-CAP-CO/76, AcNPV-CAP-CO/76/467, AcNPV-CAP-CO/205, AcNPV-CAP-CO/205/467, AcNPV-CA39-HA1/Anhui, AcNPV-CA64-HA1/Anhui, AcNPV-CA39-PfCSP(A361E), AcNPV-CA64-PfCSP(A361E), AcNPV-CAP-CO/full/VSV, AcNPV-CAP-CO/19/VSV, AcNPV-CAP-CO/76/VSV, AcNPV-CAP-CO/205/VSV, AcNPV-Dual-Pfs25-PfCSP-gp64, and AcNPV-Dual-PfMSP1-PfCSP-gp64) can be performed. Further, use of various other drugs with the vaccine of the invention, as mentioned above, may enhance the therapeutic effect achieved by the administration of the vaccine (pharmaceutical composition).

In one embodiment of the vaccine (pharmaceutical composition) of the present invention, the recombinant baculovirus, used as one of the active ingredients of the vaccine (pharmaceutical composition) of the invention, is generally prepared by homologous recombination of a baculovirus DNA and a transfer vector into which a fusion gene obtained by fusion of a foreign gene having the desired immunogenicity and a gene encoding a protein capable of being a component of viral particles has been introduced. The recombinant baculovirus is mixed, in an injectable dosage form (a solution, suspension or emulsion), with a pharmaceutically acceptable carrier (i.e., non-toxic to humans and other vertebrates in the dosage and concentration used, and compatible with other ingredients in the formulation) to produce a formulation. Preferably, the formulation thus obtained does not contain oxidizing agents or any other compound that is publicly known to be harmful to recombinant baculovirus.

The carrier may contain a trace amount of additives, such as substances that enhance the isotonicity and chemical stability. Such additives are non-toxic to mammals, including humans, in the dosage and concentration used, and examples thereof include buffers such as phosphoric acid, citric acid, succinic acid, acetic acid, and other organic acids, and salts thereof; antioxidants such as ascorbic acid; low molecular weight (e.g., less than about 10 residues) polypeptides (e.g., polyarginine and tripeptide) proteins (e.g., serum albumin, gelatin, and immunoglobulin); amino acids (e.g., glycine, glutamic acid, aspartic acid, and arginine); monosaccharides, disaccharides, and other carbohydrates (e.g., cellulose and derivatives thereof, glucose, mannose, and dextrin), chelating agents (e.g., EDTA); sugar alcohols (e.g., mannitol and sorbitol); counterions (e.g., sodium); nonionic surfactants (e.g., polysorbate and poloxamer); and PEG.

The pharmaceutical vaccine (composition) containing the recombinant baculovirus can typically be stored as an aqueous solution or a lyophilized product in a unit or multiple dose container such as a sealed ampoule or a vial.

The present disclosure further provides a method of preventing malaria or influenza infection, or a method of treating malaria or influenza, comprising administering an effective amount of the recombinant baculovirus, vaccine, formulation, and pharmaceutical composition of the disclosure to a subject. The present disclosure further provides a method of immunostimulation comprising administering an effective amount of the recombinant baculovirus, vaccine, formulation, and pharmaceutical composition of the disclosure to a subject. Examples of the subjects used include those that may be infected with malaria parasites or influenza viruses, such as humans and other animals (such as mammals, birds, reptiles, fish, and amphibians), and those infected with malaria parasites or influenza viruses. The influenza virus with which the subject is infected is preferably an influenza A virus, and more preferably an influenza A subtype H1 virus, or an influenza A subtype H3 virus. Examples of malaria parasites include *Plasmodium falciparum, Plasmodium vivax, Plasmodium malariae,* and *Plasmodium ovale.*

The recombinant baculovirus of the present disclosure is formed alone or together with a pharmaceutically acceptable carrier into a vaccine, formulation, or pharmaceutical composition, and administered to the subject.

The administration route may be, for example, any administration route mentioned above. The pharmaceutically acceptable carrier for use in the present invention can be suitably selected from carriers commonly used in this technical field, according to the form of the pharmaceutical composition to be produced.

For example, when the pharmacological composition is formed into an aqueous solution, purified water (sterile water) or a physiological buffer solution can be used as the carrier. When the pharmaceutical composition is formed into other appropriate solutions, organic esters capable of being injected, such as glycol, glycerol and olive oil, can be used as the carrier. The composition may contain stabilizers, excipients and the like commonly used in this technical field, and particularly in the field of vaccine formulations.

The amount of recombinant baculovirus in the vaccine, formulation, or pharmaceutical composition of the present invention is not particularly limited, and can be suitably selected from a wide range. In general, the amount of recombinant baculovirus in the composition is preferably about 0.0002 to about 0.2 (w/v %), and more preferably 0.001 to 0.1 (w/v %). The administration method of the recombinant baculovirus, vaccine, formulation, or pharmaceutical composition of the invention is not particularly limited, and can be suitably selected according to the dosage form, the patient's age, gender and other conditions, the severity of the disease, etc. A preferable dosage form thereof is a form for parenteral administration, such as injections, drops, nasal drops, and inhalants. When the composition is formed into an injection or drops, the injection can be intravenously administered as mixed with a replacement fluid such as a glucose solution or an amino acid solution as required, or can be administered intramuscularly, intracutaneously, subcutaneously or intraperitoneally.

The daily dosage of the recombinant baculovirus, vaccine, formulation, or pharmaceutical composition of the present invention may vary depending on the subject's condition, body weight, age, gender, etc., and therefore cannot be completely specified. However, the dosage is usually such that the recombinant baculovirus is administered in an amount of 0.001 to 100 mg per kg of body weight per day. The vaccine, formulation, or composition of the invention can be administered in one or more administrations per day.

When the recombinant baculovirus as an active ingredient of the vaccine (formulation or pharmaceutical composition) of the present invention is administered, the recombinant baculovirus is administered in an amount corresponding to 10² to 10¹⁴ PFU, preferably 10⁵ to 10¹² PFU, and more preferably 10⁶ to 10¹⁰ PFU per patient, calculated as the PFU of the recombinant virus.

The vaccine (composition) of the present invention is administered according to Good Medical Practice, considering the clinical condition (for example, the condition to be prevented or treated) of each patient, the delivery site of the vaccine (composition) containing the recombinant baculovirus, the target tissue, the administration method, the dosage regimen, and other factors publicly known to those skilled in the art. Therefore, the proper dosage of the vaccine (composition) herein is determined in consideration of the above.

### EXAMPLES

The present invention will be described below in more detail with reference to Examples. These Examples are merely examples of the present invention, and do not limit the present invention.

### Example 1: Transfer Vector Plasmid and Method for Production thereof of the Present Invention

### (1) Construction of Transfer Vector Plasmids pCAP-PfCSP, pCAP-PfCSP/272, and pCAP-PfCSP/467

### (1.1) Construction of Plasmid pBACsurf-Hsp65

An Hsp65 gene was obtained by extracting genomic DNA from *M. tuberculosis* H37Rv strain using a QIAamp DNA Midi Kit (Qiagen), and cloning by PCR. More specifically, the genomic DNA extracted from the *M. tuberculosis* H37Rv strain was amplified by PCR using primers phsp65-F1 (5'-AATAATAGATCTAATGGCCAAGACAATTGCGTACGACGAAGA -3' (SEQ ID NO: 1); the BglII site is underlined) and phsp65-R1 (5'-AATCCAATGCGGCCGCGGGAATTCGATTCCTGCAGGTCAGAAATCCATGCCACCCATGTCGCC -3' (SEQ ID NO: 2); the NotI site is underlined). The PCR product was purified, cleaved with restriction enzymes BglII and NotI, ligated to pcDNA3.1(+) (from Invitrogen) digested with BamHI and NotI. The resulting plasmid was designated pcDNA-hsp65. PCR was performed with pcDNA-hsp65 as a template using primers phsp65-F2 (5' - CACCCCTGCAGG*ACTACAAGGACGACGATGACAAG*GAATTCATGGCCAAGAC AATTGCGTACGACGAAGAGGCC - 3' (SEQ ID NO: 3); the Sse8387I and EcoRI sites are underlined, and the FLAG sequence is italicized), and phsp65-R2 (5' - CCCGGGCGAAATCCATGCCACCCATGTCGCCGCCACC - 3' (SEQ ID NO: 4); the Cfr9I site is underlined). The resulting Hsp65 gene DNA fragment was cloned into pENTR/D-TOPO (from Invitrogen), then cleaved with Sse8387I/Cfr9I and inserted into the PstI/Cfr9I site of pBACsurf-CSP (Yoshida et al., Virology 316: 161-70, 2003). The plasmid thus constructed was designed pBACsurf-Hsp65.

### (1.2) Construction of Plasmid pENTR-gp64

PCR was performed with pBACsurf-1 (from Novagen) as a template using primers pPolh-F2 (5' - CACCCGGACCGGATAATTAAAATGATAACCATCTCGCAAATAAATAAG - 3' (SEQ ID NO: 5); the RsrII site is underlined), and pgp64-R2 (5' - GGTACCATATTGTCTATTACGGTTTCTAATCATAC - 3' (SEQ ID NO: 6); the KpnI site is underlined). The resulting gp64 gene DNA fragment was inserted into pENTR/D-TOPO to construct a plasmid pENTR-gp64. The plasmid thus constructed was designated pENTR-gp64.

### (1.3) Construction of Transfer Vector pDual-Hsp65-gp64

pBACsurf-Hsp65 was cleaved with PstI/Cfr9I, and the hsp65 gene DNA fragment was inserted into the PstI/Cfr9I site of pENTR-gp64 to construct a plasmid pENTR-Hsp65-gp64. The pENTR-Hsp65-gp64 was cleaved with RsrII/KpnI, and a DNA fragment consisting of a polyhedrin promoter and hsp65-gp64 gene was inserted into pTriEx-3.1 (Novagen) cleaved with RsrII and KpnI to construct a transfer vector plasmid pDual-Hsp65-gp64 enabling the expression of a fusion protein of Hsp65 antigen and gp64 protein in mammalian and insect cells under the control of the desired dual promoter consisting of CMA promoter and polyhedrin promoter.

### (1.4) Construction of Transfer Vector pDual-H1N1/HA1-gp64

RNA was extracted from a culture supernatant of MDCK cells infected with influenza virus PR/8/34 strain using a QIAamp MinElute Virus Spin Kit (from Qiagen), and amplified by RT-PCR using primers HA-f (5' - CCTGCAGGTATGAAGGCAAACCTACTGGTC - 3' (SEQ ID NO: 7) ; the SbfI site is underlined) and HA-r (5' - GCCCGGGCGATGCATATTCTGCA - 3 (SEQ ID NO: 8); the SbfI site is underlined). The resulting influenza virus HA gene fragment was cloned into pCR-Blunt II-TOPO (from Invitrogen). The resulting plasmid was designated as pCR-Blunt-HA. PCR was performed with the pCR-Blunt-HA as a template using primers pHA-F1 (5' - CACCGAATTCGACACAATATGTATAGGCTACCATGCG-3' (SEQ ID NO: 9); the EcoRI site is underlined) and pHA-R1 (5' - CCCGGGCACCTCTGGATTGGATGGACGGAATG - 3' (SEQ ID NO: 10); the Cfr9I site is underlined). The resulting H1N1/HA1 gene DNA fragment was cloned into pENTR/D-TOPO, then cleaved with EcoRI/Cfr9I and inserted into the EcoRI/Cfr9I site of pDual-Hsp65-gp64 to construct a plasmid pDual-H1N1/HA1-gp64.

### (1.5) Construction of Plasmid pBACsurf-HA1

pDual-H1N1/HA1-gp64 was cleaved with EcoRI/CfrI, and the DNA fragment of H1N1/HA1 gene was inserted into pBACsurf-Hsp65 digested with EcoRI and CfrI to construct a plasmid pBACsurf-HA1.

### (1.6) Construction of Plasmid pCP-H1N1/HA1-gp64

PCR was performed with pBACsurf-HA1 as a template using Polh-f RsrII (5' - GGGCGGACCGGATAATTAAAATGATAACCATCTCG - 3' (SEQ ID NO: 11); the RsrII site is underlined) and GP64-r DraIII (5' - GGGCACTTAGTGATATTGTCTATTACGGTTTCTAATC - 3' (SEQ ID NO: 12); the DraIII site is underlined). The resulting DNA fragment was inserted into pDual-H1N1/HA1-gp64 digested with RsrII and DraIII to yield pCP-H1N1/HA1-gp64.

### (1.7) Construction of Plasmid pCAP-H1N1/HA1-gp64

pCP-H1N1/HA1-gp64 was cleaved with restriction enzymes RsrII and DraIII to prepare HA1 and gp64 gene fragments. The fragments were inserted into a vector prepared by digesting pTriEx-1.1 (from Novagen) with restriction enzymes RsrII and DraIII to yield a transfer vector plasmid pCAP-H1N1/HA1-gp64 enabling the expression of a fusion protein of HA1 antigen and gp64 protein in mammalian and insect cells under the control of the desired dual promoter consisting of CAG promoter and polyhedrin promoter.

### (1.8) Construction of Plasmid pCAP-H1N1/NP-gp64

RT-PCR was performed with genomic RNA of influenza virus PR/8/34 strain as a template using NP-f EcoRI (5'-ACGGAATTCCATTCAATTCAAACTGGA - 3' (SEQ ID NO: 13); the EcoRI site is underlined) and NP-r Cfr9I (5'. GATCCCGGGCCTTGTCAATGCTGAATGGCAA - 3' (SEQ ID NO: 14); the Cfr9I site is underlined). The obtained fragments were digested with restriction enzymes EcoRI and Cfr9I, and inserted into pCAP-H1N1/HA1-gp64 digested with restriction enzymes EcoRI and Cfr9I to yield pCAP-H1N1/NP-gp64.

### (1.9) Construction of Plasmids pCAP-H1N1/NP/272 and pCAP-H1N1/NP/467

PCR was performed using gp64(272)-f (5' - GACTCCCCGGGTCGAGCACCGAGTCAAGAAG - 3' (SEQ ID NO: 15); the XmaI site is underlined), gp64(467)-f (5'-GACTCCCCGGGACATCACTTCCATGGCTGAA-3' (SEQ ID NO: 16); the XmaI site is underlined), and GP64-r DraIII (5'-GGGCACTTAGTGATATTGTCTATTACGGTTTCTAATC-3' (SEQ ID NO: 12); the DraIII site is underlined) of pCAP-H1N1/HA1-gp64. The obtained fragments were digested with restriction enzymes XmaI and DraIII, and inserted into pCAP-H1N1/NP-gp64 digested with XmaI and DraIII to yield pCAP-H1N1/NP/272 and pCAP-H1N1/NP/467.

### (1.10) Construct of Transfer Vectors pCAP-PfCSP, cCAP-PfCSP/272 and pCAP-PfCSP/467

*P. falciparum* genomic DNA was extracted from human erythrocytes infected with *Plasmodium falciparum* 3D7 strain using a QIAamp DNA Midi Kit (from Qiagen). A PfCSP gene was cloned by PCR with this genomic DNA as a template according to the following method. The PCR was performed using primers PfCSP-f(19)(5' - GACTCTGCAGTTATTCCAGGAATACCAGTGCTATGGAAG - 3' (SEQ ID NO: 17); the PstI site is underlined) and PfCSP-r(373)(5'-CGATCCCGGGCTTTTTCCATTTTACAAATTTTTTTTTCAATATC - 3' (SEQ ID NO: 18); the XmaI site is underlined). The resulting DNA fragment was inserted into pCAP-H1N1/NP-gp64, pCAP-H1N1/NP/272, and pCAP-H1N1/NP/467, each digested with PstI and XmaI. The constructed plasmids were designated pCAP-PfCSP, pCAP-PfCSP/272, and pCAP-PfCSP/467.

The GenBank accession number of the amino acid sequence of the *Plasmodium falciparum* 3D7 circumporozoite (CS) protein is XP_001351122.

### (2) Construction of Transfer Vector pDual-Pfs25-PfCSP-gp64

### (2.1) Construction of Plasmid pDual-PbAMAlD123-gp64

A blood sample was collected from a BALB/c mouse infected with malaria parasite *P*. *berghei* ANKA strain, and P. *berghei* genomic DNA was extracted using a QIAamp DNA Midi Kit (Qiagen). A PbAMA1 gene domain 123 (D123) was cloned by PCR with this genomic DNA as a template according to the following method. PCR was performed using primers pAMA-F1 (5' - CACCGAATTCAATCCATGGGAAAAGTATACGGAAAAATAT - 3' (SEQ ID NO: 19); the EcoRI site is underlined) and pAMA1-R1 (5' - CCCGGGCTTCTCTGGTTTGATGGGCTTTCATATGCAC - 3' (SEQ ID NO: 20); the Cfr9I site is underlined). The resulting PbAMA1D123 DNA fragment was cloned into pENTR/D-TOPO, then cleaved with EcoRI/Cfr9I and inserted into pBACsurf-Hsp65 digested with EcoRI and Cfr9I. The constructed plasmid was designated pBACsurf-PbAMA1D123.

Subsequently, the pBACsurf-PbAMA1D123 was cleaved with EcoRI/Cfr9I, and the PbAMA1D123 gene DNA fragment was inserted into pDual-Hsp65-gp64 digested with EcoRI and Cfr9I to yield a plasmid pDual-PbAMA1D123-gp64.

### (2.2) Construction of Plasmid pDual-PfCSP-gp64

A PfCSP gene was cloned by PCR with *P*. *falciparum* genomic DNA as a template according to the following method. The PCR was performed using primers pPfCSP-F1 (5' - CACCGAATTCTTATTCCAGGAATACCAGTGCTATGGAAGT-3' (SEQ ID NO: 21); the EcoRI site is underlined) and pPfCSP-R1 (5'-CCCGGGCTTTTTCCATTTTACAAATTTTTTTTTC-3' (SEQ ID NO: 22); the Cfr9I site is underlined). The resulting PfCSP DNA fragment was cloned into pENTR/D-TOPO, then cleaved with EcoRI/Cfr9I and inserted into pDual-PbAMA1D123-gp64 digested with EcoRI and Cfr9I. The constructed plasmid was designated pDual-PfCSP-gp64.

### (2.3) Construction of Transfer Vector pDual-Pfs25-PfCSP-gp64

A Pfs25 gene was cloned by PCR with *P*. *falciparum* genomic DNA as a template according to the following method. The PCR was performed using primers pPfs25-F1 (5' - CACCGAATTCAAAGTTACCGTGGATACTGTATGCAAAAGAGGA - 3' (SEQ ID NO: 23); the EcoRI site is underlined) and pPfs25-R2 (5' - CAATTGAGATCCGCCGCCACCGCCACCAGTACATATAGAGCTTTCATTATCTATTATAAATCCAT C - 3' (SEQ ID NO: 24); the MunI site is underlined). The resulting Pfs25 DNA fragment was cloned into pENTR/D-TOPO, then cleaved with EcoRI/MunI, and inserted into pDual-PfCSP-gp64 digested with EcoRI. The constructed plasmid was designated pDual-Pfs25-PfCSP-gp64.

### (3) Construction of Transfer Plasmid pDual-PfMSP1-PfCSP-gp64

A PfMSP1 gene was cloned by PCR with *P*. *falciparum* genomic DNA as a template according to the following method. The PCR was performed using primers pPfMSPH9-F1 (5' - CACCGAATTCAACATTTCACAACACCAATGCGTAAAAAAAC - 3': (SEQ ID NO: 25); the EcoRI site is underlined) and pPfMSP119-R2 (5' - CAATTGAGATCCGCCGCCACCGCCACCGTTAGAGGAACTGCAGAAAATACCATCGAAAAGTGGA - 3' (SEQ ID NO: 26); the MunI site is underlined). The resulting PfMSP119 DNA fragment was cloned into pENTR/D-TOPO, then cleaved with EcoRI and MunI, and inserted into pDual-PbCSP-gp64 digested with EcoRI. The constructed plasmid was designated pDual-PfMSP1-PfCSP-gp64.

### (4) Construction of the Transfer Vector Plasmids pCAP-PfCSP(A361E), pCAP-PfCSP(A361E)/272, and pCAP-PfCSP(A361E)/467

PCR was performed with pCAP-PfCSP using PfCSP-f(19)(5'-GACTCTGCAGTTATTCCAGGAATACCAGTGCTATGGAAG-3': (SEQ ID NO: 17); the PstI site is underlined) and PfCSP-r(373 A361E) (5'-CGATCCCGGGCTTTTTCCATTTTACAAATTTTTTTTTCAATATCATTTTC-3': (SEQ ID NO: 27); the XmaI site is underlined). The obtained DNA fragment was cleaved with PstI and XmaI, and inserted into pCAP-H1N0/NP-gp64, pCAP-H1N1/NP/272, and pCAP-H1N1/NP/467, each digested with PstI and XmaI. The constructed plasmids were designated pCAP-PfCSP(A361E), pCAP-PfCSP(A361E)/272, and pCAP-PfCSP(A361E)/467.

### (5) Construction of Transfer Plasmids pCAP-PfCSP-76 and pCAP-PfCSP-76/467

PCR was performed with pCAP-PfCSP(A361E) using PfCSP-f(76) (5' -GACTCTGCAGGATGATGGAAATAACGAAGACAACG - 3': (SEQ ID NO: 28); the PstI site is underlined) and PfCSP-r(373 A361E) (5' - CGATCCCGGGCTTTTTCCATTTTACAAATTTTTTTTTCAATATCATTTTC - 3': (SEQ ID NO: 27); the XmaI site is underlined). The resulting DNA fragment was cleaved with PstI and XmaI, and then inserted into pCAP-H1N1/NP-gp64 and pCAP-H1N1/NP/467, each cleaved with PstI and XmaI. The constructed plasmids were designated pCAP-PfCSP-76 and pCAP-PfCSP-76/467.

### (6) Construction of Transfer Plasmids pCAP-PfCSP+209 and pCAP-PfCSP+209/467

An artificial gene sequence (PfCSP+: SEQ ID NO: 29) was prepared from the amino acid sequence of PfCSP of *P. falciparum* 3D7 strain (in which, however, the A at the 361-position was replaced by E) using codons frequently used in Sf9 and human cells. Using the obtained artificial gene sequence as a template, PCR was performed using PfCSP-f(+209) (5'-GACTCTGCAGAACGCTAATCCAAACGCTAATCCCAACGCTAATCCCAATGCC -3' (SEQ ID NO: 30); the PstI site is underlined) and PfCSP-r(+A361E) (5'-CGATCCCGGGCTTTTTCCATTTTGCAAATTTTTTT -3' (SEQ ID NO: 31); the XmaI site is underlined). The resulting DNA fragments were cleaved with PstI and XmaII, and then inserted into pCAP-H1N1/NP-gp64 and pCAP-H1N1/NP/467 digested with PstI and XmaII. The constructed plasmids were designated pCAP-PfCSP+209 and pCAP-PfCSP+209/467.

### (7) Construction of Transfer Plasmids pCAP-PfCSP+76/209 and pCAP-PfCSP+76/209/467

Using the artificial gene sequence (PfCSP+: SEQ ID NO: 29) as a template, PCR was performed using PfCSP-f(+76) (5'-GACTCTGCAGGACGACGGCAACAACGAAGACAACG -3' (SEQ ID NO: 32); the PstI site is underlined), PfCSP-r(+128) (5'-CGTTAGGATCCACATTTGGGTTGGCATTTGGG -3' (SEQ ID NO: 33); the BamH I site is underlined), PfCSP-f (+209) BamHl (5'-GACTGGATCCTAACGCTAATCCAAACGCTAATCCC- 3':(SEQ ID NO: 34); the BamH I site is underlined), and PfCSP-r(+A361E) (5'-CGATCCCGGGCTTTTTCCATTTTGCAAATTTTTTT -3' (SEQ ID NO: 31); the XmaI site is underlined) from the obtained artificial gene sequence. The resulting DNA fragments were cleaved with PstI/BamHl and BamH1/XmaI, respectively, and then inserted into pCAP-H1N1/NP-gp64 and pCAP-H1N1/NP/467, each digested with PstI and XmaI. The constructed plasmids were designated pCAP-PfCSP+76/209 and pCAP-PfCSP+76/209/467.

### (8) Construction of Transfer Plasmids pCAP-HA1/Anhui, pCAP-HA1/Anhui/272, and pCAP-HA1/Anhui/467

An artificial gene sequence (SEQ ID NO: 35) was prepared from the amino acid sequence of the hemagglutinin HA1 region of influenza virus H5N1/Anhui/1/05 using codons frequently used in Sf9 and human cells. Using the obtained artificial gene sequence as a template, PCR was performed using AH-F1 (5'-CAGTCTGCAGGACCAGATTTGCATC-3': (SEQ ID NO: 36); the PstI site is underlined) and AH-R4 (5'-CAGTCCCGGGCTCTCTTGCGCCTGC-3': (SEQ ID NO: 37); the XmaI site is underlined). The obtained DNA fragment was cleaved with PstI and XmaI, and then inserted into pCAP-H1N1/NP-gp64, pCAP-H1N1/NP/272 and pCAP-H1N1/NP/467, each digested with PstI and XmaI. The constructed plasmids were designated pCAP-HA1/Anhui, pCAP-HA1/Anhui/272, and pCAP-HA1/Anhui/467.

The GenBank accession number of the amino acid sequence of the hemagglutinin of influenza virus A/H5N1/Anhui/1/05 is ABD28180.

### (9) Construction of Transfer Vector Plasmids pCAP-HA1/Vietnam, pCAP-HA1/Vietnam/51, pCAP-HA1/Vietnam/101, pCAP-HA1/Vietnam/154, pCAP-HA1/Vietnam/201, pCAP-HA1/Vietnam/272, and pCAP-HA1/Vietnam /467

An artificial gene sequence (SEQ ID NO: 38) was prepared from the amino acid sequence of the HA1 region of the hemagglutinin of influenza virus H5N1/Vietnam/1203/4 using codons frequently used in Sf9 and human cells. Using the obtained artificial gene sequence as a template, PCR was performed using VN-F1 (5'-CAGTCTGCAGGACCAGATCTGTATC-3': (SEQ ID NO: 39); the PstI site is underlined), and VN-R4 (5'-CAGTCCCGGGCTCTCTTCTTCCTGC-3': (SEQ ID NO: 40); the XmaI site is underlined). The obtained DNA fragment was cleaved with PstI and XmaI, and inserted into pCAP-H1N1/NP-gp64, pCAP-H1N1/NP/272 and pCAP-H1N1/NP/467, each digested with PstI and XmaI. The constructed plasmids were designated pCAP-HA1/Vietnam, pCAP-HA1/Vietnam/272, and pCAP-HA1/Vietnam/467.

Further, using pCAP-HA1/Vietnam as a template, PCR was performed using gp64(51)-f (5'-GACTCCCCGGGTGGAAATCACCATCGTGGAGACG-3': (SEQ ID NO: 41); the XmaI site is underlined), or gp64(101)-f (5'-GACTCCCCGGGATTTGCTTATGTGGAGCATCAGG-3': (SEQ ID NO: 42); the XmaI site is underlined), or gp64(154)-f (5'-GACTCCCCGGGCGCACCACACGTGCAACAAATCG-3': (SEQ ID NO: 43); the XmaI site is underlined), or gp64(201)-f (5'-GACTCCCCGGGACACTGTGCTTCATCGAGACGGC-3': (SEQ ID NO: 44); the XmaI site is underlined), and GP64-r DraIII (5'-GGGCACTTAGTGATATTGTCTATTACGGTTTCTAATC-3' (SEQ ID NO: 12); the Dralll site is underlined). The obtained DNA fragments were cleaved with XmaI and DraIII, and inserted into pCAP-HA1/Vietnam digested with XmaI and DraIII. The constructed plasmids were designated pCAP-HA1/Vietnam/51, pCAP-HA1/Vietnam/101, pCAP-HA1/Vietnam/154, and pCAP-HA1/Vietnam/201.

The GenBank accession number of the amino acid sequence of the hemagglutinin of influenza virus A/H5N1/Vietnam/1203/2004 is AAW80717.

### (10) Construction of Transfer Vector Plasmids pCAP-AH/345, pCAP-AH/345/467, pCAP-AH/410, pCAP-AH/410/467, pCAP-AH/473, pCAP-AH/473/467, pCAP-AH/520, pCAP-AH/520/467

An artificial gene sequence (SEQ ID NO: 45) was prepared from the amino acid sequence of the HA region of the hemagglutinin of influenza virus A/H5N1/Anhui/1/05 by codon optimization using Gene Designer available from DNA2.0, Inc. Using this artificial sequence as a template, PCR was perfomed using AH17-F (5'-GACTCTGCAGGATCAGATCTGTATTGGGTACC-3' : (SEQ ID NO: 46); the PstI site is underlined, and AH345-R (5'-CGATCCCGGGCTCTCTTTCTCCTCCGCTCGC-3': (SEQ ID NO: 47); the XmaI site is underlined), or AH410-R (5'-CGATCCCGGGCGGCCTCGAACTGGGTGTTCATT-3': (SEQ ID NO: 48); the XmaI site is underlined), or AH473-R (5'-CGATCCCGGGCGTCTCTGAGTTGAAGGCGCAC-3':(SEQ ID NO: 49); the XmaI site is underlined, or AH520-R (5'-CGATCCCGGGCACCACTAATTTCCTCTCGCTTC-3':(SEQ ID NO: 50); the XmaI site is underlined). The obtained DNA fragment was cleaved with PstI and XmaI, and inserted into pCAP-HA1/Anhui or pCAP-HA1/Anhui/467 digested with PstI and XmaI. The constructed plasmids were designated pCAP-AH/345, pCAP-AH/345/467, pCAP-AH/410, pCAP-AH/410/467, pCAP-AH/473, pCAP-AH/473/467, pCAP-AH/520, and pCAP-AH/520/467.

### (11) Construction of Transfer Vector Plasmids pCAP-VN/346, pCAP-VN/346/467, pCAP-VN/410, pCAP-VN/410/467, pCAP-VN/473, pCAP-VN/473/467, pCAP-VN/520, and pCAP-VN/520/467

An artificial gene sequence (SEQ ID NO: 51) was prepared from the amino acid sequence of the HA region of the hemagglutinin of influenza virus A/H5N1/Vietnam/1203/2004 by codon optimization using Gene Designer available from DNA2.0, Inc. Using this artificial sequence as a template, PCR was performed using VN17-F (5'-GACTCTGCAGGATCAGATCTGTATCGGATATC-3': (SEQ ID NO: 52); the PstI site is underlined), and VN346-R (5'-CGATCCCGGGCCCGCTTTTTCCTCCTCCGTTCG-3': (SEQ ID NO: 53); the XmaI site is underlined), or VN410-R (5'-CGATCCCGGGCCTCAAACTGCGTATTCATTTTG-3': (SEQ ID NO: 54); the XmaI site is underlined), or VN473-R (5'-CGATCCCGGGCTCTAAGCTGGAGCCTGACTTTGTC-3': (SEQ ID NO: 55); the XmaI site is underlined), or VN520-R (5'-CGATCCCGGGCACTAATCTCCTCTCTTTTAAGTC-3': (SEQ ID NO: 56); the XmaI site is underlined). The obtained DNA fragment was cleaved with PstI and XmaI, and inserted into pCAP-HA1/Anhui or pCAP-HA1/Anhui/467 digested with PstI and XmaI. The constructed plasmids were designated pCAP-VN/346, pCAP-VN/346/467, pCAP-VN/410, pCAP-VN/410/467, pCAP-VN/473, pCAP-VN/473/467, pCAP-VN/520, and pCAP-VN/520/467.

### (12) Construction of Transfer Vector Plasmids pCAP-CO/full, pCAP-CO/full/467, pCAP-CO/19, pCAP-CO/19/467, pCAP-CO/76, pCAP-CO/76/467, pCAP-CO/205, and pCAP-CO/205/467

An artificial gene sequence (SEQ ID NO: 57) was prepared from the amino acid sequence of the CSP of *Plasmodium falciparum* 3D7 strain by codon optimization using Gene Designer available from DNA2.0, Inc. Using this artificial sequence as a template, PCR was performed using a pair of primers consisting of PfCSP_opt-f (5'-GACTCTGCAGATGATGCGAAAATTGGCCATACTG-3': (SEQ ID NO: 58); the PstI site is underlined) and PfCSP_opt-r (397) (5'-CGATCCCGGGCATTGAGGAACAGAAAGGAAAGAACCATG-3': (SEQ ID NO: 59); the XmaI site is underlined); PfCSP_opt-f (19) (5'-GACTCTGCAGCTGTTTCAGGAATACCAGTGCTATGG-3': (SEQ ID NO: 60); (the PstI site is underlined) and PfCSP_opt-r (373) (5'-CGATCCCGGGCCTTCTCCATCTTACAAATTTTCTTTTCAATATCATTAGC-3': (SEQ ID NO: 61); the XmaI site is underlined); PfCSP_opt-f (76) (5'-GACTCTGCAGGACGACGGAAATAATGAGGACAACG-3' : (SEQ ID NO: 62); the PstI site is underlined) and PfCSP_opt-r (373) (5'-CGATCCCGGGCCTTCTCCATCTTACAAATTTTCTTTTCAATATCATTAGC-3': (SEQ ID NO: 61); the XmaI site is underlined); and PfCSP_opt-f (205) (5'-GACTCTGCAGAATGCAAACCCAAATGCCAATCCAAACGC-3': (SEQ ID NO: 63); the PstI site is underlined) and PfCSP_opt-r (373) (5'-CGATCCCGGGCCTTCTCCATCTTACAAATTTTCTTTTCAATATCATTAGC-3': (SEQ ID NO: 61); the XmaI site is underlined). The obtained DNA fragments were cleaved with PstI and XmaI, and inserted into pCAP-HA1/Anhui or pCAP-HA1/Anhui/467 digested with PstI and XmaI. The constructed plasmids were designated pCAP-CO/full, pCAP-CO/full/467, pCAP-CO/19, pCAP-CO/19/467, pCAP-CO/76, pCAP-CO/76/467, pCAP-CO/205, and pCAP-CO/205/467.

### (13) Construction of Transfer Vector Plasmids pCA64-HA1/Anhui and pCA64-PfCSP (A361E)

Using the Triple Cut DNA of BacVector-2000 DNA (from Novagen), PCR was performed using gp64-p-f (5'-GACTCGGACCGGCCAGATAAAAATAATCTTATCAATTAAG-3': (SEQ ID NO: 64); the RsrII site is underlined) and gp64-p-r (5'-CGATACTAGTAGCACTGAGGCTTCTTATATACCCG-3': (SEQ ID NO: 65); the SpeI site is underlined). The obtained DNA fragment was cleaved with RsrII and SpeI, and inserted into pCAP-HA1/Anhui or pCAP-PfCSP(A361E) digested with RsrII and SpeI to construct transfer vector plasmids pCA64-HA1/Anhui and pCA64-PfCSP(A361E) enabling the expression of a fusion protein of HA1 antigen or PfCSP antigen and gp64 protein in mammalian and insect cells under the control of the desired dual promoter consisting of CAG promoter and gp64 promoter.

### (14) Construction of Transfer Vector Plasmids pCA39-HA1/Anhui and pCA39-PfCSP (A361E)

Using the Triple Cut DNA of BacVector-2000 DNA (from Novagen), PCR was performed using vp39-p-f (5'-GACTCGGACCGCGTCGTACAAATCGAAATATTGTTGTG-3':(SEQ ID NO: 66); the RsrII site is underlined) and vp39-p-r (5'-CGATACTAGTGTGATTGAGAAAGAAATCTCTTATTC-3': (SEQ ID NO: 67); the SpeI site is underlined). The obtained DNA fragment was cleaved with RsrII and SpeI, and inserted into pCAP-HA1/Anhui or pCAP-PfCSP(A361E) digested with RsrII and SpeI to construct transfer vector plasmids pCA39-HA1/Anhui and pCA39-PfCSP(A361E) enabling the expression of a fusion protein of HA1 antigen or PfCSP antigen and gp64 protein in mammalian and insect cells under the control of the desired dual promoter consisting of CAG promoter and vp39 promoter.

### (15) pCAP-CO/full/VSV, pCAP-CO/19/VSV, pCAP-CO/76/VSV, and pCAP-CO/205/VSV

Using pVSV-G (from Clonetech) as a template, PCR was performed using VSV-G-f (5'-GACTCCCCGGGCGTTCGAACATCCTCACATTCAAG - 3' (SEQ ID NO: 68); the XmaI site is underlined), VSV-G-r (5'-GACTCACTTAGTGCTTTCCAAGTCGGTTCATCTC-3': (SEQ ID NO: 69); the DraIII site is underlined). The obtained DNA fragment was inserted into pCAP-CO/full, pCAP-CO/19, pCAP-CO/76, and pCAP-CO/205, each digested with XmaI and DraIII. The constructed plasmids were designated pCAP-CO/full/VSV, pCAP-CO/19/VSV, pCAP-CO/76/VSV, and pCAP-CO/205/VSV.

### Example 2: Recombinant Baculoviruses of the Present disclosure and Method for Production thereof

(1) Recombinant baculoviruses were produced using a kit for producing recombinant baculoviruses (BacVector-2000 Transfection Kit from Novagen) by co-transfecting BacVector-2000 DNA with each of the following transfer vectors constructed in Example 1: pCAP-PfCSP, pCAP-PfCSP/272, pCAP-PfCSP/467, pCAP-PfCSP(A361E), pCAP-PfCSP(A361E)/272, pCAP-PfCSP(A361E)/467, pCAP-PfCSP-76, pCAP-PfCSP-76/467, pCAP-PfCSP+209, pCAP-PfCSP+209/467, pCAP-PfCSP+76/209, pCAP-PfCSP+76/209/467, pCAP-HA1/Anhui, pCAP-HA1/Anhui/272, pCAP-HA1/Anhui/467, pCAP-HA1/Vietnam, pCAP-HA1/Vietnam/51, pCAP-HA1/Vietnam/101, pCAP-HA1/Vietnam/154, pCAP-HA1/Vietnam/201, pCAP-HA1/Vietnam/272, pCAP-HA1/Vietnam/467, pCAP-AH/345, pCAP-AH/345/467, pCAP-AH/410, pCAP-AH/410/467, pCAP-AH/473, pCAP-AH/473/467, pCAP-AH/520, pCAP-AH/520/467, pCAP-VN/346, pCAP-VN/346/467, pCAP-VN/410, pCAP-VN/410/467, pCAP-VN/473, pCAP-VN/473/467, pCAP-VN/520, pCAP-VN/520/467, pCAP-CO/full, pCAP-CO/full/467, pCAP-CO/19, pCAP-CO/19/467, pCAP-CO/76, pCAP-CO/76/467, pCAP-CO/205, pCAP-CO/205/467, pCA39-HA1/Anhui, pCA64-HA1/Anhui, pCA39-PfCSP(A361E), pCA64-PfCSP(A361E), pCAP-CO/full/VSV, pCAP-CO/19/VSV, pCAP-CO/76/VSV, pCAP-CO/205/VSV, pDual-Pfs25-PfCSP-gp64, pDual-PfMSP1-PfCSP-gp64 into Sf9 cells. The resulting recombinant baculoviruses were designated AcNPV-CAP-PfCSP, AcNPV-CAP-PfCSP/272, AcNPV-CAP-PfCSP/467, AcNPV-CAP-PfCSP(A361E), AcNPV-CAP-PfCSP(A361E)/272, AcNPV-CAP-PfCSP(A361E)/467, AcNPV-CAP-PfCSP-76, AcNPV-CAP-PfCSP-76/467, AcNPV-CAP-PfCSP+209, AcNPV-CAP-PfCSP+209/467, AcNPV-CAP-PfCSP+76/209, AcNPV-CAP-PfCSP+76/209/467, AcNPV-CAP-HA1/Anhui, AcNPV-CAP-HA1/Anhui/272, AcNPV-CAP-HA1/Anhui/467, AcNPV-CAP-HA1/Vietnam, AcNPV-CAP-HA1/Vietnam/51, AcNPV-CAP-HA1/Vietnam/101, AcNPV-CAP-HA1/Vietnam/154, AcNPV-CAP-HA1/Vietnam/201, AcNPV-CAP-HA1/Vietnam/272, AcNPV-CAP-HA1/Vietnam/467, AcNPV-CAP-AH/345, AcNPV-CAP-AH/345/467, AcNPV-CAP-AH/410, AcNPV-CAP-AH/410/467, AcNPV-CAP-AH/473, AcNPV-CAP-AH/473/467, AcNPV-CAP-AH/520, AcNPV-CAP-AH/520/467, AcNPV-CAP-VN/346, AcNPV-CAP-VN/346/467, AcNPV-CAP-VN/410, AcNPV-CAP-VN/410/467, AcNPV-CAP-VN/473, AcNPV-CAP-VN/473/467, AcNPV-CAP-VN/520, AcNPV-CAP-VN/520/467, AcNPV-CAP-CO/full, AcNPV-CAP-CO/full/467, AcNPV-CAP-CO/19, AcNPV-CAP-CO/19/467, AcNPV-CAP-CO/76, AcNPV-CAP-CO/76/467, AcNPV-CAP-CO/205, AcNPV-CAP-CO/205/467, AcNPV-CA39-HA1/Anhui, AcNPV-CA64-HA1/Anhui, AcNPV-CA39-PfCSP(A361E), AcNPV-CA64-PfCSP(A361E), AcNPV-CAP-CO/full/VSV, AcNPV-CAP-CO/19/VSV, AcNPV-CAP-CO/76/VSV, AcNPV-CAP-CO/205/VSV, AcNPV-Dual-Pfs25-PfCSP-gp64, and AcNPV-Dual-PfMSP1-PfCSP-gp64.

### Example 3: Test of Expression of Vaccine Antigen from Recombinant Baculovirus of the Present disclosure in Insect Cells

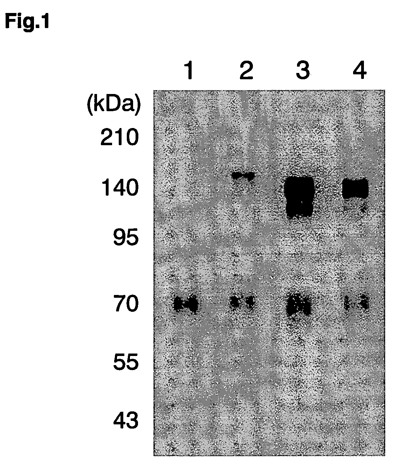

Sf9 cells were cultured at a concentration of 1 x 10⁵ cells per well in a 48-well plate (from Corning), and infected with baculoviruses AcNPV-CAP-PfCSP, AcNPV-CAP-HA1/Anhui, and AcNPV-CAP-HA1/Vietnam obtained in Example 2, or a wild-type baculovirus AcNPV-WT as a control at an infection multiplicity of about 0.1. After 5 days, the culture supernatant was removed from each well, and then Sample Buffer Solution (+2ME, x2) (from Wako) was added in an amount of 0.05 mL per well to completely lyse the cells. The cell lysate was heated at 100°C for 5 minutes, and electrophoresed on 7.5% SDS-PAGE. After electrophoresis, the protein was transferred to a PVDF membrane (Immobilon-P from Millipore), and blocking was performed at 4°C overnight by immersing the membrane in 2.5% Skim Milk/SuperBlock (from Pierce). The membrane to which the protein of Sf9 cells infected with each baculovirus had been transferred was incubated with an anti-gp64 antibody (AcV5 from eBioScience) as the primary antibody, and then incubated with a HRP-labeled goat anti-mouse IgG (H+L) antibody (from BioRad) as the second antibody. Color was developed with an ECLplus Western Blotting Detection kit (from GE Healthcare) to detect the protein band. FIG. 1 shows the results.

FIG. 1 shows Western blotting analysis showing the expression of fusion antigens in insect cells from recombinant baculoviruses containing PfCSP gene of human malaria, HA1 gene of influenza virus H5N1/Anhui/1/05 strain, and HA1 gene of H5N1/Vietnam/1203/04 strain. In FIG. 1, Lane 1 shows the band of a wild-type baculovirus (AcNPV-WT); Lane 2 shows the band of a recombinant baculovirus (AcNPV-CAP-PfCSP) containing PfCSP gene and full-length gp64 gene inserted downstream of the dual promoter used in the present invention Lane 3 shows the band of a recombinant baculovirus (AcNPV-CAP-HA1/Anhui) containing HA1 gene of influenza virus H5N1/Anhui/1/05 strain and full-length gp64 gene inserted downstream of the dual promoter used in the present invention; and Lane 4 shows the band of a recombinant baculovirus ((AcNPV-CAP-HA1/Vietnam) containing HA1 gene of influenza virus H5N1/Vietnam/1203/04 strain and full-length gp64 gene inserted downstream of the dual promoter used in the present invention.

As shown in Lanes 2, 3 and 4 of FIG. 1, a band corresponding to the expressed fusion product of an immunogenic foreign antigen gene and gp64 gene was observed in the recombinant baculoviruses having an antigen gene and gp64 gene fused and expressed downstream of the dual promoter used in the present invention.

The above results confirmed that when using the recombinant virus used in the present invention, an immunogenic foreign antigen gene and gp64 gene can be fused and expressed as an expressed fusion product in insect cells.

### Example 4: Test of Identification of Fusion Antigen in Vaccine Antigen Presented on Viral Particle (Virion) of Recombinant Baculovirus of the Present disclosure

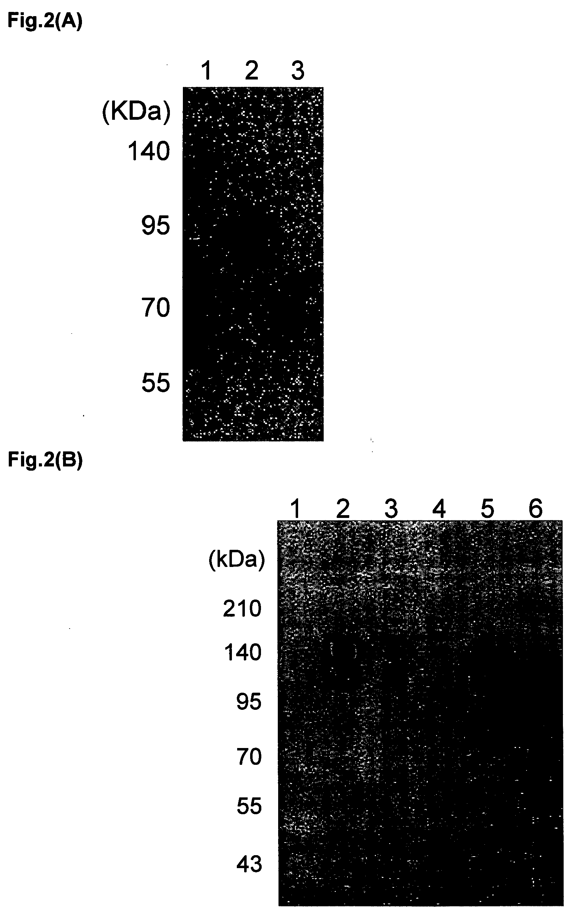

Sf9 cells were cultured to a concentration of 1 x 10⁷ cells per 150 mm cell culture plate (from Sumilon), and infected with each of the above-mentioned baculoviruses at an infection multiplicity of about 0.1. After 7 days, the medium was centrifuged at 3,000 xg at 4°C for 15 minutes twice, and the virus solution was layered over a 25% sucrose solution, and centrifuged using an ultracentrifuge at 25,000 rpm at 4°C for 90 minutes to yield viral particles. 0.05 mL of Sample Buffer Solution (+2ME, x2) (from Wako) was added to 0.05 mL each of the virus concentrates (1 x 10⁸ PFU/mL) of AcNPV-CAP-PfCSP, AcNPV-CAP-PfCSP/272, AcNPV-CAP-PfCSP/467, AcNPV-CAP-HA1/Vietnam, and AcNPV-WT collected by ultracentrifugation. The resulting mixtures were heated at 100°C for 5 minutes, and electrophoresed on 7.5% SDS-PAGE. After the electrophoresis, the obtained proteins were transferred to PVDF membranes (Immobilon-P from Millipore), and immersed in 2.5% Skim Milk/SuperBlock (from Pierce) to perform blocking at 4°C overnight. The membranes to which the virus solutions of AcNPV-CAP-PfCSP, AcNPV-CAP-PfCSP/272, and AcNPV-CAP-PfCSP/467 had been transferred were incubated with an anti-PfCSP antibody (2A10, MR-4) as the primary antibody, and then incubated with a HRP-labeled goat anti-mouse IgG (H+L) antibody (from BioRad) as the second antibody. The membrane to which the virus solution of AcNPV-CAP-HA1/Vietnam had been transferred was incubated with an anti-H5N1 antibody (IT-003-005 from Immune Technology) as the primary antibody, and then incubated with a HRP-labeled goat anti-rabbit IgG antibody (from GE Healthcare) as the second antibody. Color was developed with an ECLplus Western Blotting Detection kit (from GE Healthcare) to detect the bands of the proteins. FIG. 2 shows the results.

FIG. 2 (A) shows Western blotting analysis showing the expression of the CSP gene (PfCSP) of human malaria in viral particles of recombinant baculoviruses prepared using recombinant transfer vectors. Lane 1, the left side lane of FIG. 2 (A), shows the band of a recombinant baculovirus (AcNPV-CAP-PfCSP) containing PfCSP gene and full-length gp64 gene inserted downstream of the dual promoter used in the invention; Lane 2 shows the band of a recombinant baculovirus (AcNPV-CAP-PfCSP/272) containing PfCSP gene and partial-length gp64 gene (241 amino acid residues from the C terminus) inserted downstream of the dual promoter used in the invention; Lane 3 shows the band of a recombinant baculovirus (AcNPV-CAP-PfCSP/467) containing PfCSP gene and partial-length gp64 gene (46 amino acid residues from the C terminus) inserted downstream of the dual promoter used in the invention. The baculoviruses were electrophoresed, and the presence of an expressed fusion product of the PfCSP gene and gp64 gene was checked. A strong band, indicating the presence of a fusion antigen of PfCSP gene and gp64 gene in the recombinant viral particles, was detected in all the lanes of FIG. 2 (A).

FIG. 2 (B) shows Western blotting analysis showing the expression of H5N1/HA1 gene in viral particles of recombinant baculoviruses prepared using recombinant transfer vectors. Lane 1, left side lane of FIG. 2 (B), shows the results obtained using the infected AcNPV-WT cell lysate prepared in Example 3; Lane 2 shows the results obtained using the AcNPV-CAP-HA1/Anhui-infected cell lysate prepared in Example 3; Lane 3 shows the results obtained using the AcNPV-CAP-HA1/Vietnam-infected cell lysate prepared in Example 3; and Lane 4 shows the results of viral particles of a wild-type baculovirus (AcNPV-WT); Lane 5 shows the results of viral particles of a recombinant baculovirus (AcNPV-CAP-HA1/Vietnam) containing the HA1 gene of influenza virus H5N1/Vietnam/1203/04 strain and full-length gp64 gene inserted downstream of the dual promoter used in the present invention; and Lane 6 shows the results of purified HA antigen of H5N1 (IT-003-0053p from Immune Technology). The baculoviruses were electrophoresed, and the presence of an expressed fusion product of the PfCSP gene and gp64 gene was checked. A strong band, indicating the presence of a fusion antigen of HA1 gene of H5N1/Vietnam/1203/04 and gp64 gene in the recombinant viral particles, was detected in Lane 5 of FIG. 2(B).

The above results of Example 4 show that a foreign gene having the desired immunogenicity and gp64 gene can be fused and expressed in recombinant viral particles of the recombinant baculovirus of the present disclosure produced by using the recombinant transfer vector of the present disclosure.

### Example 5: Test of Expression of Vaccine Antigen from Recombinant Baculovirus of the Present disclosure in Mammals

HepG2 cells were infected with AcNPV-Dual-PfMSP1-PfCSP at an infection multiplicity of 1. After 48 hours, the culture supernatant was removed, and the plate was rinsed with PBS three times. An acetone/ethanol solution (a mixed ratio of 7:3) cooled to -20°C was added to immobilize the cells at -20°C for 5 minutes. A 5% normal goat serum (from Sigma) was added to perform blocking at room temperature for 1 hour. To detect the expression of PfCSP, an anti-PfCSP antibody (2A10, MR-4) labeled with Alexa Flour 594 was added; and to detect the expression of PfMSP-1₁₉, an anti-PfMSP-1₁₉ antibody (5.2, MR-4) and then an anti-mouse antibody labeled with FITC were added. After incubation, the reacted cells were detected under a fluorescence microscope.

FIG. 3 shows the results.

FIG. 3 shows HepG2 cells stained with a fluorescence-labeled antibody, which indicates that an antigen has been expressed from a recombinant baculovirus containing a fusion gene of the PfMSP1 gene and PfCSP gene in the HepG2 cells. The results of FIG. 3 (A) confirmed that a PfCSP antigen has been expressed. The results of FIG. 3 (B) confirmed that a PfMSP-1₁₉ antigen has been expressed. It was thus confirmed that fusion antigens can be expressed in mammalian cells. The results of FIG. 3 (A) and (B) clearly show that the recombinant baculovirus produced by using the transfer vector containing the dual promoter of the present disclosure can express the desired antigen in mammalian cells.

This suggests that when the recombinant baculovirus produced using the recombinant transfer vector of the present disclosure is administered to humans and other mammals, the virus particles enter the mammalian cells, and a mammalian promoter operates to produce a fusion product of the desired foreign antigen gene and gp64 gene in the mammalian cells, thus inducing the acquired immunity.

### Example 6: Induction of Antibody by PfCSP Antigen Recombinant Virus and H5N1/HA1 Antigen Recombinant Virus

### 1. Inoculation of Virus Solution

Virus solutions of AcNPV-WT, AcNPV-CAP-PfCSP, AcNPV-CAP-PfCSP/467, AcNPV-CAP-HA1/Anhui, and AcNPV-CAP-HA1/Vietnam concentrated by ultracentrifugation were inoculated into the thigh muscles of BALB/c female mice in an amount of 1 x 10⁸ PUF twice at two week-intervals.

### 2. Measurement of Antibody Titers

The mice were euthanized two weeks after the final immunization, and sera were collected from the mice and used for measuring antigen-specific antibody titers. Induction of PfCSP antigen-specific antibody titers by AcNPV-CAP-PfCSP and AcNPV-CAP-PfCSP/467 was measured by ELISA using a plate on which (NANP)₄NVDPC peptide (from Sigma), i.e., B-cell epitope of PfCSP, had been immobilized. Induction of H5N1/HA antigen-specific antibody titers by AcNPV-CAP-HA1/Anhui and AcNPV-CAP-HA1/Vietnam was measured by ELISA using a plate on which purified HA antigen of H5N1 virus (IT-003-005p from Immune Technology) had been immobilized. The absorbance at OD450 nm was measured using MaxiSorp (from NUNC) as the ELISA plate, HRP-labeled goat anti-mouse IgG (H&L) antibody (from American Qualex) as the secondary antibody, and TMB (from Calbiochem) for the color reaction.

FIG. 4 shows the results.

FIG. 4 (A) is a graph plotting the average absorbance of each group at OD450 nm obtained when the mouse sera were subjected to two-fold serial dilution from 800-fold to 102,400-fold dilutions. In the groups inoculated with PBS and AcNPV-WT, whose sera contained no antibody to the antigen, absorbance of even the 800-fold dilutions was 0.1 or less, indicating low reactivity. In contrast, in the groups inoculated with AcNPV-CAP-PfCSP and AcNPV-CAF-PfCSP/467, absorbance of the 800-fold dilutions was 1.138 and 1.878, respectively, indicating strong reactivity and clearly showing that antigen-specific antibodies were induced. FIG. 4 (B) is a graph plotting the average absorbance of each group at OD450 nm obtained when the mouse sera were subjected to two-fold serial dilution from 400-fold to 25,600-fold dilutions. In the groups inoculated with PBS and AcNPV-WT, whose sera contained no antibody to the antigen, absorbance of the 400-fold dilutions was 0.1 or less, indicating low reactivity. In contrast, in the group inoculated with AcNPV-CAP-HA1/Anhui and AcNPV-CAP-HA1/Vietnam, absorbance of the 3,200-fold dilution was 1.551 and 2.503, respectively, indicating strong reactivity and clearly showing that antigen-specific antibodies were induced. FIG. 4 clearly shows that the recombinant baculovirus produced using a transfer vector containing the dual promoter used in the present invention can induce an antibody to the desired antigen in mammals.

### 3. Measurement of Neutralization Value

A hemagglutination inhibition (HI) test was performed using a mouse serum inoculated with AcNPV-CAP-HA1/Anhui. More specifically, the test was performed according to the method described in the instructions packaged with an influenza HI reagent "Seiken" (from Denka Seiken Co., Ltd.), using purified HA antigen of H5N1 virus (IT-003-0053p from Immune Technology). Absorption of non-specific agglutinins was performed using erythrocyte, and removal of non-specific agglutination inhibitors was performed using RDE (II) of "Seiken" (from Denka Seiken Co., Ltd.). The HI test was performed in the following manner. 0.025 mL of 10-fold diluted antiserum in a 96-well plate was subjected to 2-fold serial dilution using a diluent. To each well of the 96-well plate containing the diluted antiserum, 0.025 mL of HA antigen of H5N1 virus diluted to obtain an HA titer of 4 per 0.025 mL thereof was added. The plate was allowed to stand at room temperature for 30 minutes. 0.05 mL of an erythrocyte suspension for the reaction was added and stirred well. The mixture was allowed to stand at room temperature for 60 minutes. The final dilution of the test sample at which hemagglutination was completely inhibited was defined as the HI antibody titer.

The results show that the sera inoculated with PBS and AcNPV-WT had HI antibody titers of 10 or less, whereas the serum inoculated with AcNPV-CAP-HA1/Anhui had an HI antibody titer of 40.

The results seem to indicate that when administered to humans and other mammals, the recombinant baculovirus produced from the recombinant transfer vector of the present disclosure can induce an antibody effective to the desired foreign antigen gene, thus providing vaccine effects.

### Sequence Listing Free Text

SEQ ID NOS: 1 and 2 are the sequences of primers phsp65-F1 and phsp65-R1 for PCR of genomic DNA of M. tuberculosis H37Rv.
SEQ ID NOS: 3 and 4 are the sequences of primers phsp65-F2 and phsp65-R2 for PCR with pcDNA-hps65 as a template.
SEQ ID NOS: 5 and 6 are the sequences of primers pPolh-F2 and pgp64-R2 for PCR with pBACsurf-1 as a template to produce a gp64 gene DNA fragment.
SEQ ID NOS: 7 and 8 are the sequences of primers HA-f and HA-r for PCR to produce an influenza virus HA gene fragment.
SEQ ID NOS: 9 and 10 are the sequences of primers pHA-F1 and pHA-R1 for PCR with pCR-Blunt-HA as a template.
SEQ ID NOS: 11 and 12 are the sequences of primers Polh-f RsrII and GP64-r DraIII for PCR with pBACsurf-HA1 as a template.
SEQ ID NOS: 13 and 14 are the sequences of primers NP-f EcoRI and NP-r Cfr9I for RT-PCR of genomic RNA of influenza virus PR/8/34 strain.
SEQ ID NOS: 15, 16, and 12 are the sequences of primers gp64(272)-f, gp64(467)-f, and GP64-r DraIII for PCR of pCAP-H1N1/HA1-gp64.
SEQ ID NOS: 17 and 18 are the sequences of primers PfCSP-f(19) and PfCSP-r(373) for PCR with *P*. *falciparum* genomic DNA as a template.
SEQ ID NOS: 19 and 20 are the sequences of primers pAMA-F1 and pAMA1-R1 for PCR with *P. berghei* genomic DNA as a template.
SEQ ID NOS: 21 and 22 are the sequences of primers pPfCSP-F1 and pPfCSP-R1 for PCR with *P*. *falciparum* genomic DNA as a template.
SEQ ID NOS: 23 and 24 are the sequences of primers pPfs25-F1 and pPfs25-R2 for PCR with *P*. *falciparum* genomic DNA as a template.
SEQ ID NOS: 25 and 26 are the sequences of primers pPfMSP119-F1 and pPfMSP119-R2 for PCR with *P*. *falciparum* genomic DNA as a template.
SEQ ID NOS: 17 and 27 are the sequences of primers PfCSP-f(19) and PfCSP-r(373 A361E) for PCR with pCAP-PfCSP as a template.
SEQ ID NOS: 28 and 27 are the sequences of primers PfCSP-f(76) and PfCSP-r(373 A361E) for PCR with pCAP-PfCSP as a template.
SEQ ID NO: 29 is the sequence of an artificial gene (PfCSP+) produced from the amino acid sequence of the PfCSP of *P*. *falciparum* 3D7 strain (in which, however, the A at the 361-position was replaced by E) using codons frequently used in Sf9 and human cells.
SEQ ID NOS: 30 and 31 are the sequences of primers PfCSP-f(+209) and PfCSP-r(+A361E) for PCR with PfCSP+ as a template.
SEQ ID NOS: 32, 33, 34 and 31 are the sequences of primers PfCSP-f(+76), PfCSP-r(+128), PfCSP-f(+209) BamHI, and PfCSP-r(+A361E) for PCR with PfCSP+ as a template.
SEQ ID NO: 35 is the sequence of an artificial gene produced from the amino acid sequence of the HA1 region of the hemagglutinin of influenza virus H5N1/Anhui/1/05 using codons frequently used in Sf9 and human cells.
SEQ ID NOS: 36 and 37 are the sequences of primers AH-F1 (5'-CAGTCTGCAGGACCAGATTTGCATC-3': (SEQ ID NO: 36); the PstI site is underlined) and AH-R4 (5'-CAGTCCCGGGCTCTCTTGCGCCTGC-3': (SEQ ID NO: 37); the XmaI site is underlined) for PCR with the artificial gene sequence of SEQ ID NO: 35 as a template.
SEQ ID NO: 38 is the sequence of an artificial gene produced from the amino acid sequence of the HA1 region of the hemagglutinin of influenza virus H5N1/Vietnam/1203/04 using codons frequently used in Sf9 and human cells.
SEQ ID NOS: 39 and 40 are the sequences of primers VN-F1 (5'-CAGTCTGCAGGACCAGATCTGTATC-3': (SEQ ID NO: 39); the PstI site is underlined), and VN-R4 (5'-CAGTCCCGGGCTCTCTTCTTCCTGC-3': (SEQ ID NO: 40); the XmaI site is underlined) for PCR with the artificial gene sequence of SEQ ID NO: 38 as a template.
SEQ ID NOS: 41, 42, 43, 44, and 12 are the sequences of primers gp64(51)-f (5'-GACTCCCCGGGTGGAAATCACCATCGTGGAGACG-3': (SEQ ID NO: 41); the XmaI site is underlined), gp64(101)-f (5'-GACTCCCCGGGATTTGCTTATGTGGAGCATCAGG-3': (SEQ ID NO: 42); the XmaI site is underlined), gp64(154)-f (5'-GACTCCCCGGGCGCACCACACGTGCAACAAATCG-3': (SEQ ID NO: 43); the XmaI site is underlined), gp64(201)-f (5'-GACTCCCCGGGACACTGTGCTTCATCGAGACGGC-3': (SEQ ID NO: 44); the XmaI site is underlined), and GP64-r DraIII (5'-GGGCACTTAGTGATATTGTCTATTACGGTTTCTAATC-3' (SEQ ID NO: 12); the Dralll site is underlined).
SEQ ID NO: 45 is the sequence of an artificial gene produced from the amino acid sequence of the HA1 region of the hemagglutinin of influenza virus H5N1/Anhui/1/05 by codon optimization using Gene Designer available from DNA2.0, Inc.
SEQ ID NOS: 46, 47, 48, 49, and 50 are the sequences of AH17-F (5'-GACTCTGCAGGATCAGATCTGTATTGGGTACC-3':(SEQ ID NO: 46); the PstI site is underlined, and AH345-R (5'-CGATCCCGGGCTCTCTTTCTCCTCCGCTCGC-3': (SEQ ID NO: 47); the XmaI site is underlined), AH410-R (5'-CGATCCCGGGCGGCCTCGAACTGGGTGTTCATT-3': (SEQ ID NO: 48); the XmaI site is underlined), AH473-R (5'-CGATCCCGGGCGTCTCTGAGTTGAAGGCGCAC-3':(SEQ ID NO: 49); the XmaI site is underlined, and AH520-R (5'-CGATCCCGGGCACCACTAATTTCCTCTCGCTTC-3':(SEQ ID NO: 50); the XmaI site is underlined) for PCR with the artificial gene sequence of SEQ ID NO: 45 as a template.
SEQ ID NO: 51 is the sequence of an artificial gene produced from the amino acid sequence of the HA1 region of the hemagglutinin of influenza virus H5N1/Vietnam/1203/04 by codon optimization using Gene Designer available from DNA2.0, Inc.
SEQ ID NOS: 52, 53, 54, 55, and 56 are the sequences of primers VN17-F (5'-GACTCTGCAGGATCAGATCTGTATCGGATATC-3': (SEQ ID NO: 52); the PstI site is underlined), and VN346-R (5'-CGATCCCGGGCCCGCTTTTTCCTCCTCCGTTCG-3' : (SEQ ID NO: 53); the XmaI site is underlined), VN410-R (5'-CGATCCCGGGCCTCAAACTGCGTATTCATTTTG-3' : (SEQ ID NO: 54); the XmaI site is underlined), VN473-R (5'-CGATCCCGGGCTCTAAGCTGGAGCCTGACTTTGTC-3': (SEQ ID NO: 55); the XmaI site is underlined), and VN520-R (5'-CGATCCCGGGCACTAATCTCCTCTCTTTTAAGTC-3': (SEQ ID NO: 56); the XmaI site is underlined) for PCR with the artificial gene sequence of SEQ ID NO: 51 as a template.
SEQ ID NO: 57 is the sequence of an artificial gene produced from the amino acid sequence of the CSP of *Plasmodium falciparum* 3D7 strain by codon optimization using Gene Designer available from DNA2.0, Inc.
SEQ ID NOS: 58, 59, 60, 61, and 62 are the sequences of primers PfCSP_opt-f (5'-GACTCTGCAGATGATGCGAAAATTGGCCATACTG-3':(SEQ ID NO: 58); the PstI site is underlined), PfCSP_opt-r (397) (5'-CGATCCCGGGCATTGAGGAACAGAAAGGAAAGAACCATG-3': (SEQ ID NO: 59); the XmaI site is underlined), PfCSP_opt-f (19) (5'-GACTCTGCAGCTGTTTCAGGAATACCAGTGCTATGG-3': (SEQ ID NO: 60); (the PstI site is underlined), PfCSP_opt-r(373) (5'-CGATCCCGGGCCTTCTCCATCTTACAAATTTTCTTTTCAATATCATTAGC -3': (SEQ ID NO: 61); (the XmaI site is underlined), PfCSP_opt-f (76) (5'-GACTCTGCAGGACGACGGAAATAATGAGGACAACG-3': (SEQ ID NO: 62); the PstI site is underlined), and PfCSP_opt-f (205) (5'-GACTCTGCAGAATGCAAACCCAAATGCCAATCCAAACGC-3': (SEQ ID NO: 63) ; the PstI site is underlined) for PCR with the artificial gene sequence of SEQ ID NO: 57 as a template.
SEQ ID NOS: 64, 65, 66, and 67 are the sequences of primers gp64-p-f (5'-GACTCGGACCGGCCAGATAAAAATAATCTTATCAATTAAG-3': (SEQ ID NO: 64); the RsrII site is underlined), gp64-p-r (5'-CGATACTAGTAGCACTGAGGCTTCTTATATACCCG-3': (SEQ ID NO: 65); the SpeI site is underlined), and vp39-p-f (5'-GACTCGGACCGCGTCGTACAAATCGAAATATTGTTGTG-3': (SEQ ID NO: 66) ; the RsrII site is underlined), and vp39-p-r (5'-CGATACTAGTGTGATTGAGAAAGAAATCTCTTATTC-3': (SEQ ID NO: 67); the SpeI site is underlined) for PCR with Baculovirus genomic DNA as a template.
SEQ ID NOS: 68 and 69 are the sequences of primers VSV-G-f (5'-GACTCCCCGGGCGTTCGAACATCCTCACATTCAAG -3' (SEQ ID NO: 68); the XmaI site is underlined), and VSV-G-r (5'-GACTCACTTAGTGCTTTCCAAGTCGGTTCATCTC-3': (SEQ ID NO: 69); the DraIII site is underlined) for PCR with pVSV-G as a template.

## Claims

1. A pharmaceutical composition comprising a recombinant Autographa californica nucleopolyhedrosis virus (AcNPV) as an active agent and, optionally, a pharmaceutically acceptable carrier,
wherein the recombinant AcNPV contains a gene structure having a fusion gene of
(A) a gene encoding an amino acid sequence consisting of amino acids 19-373 of SEQ ID NO:70 (Plasmodium falciparum CircumSporozoite Protein; PfCSP), and
(B) a gene encoding an amino acid sequence consisting of amino acids 21-512 of gp64 viral particle protein (gp64) encoded in the AcNPV genome (GenBank Accession No. L22858)
under the control of a dual promoter comprising Polyhedrin promoter and CAG promoter linked to each other (CAP).

2. A pharmaceutical composition of claim 1, which is a malaria vaccine.

## Patentansprüche

1. Pharmazeutische Zusammensetzung, umfassend ein rekombinantes AcNPV (Autographa californica Nucleopolyhedrosis Virus) als einen Wirkstoff und gegebenenfalls einen pharmazeutisch unbedenklichen Trägerstoff,
wobei das rekombinante AcNPV eine Genstruktur mit einem Fusionsgen von
(A) einem für eine aus Aminosäuren 19-373 von SEQ ID NO:70 (Plasmodium falciparum CircumSporozoite Protein; PfCSP) bestehende Aminosäuresequenz codierenden Gen und
(B) einem Gen, das für eine Aminosäuresequenz codiert, die aus Aminosäuren 21-512 des im AcNPV-Genom (GenBank Accession No. L22858) codierten gp64-Viruspartikelproteins (gp64) besteht,
unter der Kontrolle eines dualen Promotors, der Polyhedrin-Promotor und CAG-Promotor miteinander verknüpft umfasst, (CAP) enthält.

2. Pharmazeutische Zusammensetzung nach Anspruch 1, bei der es sich um einen Malaria-Impfstoff handelt.

## Revendications

1. Composition pharmaceutique comprenant un virus Autographa californica de la polyhydrose nucléaire (AcVPN) recombinant comme un agent actif et, en option, un véhicule acceptable d'un point de vue pharmaceutique,
dans laquelle l'AcVPN recombinant contient une structure génique ayant un gène de fusion de
(A) un gène codant pour une séquence d'acides aminés constituée par les acides aminés 19 à 373 de SEQ ID n° : 70 (protéine Circumsporozoïte de Plasmodium falciparum ; PfCSP), et
(B) un gène codant pour une séquence d'acides aminés constituée par les acides aminés 21 à 512 de la protéine gp64 des particules virales (gp64) codée dans le génome de l'AcVPN (n° d'ordre GenBank : L22858)
étant sous le contrôle d'un promoteur double, comprenant un promoteur de polyhédrine et un promoteur CAG liés l'un à l'autre (CAP).

2. Composition pharmaceutique, selon la revendication 1, qui est un vaccin contre la malaria.
